# EUROPEAN PATENT SPECIFICATION

(11) **EP 4 149 949 B1**
(45) Date of publication and mention of the grant of the patent: **14.10.2026**
(21) Application number: 21800914.0
(22) Date of filing: 04.05.2021
(51) Int. Cl.: C07F 5/02, C07F 5/04

(54) **BORONIC ACID DERIVATIVES AND SYNTHESIS, POLYMORPHIC FORMS, AND THERAPEUTIC USES THEREOF**
BORSÄUREDERIVATE UND SYNTHESE, POLYMORPHE FORMEN UND THERAPEUTISCHE VERWENDUNGEN DAVON
DÉRIVÉS D'ACIDE BORONIQUE ET SYNTHÈSE, FORMES POLYMORPHES ET UTILISATIONS THÉRAPEUTIQUES ASSOCIÉES

(30) Priority: 05.05.2020 US 202063020494 P
(43) Date of publication of application: 22.03.2023
(73) Proprietor: QPEX BIOPHARMA, INC., San Diego, CA 92121 (US)
(72) Inventor: HECKER, Scott J., San Diego, California 92121 (US); BOYER, Serge Henri, San Diego, California 92121 (US); BIO, Matthew M., Waltham, Massachusetts 02451 (US); FANG, Yuanqing, Waltham, Massachusetts 02451 (US); GONZALES DE CASTRO, Angela, 6167 RD Geleen (NL); LEFORT, Laurent, 6167 RD Geleen (NL); ZHU, Zuolin, San Diego, California 92121 (US); LINDER, Thomas, 4040 Linz (AT)
(74) Representative: Carpmaels & Ransford LLP
(86) International application number: PCT/US2021/030690
(87) International publication number: WO 2021/226114

(56) References cited:
- WO-A1-2018/005662
- WO-A1-2019/075084
- MICHAEL B. HURSTHOUSE ET AL: "Why Do Organic Compounds Crystallise Well or Badly or Ever so Slowly? Why Is Crystallisation Nevertheless Such a Good Purification Technique? +", ORGANIC PROCESS RESEARCH & DEVELOPMENT, vol. 13, no. 6, 20 November 2009 (2009-11-20), US, pages 1231 - 1240, XP055576610, ISSN: 1083-6160, DOI: 10.1021/op900169b
- DARIO BRAGA ET AL: "Crystal Polymorphism and Multiple Crystal Forms", STRUCT BOND, 1 January 2009 (2009-01-01), pages 25 - 50, XP055378617, Retrieved from the Internet <URL:http://www.springer.com/cda/content/document/cda_downloaddocument/9783642013669-c1.pdf?SGWID=0-0-45-744699-p173898909> DOI: 10.1007/430
- DUNITZ J D ET AL: "DISAPPEARING POLYMORPHS", ACCOUNTS OF CHEMICAL RESEARCH, ACS , WASHINGTON , DC, US, vol. 28, no. 4, 1 January 1995 (1995-01-01), pages 193 - 200, XP000916646, ISSN: 0001-4842, DOI: 10.1021/AR00052A005
- CRUZ-CABEZA AURORA J. ET AL: "Facts and fictions about polymorphism", CHEMICAL SOCIETY REVIEWS, vol. 44, no. 23, 1 December 2015 (2015-12-01), England, pages 8619 - 8635, XP093296423, ISSN: 0306-0012, Retrieved from the Internet <URL:https://pubs.rsc.org/en/content/articlepdf/2015/cs/c5cs00227c> DOI: 10.1039/C5CS00227C
- HECKER, S. J. ET AL.: "Discovery of Cyclic Boronic Acid QPX7728, an Ultrabroad- Spectrum Inhibitor of Serine and Metallo-beta-lactamases", J. MED. CHEM., vol. 63, 2020, pages 7491 - 7507, XP055858664, DOI: 10.1021/acs.jmedchem.9b01976

## Description

### STATEMENT REGARDING FEDERALLY SPONSORED R&D

This invention was made with U.S. government support under the Department of Health and Human Services Contract No. HHSO100201600026C. The U.S. government has certain rights in the invention.

### BACKGROUND

### Field

The present application relates to the fields of chemistry and medicine. More particularly, the present application relates to boronic acid crystalline forms, a method of making a boronic acid compound and intermediates.

### Description of the Related Art

Antibiotics have been effective tools in the treatment of infectious diseases during the last half-century. From the development of antibiotic therapy to the late 1980s there was almost complete control over bacterial infections in developed countries. However, in response to the pressure of antibiotic usage, multiple resistance mechanisms have become widespread and are threatening the clinical utility of anti-bacterial therapy. The increase in antibiotic resistant strains has been particularly common in major hospitals and care centers. The consequences of the increase in resistant strains include higher morbidity and mortality, longer patient hospitalization, and an increase in treatment costs.

Various bacteria have evolved β-lactam deactivating enzymes, namely, β-lactamases, that counter the efficacy of the various β-lactam antibiotics. β-lactamases can be grouped into 4 classes based on their amino acid sequences, namely, Ambler classes A, B, C, and D. Enzymes in classes A, C, and D include active-site serine β-lactamases, and class B enzymes, which are encountered less frequently, are Zn-dependent. These enzymes catalyze the chemical degradation of β-lactam antibiotics, rendering them inactive. Some β-lactamases can be transferred within and between various bacterial strains and species. The rapid spread of bacterial resistance and the evolution of multi-resistant strains severely limits β-lactam treatment options available.

The increase of class D β-lactamase-expressing bacterium strains such as *Acinetobacter baumannii* has become an emerging multidrug-resistant threat. *A. baumannii* strains express A, C, and D class β-lactamases. The class D β-lactamases such as the OXA families are particularly effective at destroying carbapenem type β-lactam antibiotics, e.g., imipenem, the active carbapenems component of Merck's Primaxin^{®} (Montefour, K. et al., Crit. Care Nurse 2008, 28, 15; Perez, F. et al., Expert Rev. Anti Infect. Ther. 2008, 6, 269; Bou, G.; Martinez-Beltran, J., Antimicrob. Agents Chemother. 2000, 40, 428. 2006, 50, 2280; Bou, G. et al., J. Antimicrob. Agents Chemother. 2000, 44, 1556). This has imposed a pressing threat to the effective use of drugs in that category to treat and prevent bacterial infections. Indeed the number of catalogued serine-based β-lactamases has exploded from less than ten in the 1970s to over 300 variants. These issues fostered the development of five "generations" of cephalosporins. When initially released into clinical practice, extended-spectrum cephalosporins resisted hydrolysis by the prevalent class A β-lactamases, TEM-1 and SHV-1. However, the development of resistant strains by the evolution of single amino acid substitutions in TEM-1 and SHV-1 resulted in the emergence of the extended-spectrum β-lactamase (ESBL) phenotype.

New β-lactamases have recently evolved that hydrolyze the carbapenem class of antimicrobials, including imipenem, biapenem, doripenem, meropenem, and ertapenem, as well as other β-lactam antibiotics. These carbapenemases belong to molecular classes A, B, and D. Class A carbapenemases of the KPC-type predominantly in *Klebsiella pneumoniae* but now also reported in other *Enterobacteriaceae, Pseudomonas aeruginosa* and *Acinetobacter baumannii.* The KPC carbapenemase was first described in 1996 in North Carolina, but since then has disseminated widely in the US. It has been particularly problematic in the New York City area, where several reports of spread within major hospitals and patient morbidity have been reported. These enzymes have also been recently reported in France, Greece, Sweden, United Kingdom, and an outbreak in Germany has recently been reported. Treatment of resistant strains with carbapenems can be associated with poor outcomes.

The zinc-dependent class B metallo-β-lactamases are represented mainly by the VIM, IMP, and NDM types. IMP and VIM-producing *K. pneumonia* were first observed in 1990s in Japan and 2001 in Southern Europe, respectively. IMP-positive strains remain frequent in Japan and have also caused hospital outbreaks in China and Australia. However, dissemination of IMP-producing *Enterobacteriaceae* in the rest of the word appears to be somewhat limited. VIM-producing enterobacteria can be frequently isolated in Mediterranean countries, reaching epidemic proportions in Greece. Isolation of VIM-producing strains remains low in Northern Europe and in the United States. In stark contrast, a characteristic of NDM-producing *K. pneumonia* isolates has been their rapid dissemination from their epicenter, the Indian subcontinent, to Western Europe, North America, Australia and Far East. Moreover, NDM genes have spread rapidly to various species other than K. *pneumonia.*

The plasmid-expressed class D carbapenemases belong to OXA-48 type. OXA-48 producing *K. pneumonia* was first detected in Turkey, in 2001. The Middle East and North Africa remain the main centers of infection. However, recent isolation of OXA-48-type producing organisms in India, Senegal and Argentina suggest the possibility of a global expansion. Isolation of OXA-48 in bacteria other than *K. pneumonia* underlines the spreading potential of OXA-48.

Treatment of strains producing any of these carbapenemases with carbapenems can be associated with poor outcomes.

Another mechanism of β-lactamase mediated resistance to carbapenems involves combination of permeability or efflux mechanisms combined with hyper production of beta-lactamases. One example is the loss of a porin combined in hyperproduction of ampC β-lactamase results in resistance to imipenem in *Pseudomonas aeruginosa.* Efflux pump over expression combined with hyperproduction of the ampC β-lactamase can also result in resistance to a carbapenem such as meropenem.

New β-lactamase inhibitors (BLIs) have recently been described. However, there is a need for efficient methods of synthesizing these BLIs. BLIs effective in treating bacterial infections when used in combination with β-lactam antibiotics are described in International Patent Publication No. WO 2019/075084.

### SUMMARY OF THE INVENTION

In some embodiments, provided herein is a crystalline form of Compound I': 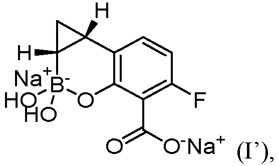 or a solvate thereof. The crystalline form of Compound I' exhibits an X-ray powder diffraction pattern comprising at least one characteristic peak, wherein said characteristic peak is selected from the group consisting of approximately 6.2, 11.7, 12.4, 12.8, 16.7, 17.6, 19.1, 20.2, 20.6, 21.7, 22.0, 22.7, and 24.7 degrees 2θ. In some embodiments, the crystalline form of Compound I' may exhibit an X-ray powder diffraction pattern comprising at least three characteristic peaks, wherein said characteristic peaks are selected from the group consisting of 6.2, 11.7, 12.4, 12.8, 16.7, 17.6, 19.1, 20.2, 20.6, 21.7, 22.0, 22.7, and 24.7 degrees 2θ.

In some embodiments, the crystalline form of Compound I' may have an endotherm at about 142° C and at about 167° C. In some embodiments, the crystalline form has an endotherm at about 152 °C.

In some embodiments, the crystalline form of Compound I' may be a hydrate. In some embodiments, the crystalline form of Compound I' may be a monohydrate.

In other embodiments, provided herein is a method of making a compound of Formula (A-II) or pharmaceutically acceptable salt thereof, 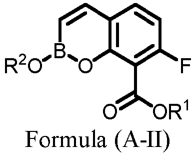
comprising the step of:
reacting a compound of Formula (A-I) 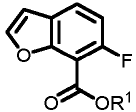 with a borylating agent in a solvent system, in the presence of a nickel catalyst and a base system
wherein
R¹ is a carboxylic acid protecting group;
R² is hydrogen or C₁-C₆ alkyl; and
the solvent system and base system is selected from the group consisting of: THF and CsOH·H₂O, respectively; THF/H₂O and Cs₂CO₃, respectively; ethanol and CsOH·H₂O, respectively; THF/ethanol and Cs₂CO₃, respectively; 2-methyl tetrahydrofuran (MeTHF) and CsOH·H₂O, respectively; MeTHF/H₂O and Cs₂CO₃, respectively; MeTHF/ethanol and Cs₂CO₃, respectively;THF and a mixture of Cs₂CO₃ and K₂CO₃, respectively; MeTHF and a mixture of Cs₂CO₃ and K₂CO₃, respectively; THF/H₂O and a mixture of Cs₂CO₃ and K₂CO₃, respectively; MeTHFH₂O and a mixture of Cs₂CO₃ and K₂CO₃, respectively; and MeTHF/ethanol and a mixture of Cs₂CO₃ and K₂CO₃, respectively; and wherein the molar ratio of base system to the compound of formula (A-I) is from 1:1 to 2:1.

In some embodiments the carbonate base can be a mixture of Cs₂CO₃ and K₂CO₃. In some embodiments the cesium to potassium carbonate base molar ratio may be from about 1:2 to 2:1. In some embodiments the cesium to potassium carbonate base molar ratio may be from 1.5:1, 1.6:1, 1.7:1 or 1.8:1, or within a range defined by any of the aforementioned ratios.

In some embodiments, the molar ratio of base system to the compound of formula (A-I) may be about 2:1, 1.5:1 or 1:1.

In some embodiments, the bases in the base system may be milled. In other embodiments, the bases in the base system may be granular.

In some embodiments, the solvent system may be THF/H₂O 5% v/v and the base system may be Cs₂CO₃. In other embodiments, the solvent system may THF/ethanol 5% v/v and the base system may be Cs₂CO₃.

In some embodiments, the solvent system may be MeTHF/H₂O 5% v/v and the base system may be Cs₂CO₃. In other embodiments, the solvent system may MeTHF/ethanol 5% v/v and the base system may be Cs₂CO₃.

In some embodiments, the ratio of borylating agent to the compound of formula (A-I) is from about 1:1 to about 2:1. In some embodiments, the ratio of borylating agent to the compound of formula (A-I) is about 1.5:1. In some embodiments, the ratio of borylating agent to the compound of formula (A-I) is about 1.25:1.

In some embodiments, the borylating agent may be selected from the group consisting of (HO)₂B-B(OH)₂, B₂(Pin)₂, B₂(Cat)₂, and B₂neop₂. In some embodiments, the borylating agent may be B₂(Pin)₂.

In some embodiments, the nickel catalyst precursor and ligand are combined to form the nickel catalyst prior to reacting the compound of Formula (A-I) with a borylating agent in a solvent system and a base system. In other embodiments, the nickel catalyst precursor and ligand combine to form the nickel catalyst while in the presence of compound of Formula (A-I) with a borylating agent in a solvent system and a base system.

In some embodiments the Ni catalyst precursor may be selected from the group consisting of NiCl₂, Ni(Acac)₂, and Ni(COD)₂.

In some embodiments, the Ni catalyst precursor may be NiCl₂. In some embodiments, the ligand may be selected from the group consisting of NHC ligand, phosphine, phosphite, phosphoramidite, amine, alcohol, aminoalcohol, and a combination thereof. In some embodiments, the ligand may be P(Octyl)₃, diPrf, dcype, P(*n*-propyl)₃, or P(*n*-Bu)₃. In some embodiments, the ligand may be P(*n*-Bu)₃.

In some embodiments, the nickel catalyst may be NiCl₂(PPh₃)₂, NiCl₂(PCy₂Ph)₂, NiCl₂(PPh₂CH₂CH₂PPh₂), NiCl₂(1,3-Bis(diphenylphosphino)propane), NiCl₂(1,3-Bis(diphenylphosphino)ethane), or NiCl₂(P(*n*-Bu)₃)₂. In some specific embodiments, the nickel catalyst is NiCl₂(P(*n*-Bu)₃)₂.

In some embodiments, the organoboron intermediate of Formula (A-II) may be reacted with (+)-pseudoephedrine as a chiral auxiliary to form an organoboron intermediate of

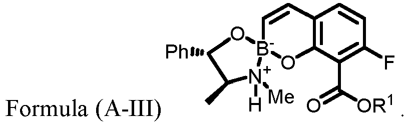

In some embodiments, the organoboron intermediate of Formula (A-III) may be converted to an organoboron intermediate of 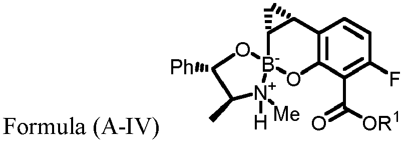 in the presence of a cyclopropanating agent and optional catalyst.

In some embodiments, the cyclopropanating agent comprises CH₂I₂ and Zn/Cu couple. In some embodiments, the cyclopropanating agent comprises CH₂Br₂, Zn and CuCl. In some embodiments, the cylopropanating agent comprises ZnEt₂ and CH₂I₂.

In some embodiments, the cyclopropanating agent is CH₂N₂ and the catalyst may be a palladium catalyst. In some embodiments, the palladium catalyst may be Pd(OAc)₂. In other embodiments, the palladium catalyst may be Pd₂(DVTMS)₃ (DVTMS = 1,3-divinyl-1,1,3,3-tetramethyldisiloxane). In some embodiments, the palladium catalyst may be Pd(acac)₂. In some embodiments, the palladium catalyst may be Pd(TFA)₂. In some embodiments, the palladium catalyst may be Pd(OPiv)₂.

In some embodiments, the compound of Formula (A-IV) may be hydrolyzed to form Compound I:

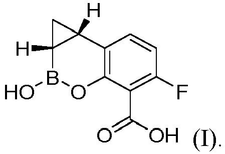

In some embodiments, the organoboron intermediate of Formula (A-II) may be reacted with a chiral auxiliary of Formula (Aux-I): 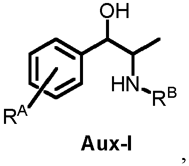 or stereoisomers thereof, to form an organoboron intermediate of Formula (B-III) 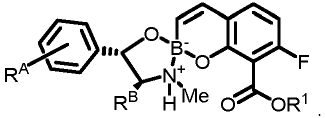 In some embodiments, R^{A} may be hydrogen. In some embodiments, R^{A} may be halogen. In other embodiments, R^{A} may be C₁₋₆ alkyl. In some specific embodiments, R^{A} may be methyl. In some embodiments, R^{B} may be C₁₋₆ alkyl. In some embodiments, R^{B} may be methyl. In other embodiments, R^{B} may be ethyl. In yet other embodiments, R^{B} may be isopropyl. In some embodiments, the organoboron intermediate of Formula (B-III) is 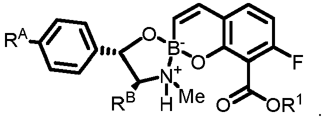 In some embodimens, the compound of Formula (B-III) has the structure of compound A-3: 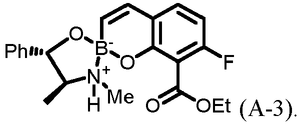 In some embodiments, the chiral auxiliary is not (+)-pseudoephedrine.

In some embodiments, the organoboron intermediate of Formula (B-III) may be converted to an organoboron intermediate of Formula (B-IV) 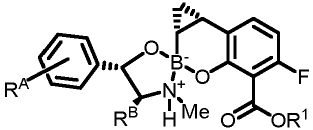 in the presence of a cyclopropanating agent and optional catalyst. In some embodiments, the organoboron intermediate of Formula (B-IV) is 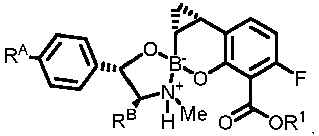 In some specific embodiments, the orgaroboron intermediate of Formula (B-IV) is

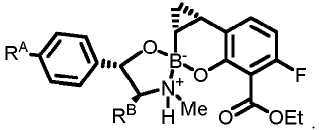

In some embodiments, the cyclopropanating agent comprises CH₂I₂ or CH₂Br₂; and one or more metal compounds. In some embodiments, the cyclopropanating reagent comprises CH₂I₂. In other embodiments, the cyclopropanating reagent comprises CH₂Br₂. In some embodiments the metal compound comprises one or more metals selected from the group consisting of zinc, copper, samarium, aluminum, and silver. In some specific embodiments, the metal compound may be Zn/Cu couple, Zn/Ag couple, Sm/Hg couple, ZnEt₂, or a mixture of zinc and CuCl.

In some embodiments, the cyclopropanating agent comprises CH₂I₂ and Zn/Cu couple. In some embodiments, the cyclopropanating agent comprises CH₂Br₂, Zn and CuCl. In some embodiments, the cylopropanating agent comprises ZnEt₂ and CH₂I₂.

In some embodiments, the cyclopropanating agent is CH₂N₂ and the catalyst may be a palladium catalyst. In some embodiments, the palladium catalyst may be Pd(OAc)₂. In other embodiments, the palladium catalyst may be Pd₂(DVTMS)₃ (DVTMS = 1,3-divinyl-1,1,3,3-tetramethyldisiloxane). In some embodiments, the palladium catalyst may be Pd(acac)₂. In some embodiments, the palladium catalyst may be Pd(TFA)₂. In some embodiments, the palladium catalyst may be Pd(OPiv)₂.

In some embodiments, the chiral auxiliary may be a compound having the fomula

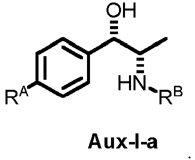

In some embodiments, the compound of Formula (B-IV) may be hydrolyzed to form Compound I.

In some embodiments, the method disclosed herein may comprise converting the organoboron intermediate of Formula (B-IV) to the organoboron intermendiate of Formula (B-V) 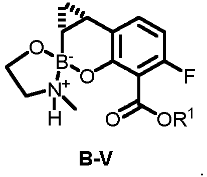 In some embodiments, the method may further comprise the step of hydrolyzing the compound of Formula (B-IV) or Formula (B-V) to form Compound I: 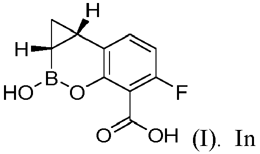 some embodiments, R¹ may be C₁₋₆ alkyl. In some embodiments, R¹ may be ethyl.

In some embodiments, the method descrbed herein may include the step of washing the compound of Formula (B-IV) or Formula (B-V) with aqueous acid prior to hydrolysis to form Compound I'.

In some embodiments, the method described herein may comprise the step of hydrolyzing the compound of Formula (A-IV), Formula (B-IV), or Formula (B-V) in the presence of a base to form crude Compound I' 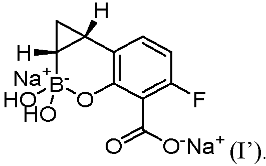 In some embodiments, R¹ may be C₁₋₆ alkyl. In other embodiments, R¹ may be ethyl. In some embodiments, the base may aqueous sodium hydroxide.

In some embodiments, the method disclosed herein the step of hydrolyzing the compound of Formula (A-IV) may performed at a temperature of from about 50 °C to about 70 °C. In some embodiments, the step of hydrolyzing the compound of Formula (A-IV) may be performed at a temperature of about 60 °C. In some embodiments, the step of hydrolyzing the compound of Formula (A-IV) may performed for from about 30 minutes to about 6 hours.

In some embodiments, the method disclosed herein may further comprise the step of treating crude Compound I' with an ion exchange resin to achieve a pH of from about 9 to about 10.5. In some embodiments, crude Compound I' may be treated with an ion exchange resin to achieve a pH of about 9.8.

In some embodiments, the method may comprise further filtering off the ion exchange resin to form an aqueous filtrate. In some embodiments, the aqueous filtrate may be concentrated in vacuo to form a concentrated aqueous filtrate. In some embodiments, the aqueous filtrate may concentrated in vacuo at a temperature of from about 20 °C to about 60 °C.

In some embodiments, the concentrated aqueous filtrate may added to methyl ethyl ketone to form a crystallization solution. In some embodiments, the crystallization solution may comprise from 1.0% to 5.0% water by weight relative to the quantity of methyl ethyl ketone. In some embodiments, the crystallization solution may comprise from 2.5% to 4.5% water by weight relative to the quantity of methyl ethyl ketone.

In some embodiments, the method may comprise crystallizing Compound I' in the crystallization solution to form crystallized Compound I'. In some embodiments, the crystallized Compound I' may be filtered and dried in vacuo. In some embodiments, the crystallized Compound I' may filtered and dried in vacuo at a pressure of from about 100 mbar to about 500 mbar to form Compound I' monohydrate. In some specific embodiments, the crystallized Compound I' may be filtered and dried in vacuo at a pressure of 100 mbar to form Compound I' monohydrate.

In some embodiments, provided herein is a crystalline form of compound A-3: 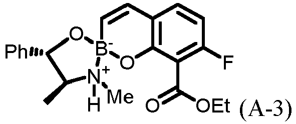 or a solvate thereof, wherein the crystalline form of compound A-3 exhibits an X-ray powder diffraction pattern comprising at least one characteristic peak, wherein said characteristic peak is selected from the group consisting of approximately 7.6. 8.0, 10.4, 13.7, 14.0, 16.0, 16.3, 19.0, 20.9, 23.2, 23.4, and 24.4 degrees 2θ.

In some embodiments, the crystalline form of compound A-3 may exhibit an X-ray powder diffraction pattern comprising at least three characteristic peaks, wherein said characteristic peaks are selected from the group consisting of 7.6. 8.0, 10.4, 13.7, 14.0, 16.0, 16.3, 19.0, 20.9, 23.2, 23.4, and 24.4 degrees 2θ.

In some embodiments, the crystalline form of compound A-3 may have a melting point of about 194 °C.

In some embodiments, the crystalline form of compound A-3 may be anhydrous.

In some embodiments, provided herein is a crystalline form of a compound of Formula (A-4): 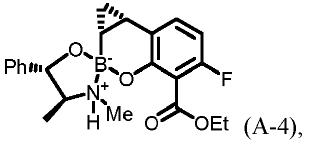 or a solvate thereof, wherein the crystalline form exhibits an X-ray powder diffraction pattern comprising at least one characteristic peak, wherein said characteristic peak is selected from the group consisting of approximately 7.4, 8.9, 10.7, 11.2, 11.5, 12.8, 13.7, 15.3, 17.0, 17.7, 18.7, 20.1, 21.4, and 22.6 degrees 2θ.

In some embodiments, the crystalline form exhibits an X-ray powder diffraction pattern comprising at least three characteristic peaks, wherein said characteristic peaks are selected from the group consisting of 7.4, 8.9, 10.7, 11.2, 11.5, 12.8, 13.7, 15.3, 17.0, 17.7, 18.7, 20.1, 21.4, and 22.6 degrees 2θ.

In some embodiments, the crystalline form of compound (A-4) may have an endotherm at about 176 °C.

In some embodiments, the crystalline form compound (A-4) may be unsolvated. In some embodiments the crystalline form of compound (A-4) may exhibit an X-ray powder diffraction pattern comprising at least one characteristic peak, wherein said characteristic peak is selected from the group consisting of approximately 7.4, 7.9, 9.1, 11.1, 12.0, 13.7, 14.4, 15.8, 16.4, 17.6, 19.8, 20.9, and 22.8 degrees 2θ. In some embodiments, the crystalline form of compound (A-4) may exhibit an X-ray powder diffraction pattern comprising at least three characteristic peaks, wherein said characteristic peaks are selected from the group consisting of 7.4, 7.9, 9.1, 11.1, 12.0, 13.7, 14.4, 15.8, 16.4, 17.6, 19.8, 20.9, and 22.8 degrees 2θ.

In some embodiments, provided herein compound I' is 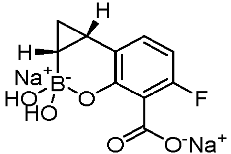 monohydrate.

In some embodiments, provided herein is a compound of Formula (A-IV): 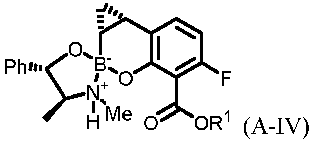 or a pharmaceutically acceptable salt or solvate thereof. In some embodiments, R¹ may be C₁₋₆ alkyl. In some embodiments, the compound may be Compound (A-4) 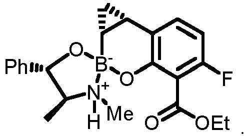 Some embodiments provide a cryrstalline form of the compound of Compound A-4.

In some embodiments, provided herein is a compound of Formula (B-III): 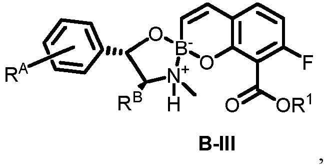 or a pharmaceutically acceptable salt or solvate thereof, wherein
R^{A} is C₁₋₆ alkyl or halogen;
R^{B} is hydrogen or C₁₋₆ alkyl; and
R¹ is C₁₋₆ alkyl.

In some embodiments, provided herein is a compound of Formula (B-IV): 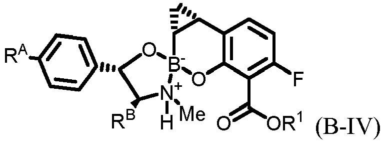 or a pharmaceutically acceptable salt or solvate thereof, wherein R^{A} is C₁₋₆ alkyl or halogen; R^{B} is hydrogen or C₁₋₆ alkyl; and R¹ is C₁₋₆ alkyl.

In some embodiments, R^{A} may be methyl.

In some embodiments, R^{B} may be methyl. In other embodiments, R^{B} may be ethyl.

In some embodiments, R¹ may be ethyl.

In some embodiments, the compound of Formula (B-IV) may be 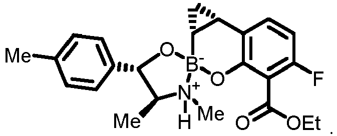 In some embodiments, the compound of Formula (B-IV) may be

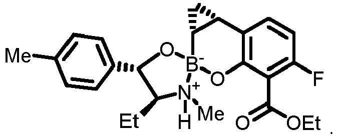

In some embodiments, provided herein is a compound of the Formula (B-V): 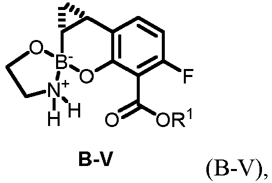 or a pharmaceutically acceptable salt or solvate thereof, wherein R¹ is C₁₋₆ alkyl. In some embodiments, the compound of Formula B-V is

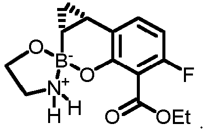

### BRIEF DESCRIPTION OF THE DRAWINGS

FIGURE 1 is an X-ray powder diffraction (XRPD) pattern of crystalline Form A of Compound I'.
FIGURE 2 shows a differential scanning calorimetry (DSC) analysis for crystalline Form A of Compound I'.
FIGURE 3 shows thermogravimetric analysis (TGA) results for crystalline Form A of Compound I'.
FIGURE 4 shows dynamic vapor sorption (DVS) results for crystalline Form A of Compound I'
FIGURE 5 shows results obtained by Fourier Transform Infrared (FTIR) spectroscopy for crystalline Form A of Compound I'.
FIGURE 6 shows results obtained by Fourier Transform Raman (FT Raman) spectroscopy for crystalline Form A of Compound I'.
FIGURE 7 shows results obtained by solid state carbon-13 nuclear magnetic resonance (¹³C-NMR) spectroscopy for crystalline Form A of Compound I'.
FIGURE 8 shows an optical microscopy image of crystals of crystalline Form A of Compound I'.
FIGURE 9 is an X-ray powder diffraction pattern of a crystalline form of Compound A-3.
FIGURE 10 shows a differential scanning calorimetry analysis for a crystalline form of Compound A-3.
FIGURE 11 shows thermogravimetric analysis results for a crystalline form of Compound A-3.
FIGURE 12 shows dynamic vapor sorption results for a crystalline form of Compound A-3.
FIGURE 13 shows results obtained by FTIR spectroscopy for a crystalline form of Compound A-3.
FIGURE 14 shows results obtained by FT Raman spectroscopy for a crystalline form of Compound A-3.
FIGURE 15 shows results obtained by low frequency Raman (LF Raman) spectroscopy for a crystalline form of Compound A-3
FIGURE 16 is an optical microscopy image of a crystalline form of Compound A-3.
FIGURE 17 shows the asymmetric unit of the crystal structure of Compound A-3.
FIGURE 18 is an X-ray powder diffraction pattern of a crystalline form of Compound A-4.
FIGURE 19 shows a differential scanning calorimetry analysis for a crystalline form of Compound A-4.
FIGURE 20 shows thermogravimetric analysis results for a crystalline form of Compound A-4.
FIGURE 21 shows dynamic vapor sorption results for a crystalline form of Compound A-4.
FIGURE 22 shows results obtained by FTIR spectroscopy for a crystalline form of Compound A-4.
FIGURE 23 shows results obtained by FT Raman spectroscopy for a crystalline form of Compound A-4.
FIGURE 24 shows an optical microscopy image of a crystalline form of Compound A-4.
FIGURE 25 shows the conversion of A-3 to A-4 for a variety of palladium catalysts.
FIGURE 26 shows the mass balance from the catalyst screening.
FIGURE 27 is an X-ray powder diffraction pattern of a crystalline form of Compound A-4 obtained after dynamic vapor sorption analysis.
FIGURE 28 shows a differential scanning calorimetry (DSC) analysis for a highly crystalline sample of Form A of Compound I'.
FIGURE 29 shows thermogravimetric analysis (TGA) results for a highly crystalline sample of Form A of Compound I'.
FIGURE 30 shows dynamic vapor sorption (DVS) results for a highly crystalline sample of Form A of Compound I'

### DETAILED DESCRIPTION OF EMBODIMENTS

Compound I and pharmaceutically acceptable salts thereof are described in WO2018005662A1. Compound I is a β-lactamase inhibitor effective in treating bacterial infections when used in combination with β-lactam antibiotics.

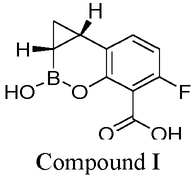

Disclosed herein is a crystalline form of the disodium salt of Compound I and methods of crystallizing the disodium salt of Compound I. The disodium salt of Compound I is shown below and will herein be refered to as Compound I':

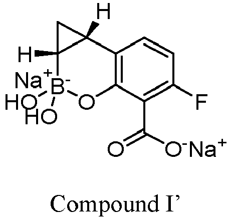

The present application relates to solid forms of crystalline Compound I' as defined in the claims. The claimed crystalline Compound I' form can be used in treating diseases and disorders by administering to a patient a therapeutically effective amount of a composition comprising the claimed crystalline form of compound I', and one or more pharmaceutically acceptable excipients.

### Crystalline Forms of Compound I'

Disclosed herein are crystalline forms of Compound I', and in particular crystalline Form A (described below).

### Crystalline Form A of Compound I'

Some embodiments include a crystalline form of Compound I', referred to herein as crystalline Form A of Compound I'. The precise conditions for forming crystalline Form A of Compound I' may be empirically determined and it is only possible to give a number of methods which have been found to be suitable in practice.

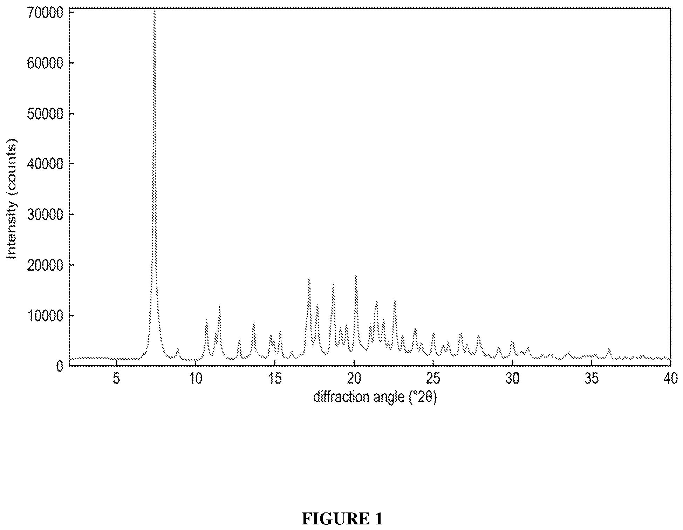

Crystalline Form A of Compound I' was characterized using various techniques which are described in further detail in the experimental methods section. FIGURE 1 shows the crystalline structure of Form A as determined by X-ray powder diffraction (XRPD). Crystalline Form A of Compound I', which may be obtained by the methods disclosed herein, exhibits prominent peaks at approximately 6.2, 11.7, 12.4, 12.8, 16.7, 17.6, 19.1, 20.2, 20.6, 21.7, 22.0, 22.7, and 24.7 degrees 20. In the claims, the crystalline form of Compound I' has at least one characteristic peak *(e.g.* , one, two, three, four, five, six, seven, eight, nine, ten, eleven, twelve, or thirteen characteristic peaks) selected from approximately 6.2, 11.7, 12.4, 12.8, 16.7, 17.6, 19.1, 20.2, 20.6, 21.7, 22.0, 22.7, and 24.7 degrees 20. In some embodiments, a crystalline form of Compound I' has at least three characteristic peaks selected from approximately 6.2, 11.7, 12.4, 12.8, 16.7, 17.6, 19.1, 20.2, 20.6, 21.7, 22.0, 22.7, and 24.7 degrees 2θ.

As is well understood in the art, because of the experimental variability when X-ray diffraction patterns are measured on different instruments, the peak positions are assumed to be equal if the two theta (2θ) values agree to within a certain degree of variability. For example, the United States Pharmacopeia states that if the angular setting of the 10 strongest diffraction peaks agree to within ± 0.2 degrees with that of a reference material, and the relative intensities of the peaks do not vary by more than 20%, the identity is confirmed. Accordingly, in some embodiments, peak positions recited herein include variability within ± 0.5 degrees 2θ. In other embodiments, peak positions recited herein include variability within ± 0.2 degrees 2θ. As disclosed herein, the term "approximately" when referring to values of 2θ is defined as ± 0.5 degrees 2θ.

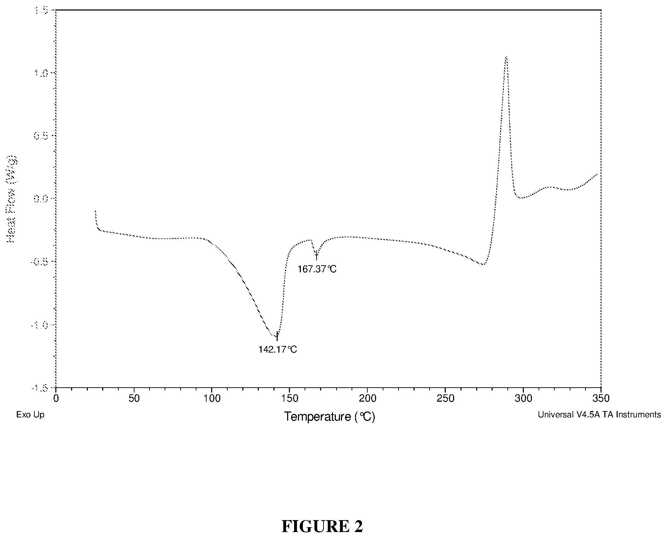

FIGURE 2 shows results obtained by differential scanning calorimetry (DSC) for crystalline Form A of Compound I'. The DSC results indicate endotherms of about 142 °C and 167 °C for crystalline Form A of Compound I'. Accordingly, in some embodiments, crystalline Form A of Compound I' exhibits an endotherm from about 139 °C to 145 °C, from about 140 °C to about 144 °C, or at about 142 °C. In some embodiments, crystalline Form A of Compound I' exhibits an endotherm from about 164 °C to 170 °C, from about 165 °C to about 169 °C, or at about 167 °C. Melting point analysis indicates that crystalline Form A of Compound I' decomposes at about 270 °C, prior to melting.

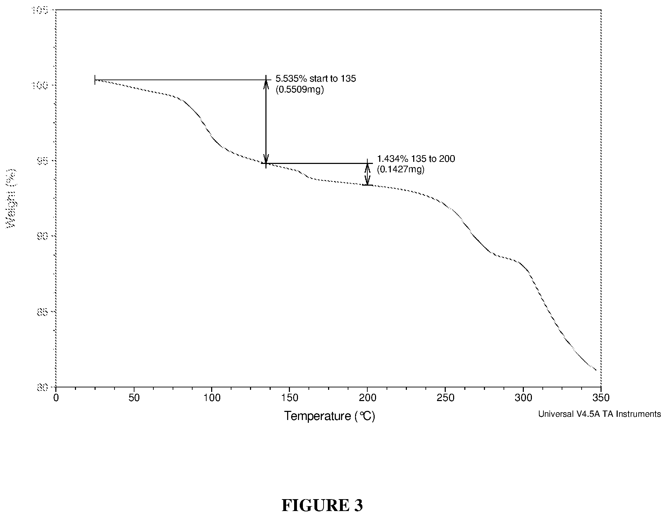

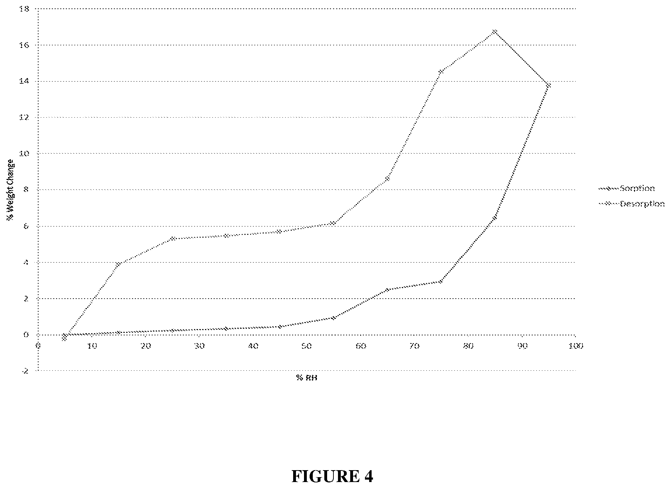

FIGURE 3 shows results obtained by thermogravimetric analysis (TGA) for crystalline Form A of Compound I'. The TGA results show that crystalline Form A of Compound I' exhibited at 5.5% weight loss when carried from 25 °C to 13 5 °C, and a 1.43% weight loss from 135 °C to 200 °C. Meanwhile, FIGURE 4 shows dynamic vapor sorption (DVS) results for crystalline Form A of Compound I', and shows significant water uptake and indicates that crystalline Form A of Compound I' is hygroscopic. Karl Fisher analysis indicates that crystalline Form A of Compound I' contains, on average, 6.86% water, corresponding to one molecule of water.

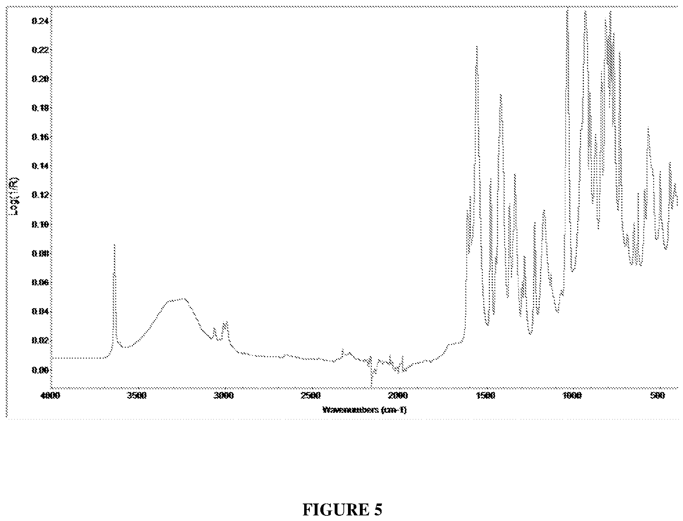

FIGURE 5 shows results obtained by Fourier Transform Infrared (FTIR) spectroscopy for crystalline Form A of Compound I'. Crystalline Form A of Compound I' exhibits prominent peaks at approximately 1608, 1592, 1553, 1473, 1416, 1364, 1334, and 1277 cm⁻¹. Thus, in some embodiments, a crystalline form of Compound I' has at least one characteristic FTIR peak *(e.g.,* one, two, three, four, five, six, seven, or eight characteristic peaks) selected from approximately 1608, 1592, 1553, 1473, 1416, 1364, 1334, and 1277 cm⁻¹. In some embodiments, a crystalline form of Compound I' has at least three characteristic peaks selected from 1608, 1592, 1553, 1473, 1416, 1364, 1334, and 1277 cm⁻¹. In some embodiments, peak positions recited herein include variability within ± 1 cm⁻¹.

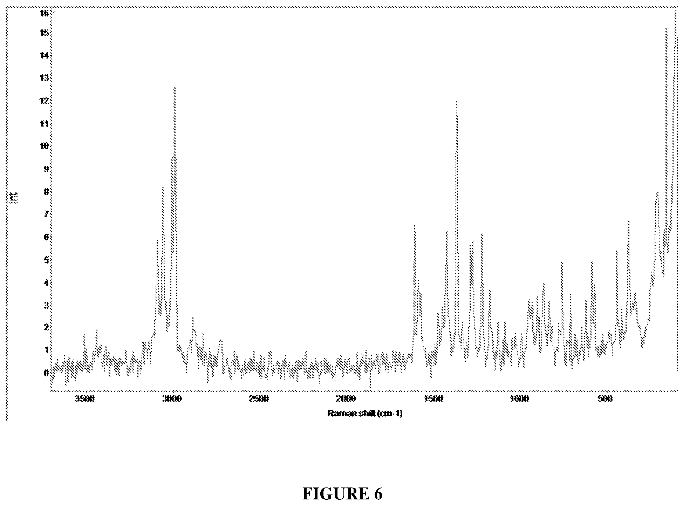

FIGURE 6 shows results obtained by Fourier Transform Raman spectroscopy for crystalline Form A of Compound I'. Crystalline Form A of Compound I' exhibits prominent peaks at approximately 1611, 1591, 1574, 1472, 1426, and 1366 cm⁻¹. Thus, in some embodiments, a crystalline form of Compound I' has at least one characteristic FT Raman peak *(e.g.* , one, two, three, four, five, or six characteristic peaks) selected from approximately 1611, 1591, 1574, 1472, 1426, and 1366 cm⁻¹. In some embodiments, a crystalline form of Compound I' has at least three characteristic peaks selected from 1611, 1591, 1574, 1472, 1426, and 1366 cm⁻¹. In some embodiments, peak positions recited herein include variability within ± 2 cm⁻¹.

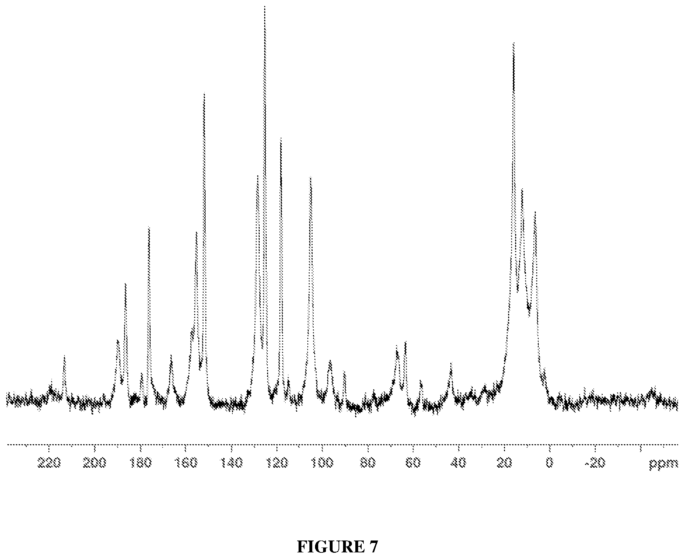

FIGURE 7 shows results obtained by solid state carbon-13 nuclear magnetic resonance (¹³C-NMR) NMR spectroscopy for crystalline Form A of Compound I'. Crystalline Form A of Compound I' exhibits prominent peaks at approximately 104.97, 117.98, 125.24, 128.28, 151.87, 155.24, 176.15, 186.58, and 190.06 ppm. Thus, in some embodiments, a crystalline form of Compound I' has at least one characteristic ¹³C-NMR peak *(e.g.,* one, two, three, four, five, or six characteristic peaks) selected from approximately 104.97, 117.98, 125.24, 128.28, 151.87, 155.24, 176.15, 186.58, and 190.06 ppm. In some embodiments, a crystalline form of Compound I' has at least three characteristic ¹³C-NMR peaks selected from 104.97, 117.98, 125.24, 128.28, 151.87, 155.24, 176.15, 186.58, and 190.06 ppm.

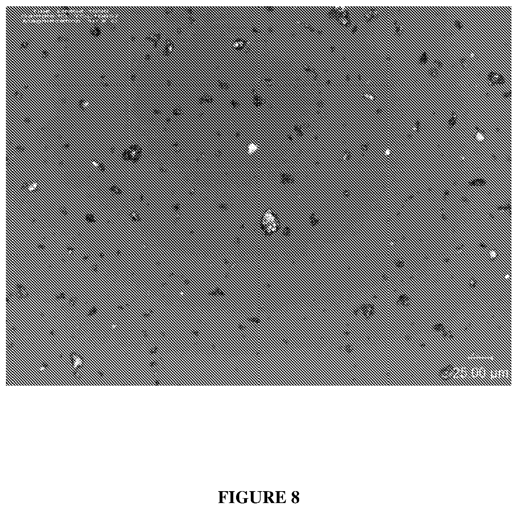

Crystalline Form A of Compound I' can therefore be characterized as a hygroscopic solid. Crystalline Form A of Compound I' also shows good crystallinity, with irregularly shaped crystals of varying sizes (FIGURE 8). Crystalline Form A of Compound I' shows evidence of hydrate formation, wherein the molar ratio of Compound I' to water is about 1:1 (i.e., a monohydrate).

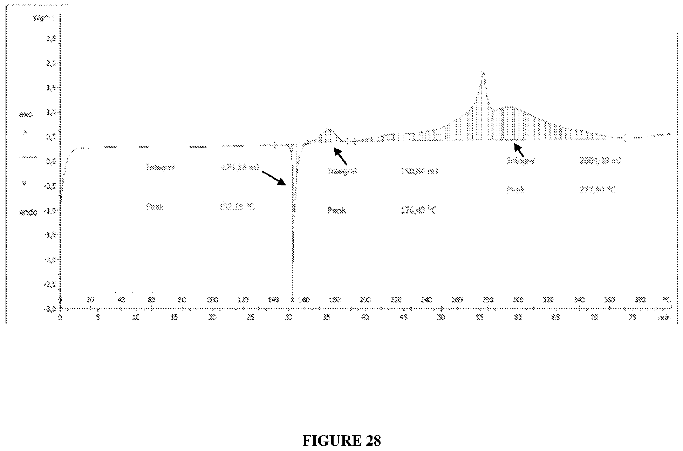

FIGURE 28 shows results obtained by differential scanning calorimetry (DSC) for a highly crystalline sample of Form A of Compound I'. The DSC results indicates an endotherm of about 152 °C for crystalline Form A of Compound I' exhibits an endotherm from about 151 °C to 153 °C, from about 150 °C to about 154 °C, or at about 152 °C.

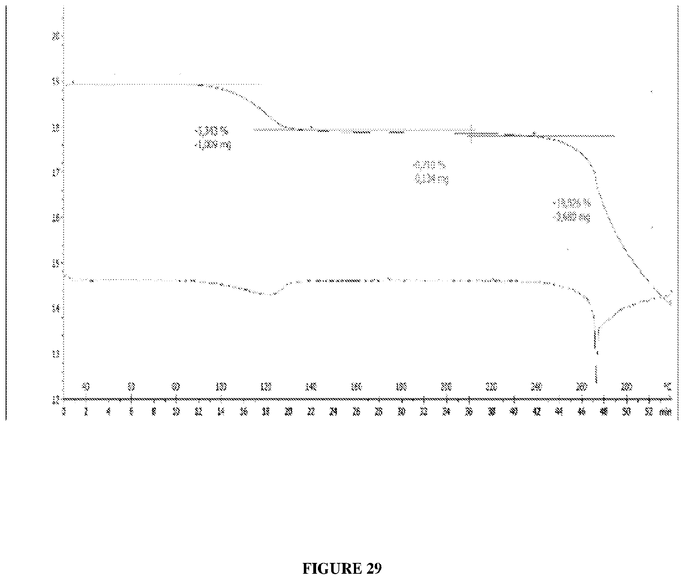

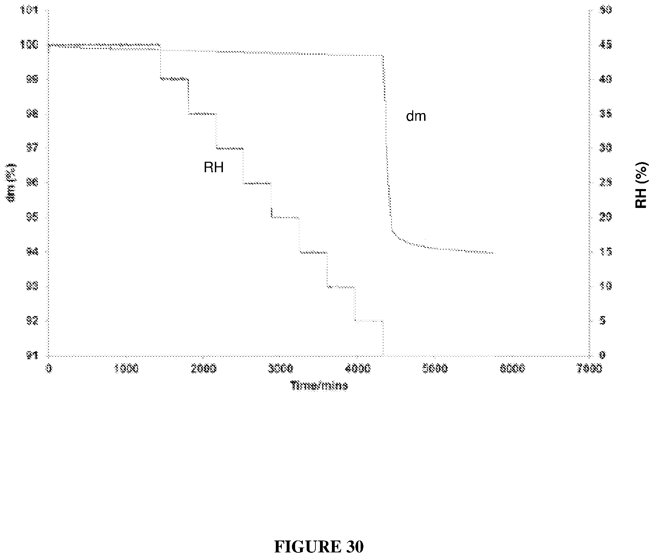

FIGURE 29 shows results obtained by thermogravimetric analysis (TGA) for a highly crystalline sample of Form A of Compound I'. The TGA results show that the highly crystalline sample of Form A of Compound I' exhibited a 5.3% weight loss when carried from 34 °C to 140 °C, and a 0.71% weight loss from 140 °C to 238 °C. The highly crystalline sample does not begin to show appreciable water loss until heated above 100 °C. Meanwhile, FIGURE 30 shows dynamic vapor sorption (DVS) results for a highly crystalline sample Form A of Compound I', and shows that the sample resists gain/loss of water over a relative humidity range of 5% to 75%.

### Methods of Crystallizing Compound I'

Disclosed are methods of crystallizing Compound I' which are not claimed but are useful for understanding the invention. Crystalline forms of Compound I' may generally be obtained or produced by crystallizing Compound I' under controlled conditions. In some embodiments, the method may produce a solvated crystalline form. In some embodiments, the method may produce the crystalline Form A of Compound I'.

Controlling the presence of oxygen may be important in the formation of the desired crystalline form. In some embodiments, crystallization may be performed in a reactor blanketed with nitrogen. In some embodiments, the crystallization solvents used may be degassed by bubbling nitrogen through the solvents for a suitable period of time.

In some embodiments, crystallization of Compound I' may be performed by adding an aqueous solution of Compound I' to an antisolvent to form a crystallization solution. In some embodiments, the aqueous solution of Compound I' may be added to the antisolvent over a period of 30 minutes, 40 minutes, 50 minutes, 60 minutes, 70 minutes, 80 minutes, 90 minutes, 100 minutes, 110 minutes or 120 minutes or a range between any two of these values. In certain embodiments, the aqueous solution of Compound I' may be added to the antisolvent while stirring.

In some embodiments, the antisolvent may be selected from acetone, acetonitrile, benzene , 1-butanol , 2-butanol, methyl ethyl ketone (MEK), t-butyl alcohol , chloroform, cyclohexane, 1,2-dichloroethane , diethylene glycol, diethyl ether, diglyme (diethylene glycol dimethyl ether), 1,2-dimethoxyethane (glyme, DME), dimethylformamide (DMF), dimethyl sulfoxide (DMSO), 1,4-dioxane, ethanol, ethyl acetate, ethylene glycol, heptane, hexamethylphosphoramide (HMPA), hexamethylphosphorous triamide (HMPT), hexanes, methanol, methyl t-butyl ether (MTBE), methylene chloride, N-methyl-2-pyrrolidinone (NMP), pentane, 1-propanol, 2-propanol, pyridine, tetrahydrofuran (THF), toluene, triethyl amine, o-xylene, m-xylene, p-xylene, or a combination thereof. In some embodiments, the antisolvent may be methyl ethyl ketone.

Temperature control may also be advantageous for crystallization. For example, in some embodiments, upon addition of the aqueous solution of Compound I' to the antisolvent, it is desirable to maintain the crystallization solution at a maximum temperature of about 50 °C. In some embodiments, the temperature of the crystallization solution is from about 5 °C to about 50 °C, from about about 10 °C to about 50 °C, from about 15 °C to about 50 °C, from about 20 °C to about 50 °C, from about 25 °C to about 50 °C, from about 30 °C to about 50 °C, from about about 35 °C to about 50 °C, from about about 40 °C to about 50 °C, or from about 45 °C to about 50 °C. For instance, in some embodiments, the crystallization at 25 °C, 30 °C, 35 °C, 40 °C, 45 °C, or 50 °C, or a range between any two of these values. In some embodiments, the crystallization solution may be maintained at the desired temperature for 0.5 hour, 1 hour, 2 hours, 3 hours, 4 hours, 5 hours, 6 hours, 7 hours, 8 hours, 9 hours, 10 hours, or more, or a range between any two of these values.

Controlling the amount of water present in the crystallization solution may be advantageous for preparing the monohydrate of Compound I'. In some embodiments, the aqueous solution of Compound I' is added to the antisolvent to form a crystallization solution such that the water content of the crystallization solution is up to 5% by weight of the antisolvent. In some embodiments, the water content may be 0.5%, 1.0%, 1.5%, 2.0%, 2.5%, 3.0%, 3.5%, 4.0%, 4.5%, 5.0% by weight relative to the quantity antisolvent or within a range defined by any of the aforementioned values. For example, the crystallization solution may comprise from 0.5% to 5.0%, from 1.0% to 5.0%, from 1.5% to 5.0%, from 2.0% to 5.0%, from 2.5% to 5.0%, from 2.5% to 4.5%, from 3.0% to 4.5%, or from 2.0% to 4.0% water by weight relative to the quantity of antisolvent.

Solid crystalline Compound I' may be recovered from the crystallization solution by filtration followed by washing with a solution of methyl ethyl ketone containing 3% (w/w) water and then subsequently drying at reduced pressure. In some embodiments, the drying is performed at a temperature no greater than 50 °C. For example, the drying may be performed at 25 °C, 30 °C, 35 °C, 40 °C, 45 °C, or 50 °C. In some embodiments, the drying is performed at a pressure no lower than 100 mbar. For example, the drying may be performed at 100 mbar, 150 mbar, 200 mbar, 250 mbar, 300 mbar, 350 mbar, 400 mbar, 450 mbar, or 500 mbar, or at a pressure within a range between any of the aforementioned values.

### Methods of Preparing Compound I

Also provided herein is an enantioselective method of making Compound I, or pharmaceutically acceptable salt thereof, 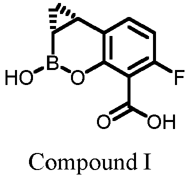 comprising the steps of: borylating a compound of Formula (A-I) 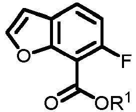 with a borylating agent in the presence of a nickel catalyst and a base system to form an organoboron intermediate of Formula (A-II) 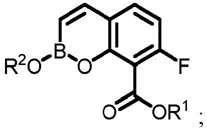 reacting organoboron intermediate of Formula (A-II) with a chiral auxiliary having the formula 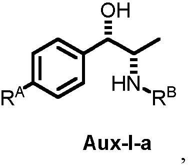 wherein R^{A} is hydrogen or C₁-C₆ alkyl; and R^{B} is C₁-C₆ alkyl; to form organoboron intermediate of Formula (B-III) 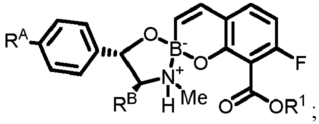 converting organoboron intermediate of Formula (B-III) to organoboron intermediate of Formula (B-IV) 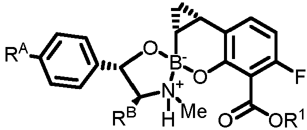 in the presence of a cyclopropanating agent and optional catalyst; and
hydrolyzing the compound of Formula (B-IV) to form the disodium salt of compound I (*i.e*., compound I'). In some embodiments, the compound of Formula (B-IV) may be isolated prior to hydrolysis to form compound I'. The preparation of the compounds of Formula (A-I) are described in International Patent Publication No. WO 2019/075084.

In some embodiments, the chiral auxiliary is (1S,2S)-(+)-pseudoephedrine. In some embodiments, the chiral auxiliary is not (1S,2S)-(+)-pseudoephedrine.

R¹ is a carboxylic acid protecting group. In some embodiments, R¹ is C₁₋₆alkyl. For example, in some specific embodiments, R¹ can be, without limitation, methyl, ethyl, n-propyl, isopropyl, n-butyl, sec-butyl, or t-butyl. In some specific embodiments, R¹ is ethyl.

In some embodiments, R² is hydrogen. In some embodiments, R² is C₁₋₆alkyl. In some embodiments, R² is substituted C₁₋₆alkyl. For example, in some specific embodiments, R² can be, without limitation, methyl, ethyl, n-propyl, isopropyl, n-butyl, sec-butyl, t-butyl, or pinacol. In some embodiments, R² is hydrogen. In some embodiments, R² is pinacol

The borylating agent can be any borylating agent suitable for introducing the boron atom into a furan ring. In some embodiments, the borylating agent is (R²O)₂B-B(OR²)₂, wherein each R² is independently H, optionally substituted C₁₋₆alkyl, or two R² together are an optionally substituted C₂₋₄ alkylene chain and form an optionally substituted 5-7 membered heterocyclyl ring with the intervening atoms. In some embodiments, the borylating agent is (HO)₂B-B(OH)₂. In some embodiments, the borylating agent is selected from the group consisting of B₂(Pin)₂ (Bis(pinacolato)diboron), B₂(Cat)₂ (Bis(catecholato)diboron), and B₂neop₂ (Bis(neopentyl glycolato)diboron). In some embodiments, the borylating agent is B₂(Pin)₂.

The molar ratio of the borylating agent to the compound of Formula (A-I) can depend on the reaction conditions used. In some embodiments, the molar ratio of the borylating agent to the compound of Formula (A-I) can be in the range of about 1.0:1 to about 3:1. In some embodiments, the molar ratio of the borylating agent to the compound of Formula (A-I) can be about 1.0:1, 1.05:1, 1.10:1, 1.15:1, 1.20:1, 1.25:1, 1.3:1, 1.35:1, 1.4:1, 1.45:1, 1.5:1, 1.6:1, 1.7:1, 1.8:1, 1.9:1, 2.0:1, 2.1:1, 2.2:1, 2.3:1, 2.4:1, 2.5:1, 2.6:1, 2.7:1, 2.8:1, 2.9:1 or 3:1. In some embodiments, the molar ratio of the borylating agent to the compound of Formula (A-I) can be less than 1.1:1, 1.2:1, 1.3:1, 1.4:1, or 1.5:1.

In some embodiments, the nickel catalyst precursor and ligand are combined to form the nickel catalyst prior to reacting the compound of Formula (A-I) with a borylating agent in a solvent system and a base system. In other embodiments, the nickel catalyst precursor and ligand combine to form the nickel catalyst while in the presence of compound of Formula (A-I) with a borylating agent in a solvent system and a base system.

In some embodiments, Ni catalyst precursor is selected from the group consisting of NiCl₂, Ni(acac)₂, and Ni(COD)₂. In some embodiments, the Ni catalyst precursor is NiCl₂ or Ni(Acac)₂. In some embodiments, the Ni catalyst precursor is NiCl₂.

In some embodiments, the amount of Ni catalyst precursor is in the range of about 5 mol% to about 25 mol% of the compound of formula (A-I). In some embodiments, the amount of Ni catalyst precursor is about 2.5mol%, 5mol%, 10mol%, 15mol%, 20mol%, 25mol%, 30mol%, 40mol%, 50mol% of the compound of formula (A-I). In some embodiments, the amount of Ni catalyst precursor is greater than about 1mol%, 5mol%, 10mol%, 15mol%, 20mol%, 25mol%, 30mol%, 40mol%, 50mol% of the compound of formula (A-I). In some embodiments, the amount of Ni catalyst precursor is less than about 5mol%, 10mol%, 15mol%, 20mol%, 25mol%, 30mol%, 40mol%, 50mol% of the compound of formula (A-I). In some embodiments, the amount of Ni catalyst precursor is in the range of about 1 mol% to about 20 mol%, about 1mol% to about 30 mol%, about 5 mol% to about 20 mol%, about 5 mol% to 30 mol%, about 5 mol% to about 40 mol%, about 5mol% to about 50mol% of the compound of formula (A-I).

In some embodiments, the ligand is monodentate or bidentate. In some embodiments, the ligand is selected from the group consisting of aliphatic, aromatic, NHC ligands, phosphites, phosphoramidites, and amines. In some embodiments, the ligand is selected from the group consisting of NHC ligands, phosphines, phosphites, phosphoramidites, amines, alcohol, aminoalcohol, and a combination thereof. In some embodiments, the ligand is an amine optionally substituted with aryl, alkyl, and/or heteroaryl groups. In some embodiments, the ligand is NHC ligand. In some embodiments, the ligand is phosphines. In some embodiments, the ligand is phosphites. In some embodiments, the ligand is phosphoramidites. In some embodiments, the ligand is amine. In some embodiments, the ligand is alcohol. In some embodiments, the ligand is aminoalcohol. In some embodiments, the ligand or catalyst is selected from the group consisting of Bis(di-cyclopentylphosphnium)ethyl tetrafluoroborate, 4,5-Bis(di-tert-butylphosphino)-9,9-dimethylxanthene, 2,2'-bis(dicyclohexylphosphaneyl)-1,1'-biphenyl, 1,2-bis((di-tert-butylphosphaneyl)methyl)benzene, 1,3-Bis(1-adamanthyl)imidazolium, 1,3-Di-tert-butylimidazolium, 1,3-Bis(2,6-diisopropyl-phenyl)-4,5-dihydroimidazol-2-ylidene, 1,3-bis(2,6-diisopropylphenyl)-1H-imidazol-3-ium-2-ide, P(Octyl)₃, Dppf, DiPrf, dcype, JosiPhos 9-1, (S,S,R,R)-TangPhos, (S,S,R,R)-DuanPhos, DavePhos, P(tBu)₃, P(*n*-Bu)₃, P(*n*-Pr)₃, XantPhos, (tBu)XantPhos, (R)-SegPhos, (R)-DM-SegPhos, (R)-MeOBIPHEP, (R,S)-BinaPhos, Binaphane, Phosphoramidite, (S)-SegphosRu(Oac)₂, trans-PdCl₂(Pcy₃)₂, [Rh(S,S)EtDuPhos(COD)]Otf, (S)-XylylPhanePhos, (R)-C3-TunePhos, (R)-DTBM-Garphos, (R)-DMM-Garphos, (R,R,R)-Xyl-SKP, Thio-XantPhos, TaniaPhos, SPANPhos, tris(4-methoxyphenyl)phosphine, Tris(2,6-dimethoxyphenyl) phosphine, Bis(dicyclohexylphosphino) methane), DCYPE, 1,3-Bis(dicyclohexyl phosphino)propane, 1,2-Bis(diphenylphosphino)ethane, (R,R)- Dipamp, Bis(dicyclohexyl phosphinophenyl) ether, DPEPhos, Bis(2-diphenylphosphinoethyl) phenylphosphine, 1,1,1-Tris(diphenyl phosphinomethyl)ethane, DPPF, 1,1'-Ferrocenediyl-bis(dicyclohexylphosphine), DTBPF, DiPrF, 1-Diphenylphosphino-1'-(di-butylphosphino)ferrocene, HiersoPhos, iPr(NHC), SIMes, IMes, (1,3-bis[bis(o-methoxyphenyl)phosphino]propane and P(*n*-Bu)₃)₂. In some embodiments, the ligand is P(Octyl)₃, P(*n*-Pr)₃, P(*n*-Bu)₃, diPrf, or dcype. In some embodiments, the ligand is P(*n*-Bu)₃. In some embodiments, the ligand is P(*n*-Pr)₃.

The molar ratio of the ligand to the Ni catalyst precursor can depend on the types of precursor and ligand used. In some embodiments, the molar ratio of the ligand to the Ni catalyst precursor can be in the range of about 0.5:1 to about 5:1. In some embodiments, the molar ratio of the ligand to the Ni catalyst precursor can be about 0.5:1, 1:1, 1.5:1, 2:1, 2.5:1, or 5:1. In some embodiments, the molar ratio of the ligand to the Ni catalyst precursor can be in the range of about 0.1:1-10:1, 0.5:1-5:1, 0.5:1-3:1, 0.5:1-2.5:1, 1:1-2:1, 1:1-3:1, 1:1-4:1, 1:1-5:1, or 1:1-2.5:1. In some embodiments, the molar ratio of the ligand to the Ni catalyst precursor can be greater than about 0.1:1, 0.5:1, 1:1, 1.25:1, 1.5:1, 1.75:1, 2:1, 3:1, 4:1, 5:1, 6:1, or 10:1. In some embodiments, the molar ratio of the ligand to the Ni catalyst precursor can be less than 0.5:1, 1:1, 1.25:1, 1.5:1, 1.75:1, 2:1, 3:1, 4:1, 5:1, 6:1, 10:1, or 20:1.

In some embodiments, the nickel catalyst can be selected from the group consisting of: NiCl₂(PPh₃)₂, NiCl₂(PCy₂Ph)₂, NiCl₂(PPh₂CH₂CH₂PPh₂), NiCl₂(1,3-Bis(diphenylphosphino)propane), NiCl₂(1,3-Bis(diphenylphosphino)ethane) and NiCl₂(P(*n*-Bu)₃)₂. In some embodiments, the nickel catalyst may be pre-formed prior to reation with the compound of formula (A-I)

The reaction of the compound of formula (A-I) and the borylating agent is performed in the presence of a base system. The base system comprises one or more inorganic bases. In some embodiments, the base system comprises K₂CO₃, Cs₂CO₃, or any combination thereof. In some embodiments, the base system comprises Cs₂CO₃. In some embodiments, the base system comprises CsOH·H₂O. In some embodiments, the base system comprises K₂CO₃. In the claims, the molar ratio of base system to the compound of formula (A-I) is from 1:1 to 2:1. In some embodiments, the molar ratio of the base system to the compound of formula (A-I) is about 1:1, 1.5:1, or 2:1 In some embodiments, the molar ratio of the base system to the compound of formula (A-I) can be in less than 1.5:1, or less than 1.25:1.

In some embodiments the carbonate base can be a mixture of Cs₂CO₃ and K₂CO₃. In some embodiments the cesium to potassium carbonate base ratio may be from about 1:2 to 2:1. In some embodiments the cesium to potassium carbonate base ratio may be from 1.5:1, 1.6:1, 1.7:1 or 1.8:1.

In some embodiments, the borylation reaction of the compound of formula (A-I) and a borylating agent is performed using a catalyst system comprising NiCl₂ and P(*n*-Bu)₃ in the presence of Cs₂CO₃. In some specific embodiments, the molar ratio of of Cs₂CO₃ to the compound of formula (A-I) is from about 1:1 to about 5:1. For example, in some embodiments, the molar ratio of Cs₂CO₃ to the compound of formula (A-I) is about 1:1, 1.5:1, 2:1, 2.5:1, 3:1, 3.5:1, 4:1, 4.5:1 or 5:1. In some embodiments, the molar ratio of Cs₂CO₃ to the compound of formula (A-I) can be in the range of about 1:1 to 5:1, 1.5:1 to 5:1, 2:1 to 4:1, 2.5:1 to 3.5:1 or 3:1 to 5:1. In some embodiments, the borylation reaction of the compound of formula (A-I) and a borylating agent is performed using a catalyst system comprising NiCl₂ and P(n-Bu)₃ in the presence of CsOH·H₂O. In some specific embodiments, the molar ratio of CsOH·H₂O to the compound of formula (A-I) is from about 1:1 to about 5:1. For example, in some embodiments, the the molar ratio of CsOH·H₂O to the compound of formula (A-I) is about 1:1, 1.5:1, 2:1, 2.5:1, 3:1, 3.5:1, 4:1, 4.5:1 or 5:1. In some embodiments, the molar ratio of CsOH·H₂O to the compound of formula (A-I) can be in the range of about 1:1 to 5:1, 1:1 to 4:1, 1:1 to 3:1, 1:1 to 2:1.

In some embodiments, the reaction of the compound of formula (A-I) and the borylating agent is performed in a solvent system. In some embodiments, the solvent system comprises tetrahydrofuran (THF). In some embodiments, the solvent system comprises 2-methyltetrahydrofuran (MeTHF).

In some embodiments, the solvent system may further comprise water. In some embodiments, water may be present in 1, 2, 3, 4, 5, 6, 7, 8, 9, 10, 15, 20, 25, 30, 35, 40, 45, or 50 % v/v. In some embodiments, the solvent system may comprise water in the range of about 1% to 10% v/v, 2% to 8% v/v 4% to 7% v/v, 3% to 8% v/v, 5% to 10% v/v or 5% to 15% v/v. In some specific embodiments, the water may be present in 5% v/v. In other embodiments, the solvent system may further comprise ethanol. In some embodiments, ethanol may be present in 1, 2, 3, 4, 5, 6, 7, 8, 9, 10, 15, 20, 25, 30, 35, 40, 45, or 50 % v/v. In some embodiments, the solvent system may comprise ethanol in the range of about 1% to 10% v/v, 2% to 8% v/v, 4% to 7% v/v, 3% to 8% v/v, 5% to 10% v/v or 5% to 15% v/v. In some specific embodiments, ethanol may be present in 5% v/v.

In some embodiments, the solvent system may be THF. In some embodiments, the solvent system may further comprise about 1% to 10% v/v, 2% to 8% v/v 4% to 7% v/v, 3% to 8% v/v, 5% to 10% v/v or 5% to 15% v/v H₂O. In some embodiments, H₂O may be present in 1, 2, 3, 4, 5, 6, 7, 8, 9, 10, 15, 20, 25, 30, 35, 40, 45, or 50 %. In some embodiments, the solvent system may be 99/1, 98/2, 97/3, 96/4 95/5 94/6, 93/7, 92/8, 91/9 or 90/10 %v/v THF/H₂O. In some embodiments, the solvent system may be THF/H₂O 95/5 %v/v. In some embodiments, the solvent system may comprise about 1% to 10% v/v, 2% to 8% v/v 4% to 7% v/v, 3% to 8% v/v, 5% to 10% v/v or 5% to 15% v/v ethanol. In some embodiments, ethanol may be present in 1, 2, 3, 4, 5, 6, 7, 8, 9, 10, 15, 20, 25, 30, 35, 40, 45, or 50%. In some embodiments, the solvent system may be 99/1, 98/2, 97/3, 96/4 95/5 94/6, 93/7, 92/8, 91/9 or 90/10 %v/v THF/ethanol. In some embodiments, the solvent system may be THF/ethanol 95/5 %v/v. In some embodiments, the solvent system may be ethanol.

In some embodiments, the solvent system may be 2-methyltetrahydrofuran (MeTHF). In some embodiments, the solvent system may further comprise about 1% to 10% v/v, 2% to 8% v/v 4% to 7% v/v, 3% to 8% v/v, 5% to 10% v/v or 5% to 15% v/v H₂O. In some embodiments, H₂O may be present in 1, 2, 3, 4, 5, 6, 7, 8, 9, 10, 15, 20, 25, 30, 35, 40, 45, or 50 %. In some embodiments, the solvent system may be 99/1, 98/2, 97/3, 96/4 95/5 94/6, 93/7, 92/8, 91/9 or 90/10 %v/v MeTHF/H₂O. In some embodiments, the solvent system may be MeTHF/H₂O 95/5 %v/v. In some embodiments, the solvent system may comprise about 1% to 10% v/v, 2% to 8% v/v 4% to 7% v/v, 3% to 8% v/v, 5% to 10% v/v or 5% to 15% v/v ethanol. In some embodiments, ethanol may be present in 1, 2, 3, 4, 5, 6, 7, 8, 9, 10, 15, 20, 25, 30, 35, 40, 45, or 50%. In some embodiments, the solvent system may be 99/1, 98/2, 97/3, 96/4 95/5 94/6, 93/7, 92/8, 91/9 or 90/10 %v/v MeTHF/ethanol. In some embodiments, the solvent system may be MeTHF/ethanol 95/5 %v/v. In some embodiments, the solvent system may be ethanol.

In some embodiments, the borylation reaction of the compound of formula (A-I) may include a step of premixing the borylating agent and the base.

In some embodiments, the borylation reaction of the compound of formula (A-I) may be performed at a temperature in the range of about 60°C to 100°C. In some embodiments, the borylation reaction of the compound of formula (A-I) may performed at a temperature of about 60, 65, 70, 75, 80, 85, 90, 95, or 100 °C. In some embodiments, the borylation reaction of the compound of formula (A-I) is performed at a temperature in the range of about 60 to 100 °C, 70 to 100 °C, 80 to 100 °C, 90 to 100 °C, 60 to 70 °C, 60 to 80 °C, 60 to 90 °C, 65 to 85 °C, 65 to 90 °C, or 75 to 95 °C.

In some embodiments, the organoboron intermediate of formula (A-II) is combined with one or more chiral auxiliaries to form a chiral complex. In some embodiments, the chiral complex has a structure of formula (B-III). In some embodiments, the chiral auxiliary is (1S,2S)-(+)-pseudoephedrine so as to form a chiral complex having the structure of Formula (A-III).

In some embodiments, the chiral auxiliary is a compound of Formula (Aux-I) 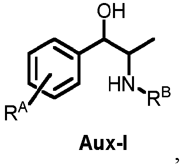 or stereoisomers thereof,
wherein
R^{A} may be hydrogen, halogen, or C₁-C₆ alkyl; and
R¹ may C₁-C₆ alkyl;

In some embodiments, the chiral auxiliary is a compound of Formula Aux-I-a: 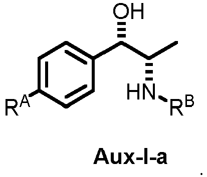 In some embodiments, the chiral auxiliary is a compound of Formula Aux-I-b: 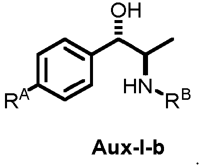 In some embodiments, the chiral auxiliary is a compound of Formula Aux-I-c: 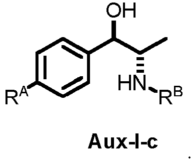 In some embodiments, the chiral auxiliary is a compound of Formula Aux-I-d:

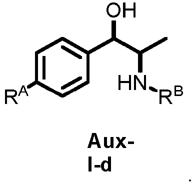

In some embodiments, R^{A} may be hydrogen; in some embodiments, R^{A} may be methyl.

In some embodiments, R^{A} may not be methyl.

In some embodiments, R^{B} may be hydrogen. In other embodiments, R^{B} may be methyl. In yet other embodiments, R^{B} may be ethyl. In some embodiments, R^{B} may be isopropyl.

In some embodiments, R^{A} may be methyl and R^{B} may be methyl. In other embodiments, R^{A} may be methyl and R^{B} may be ethyl.

In some embodiments, the chiral auxiliary is (1S,2S)-(+)-pseudoephedrine. In some embodiments, the chiral auxiliary is not (1S,2S)-(+)-pseudoephedrine. In other emodiments, the chiral auxiliary is (1S,2S)-2-(methylamino)-1-(p-tolyl)propan-1-ol. In other embodiments, the chiral auxiliary may be (1S,2S)-2-(ethylamino)-1-(p-tolyl)propan-1-ol. In some embodiments, the chiral auxiliary may be (1S,2S)-2-(isopropylamino)-1-(p-tolyl)propan-1-ol. In some embodiments, the chiral auxiliary may be (1S,2R)-2-(methylamino)-1-phenylpropan-1-ol. In other embodiments, the chiral auxiliary may be (1R,2S)-2-(methylamino)-1-(p-tolyl)propan-1-ol. In yet other embodiments, chiral auxiliary may be (1R,2S)-2-(ethylamino)-1-(p-tolyl)propan-1-ol. In some embodiments, the chiral auxiliary may be (1R,2S)-2-(isopropylamino)-1-(p-tolyl)propan-1-ol.

Some embodiments relate to a compound having the structure of Formula (B-III). In some embodiments, the compound of Formula (B-III) has the structre of Formula (A-III). In some embodiments, the compound of Formula (A-III) has the structure of compound A-3. In some embodiments, the compound of Formula (B-III) is 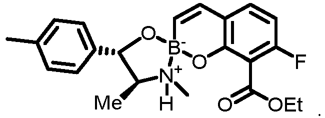 In other embodiments, the compound of Formula (B-III) is

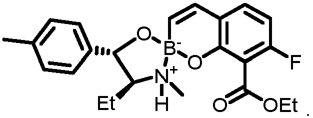

In some embodiments, the chiral complex of formula (A-III) can be reacted with a cyclopropanating agent to introduce a cyclopropyl group to form a compound of Formula (A-IV). In some embodiments, the cyclopropyl group is introduced by reacting the compound of formula (A-III) with a carbenoid or with diazomethane in the presence of a second catalyst. In some embodiments, the cyclopropyl group is introduced by reacting the compound of formula (A-III) with diazomethane. In some embodiments, the introduction of the cyclopropyl group in the compound of formula (A-III) is performed in the presence of a second catalyst. In some embodiments, the cyclopropyl group is introduced by reacting the compound of formula (A-III) with a cyclopropanating reagent comprising CH₂I₂ or CH₂Br₂; and one or more metal compounds. In some embodiments, the cyclopropanating reagent comprises CH₂I₂. In other embodiments, the cyclopropylating reagent comprises CH₂Br₂. In some embodiments, the metal compound comprises one or more metals selected from the group consisting of zinc, copper, samarium, aluminum, and silver. In some specific embodiments, the metal compound may be Zn/Cu couple, Zn/Ag couple, Sm/Hg couple, ZnEt₂, or a mixture of zinc and CuCl. In some embodiments, the cyclopropyl group is introduced by reacting the compound of formula (A-III) with CH₂I₂ and Zn/Cu couple. In other embodiments, the cyclopropyl group is introduced by reacting the compound of formula (A-III) with CH₂Br₂, Zn, and CuCl. In yet other embodiments, the cyclopropyl group is introduced by reacting the compound of formula (A-III) with CH₂I₂ and diethylzinc. In some embodiments, the cyclopropyl group is introduced using diazomethane. In some embodiments, the cyclopropyl group is introduced using diazomethane and a palladium catalyst. In some embodiments, the cyclopropyl group is introduced using diazomethane and palladium (II) acetate. In some embodiments, the cyclopropyl group is introduced using diazomethane and Pd₂(DVTMS)₃. In some embodiments, the cyclopropyl group is introduced using diazomethane and Pd(acac)₂. In some embodiments, the cyclopropyl group is introduced using diazomethane and Pd(TFA)₂. In some embodiments, the cyclopropyl group is introduced using diazomethane and Pd(OPiv)₂.

In some embodiments, the compound of Formula (B-III) undergoes a cyclopropanation reaction to form a compound of Formula (B-IV). In some embodiments, the cyclopropyl group is introduced by reacting the compound of formula (B-III) with a carbenoid or with diazomethane in the presence of a second catalyst. In some embodiments, the cyclopropyl group is introduced by reacting the compound of formula (B-III) with diazomethane. In some embodiments, the introduction of the cyclopropyl group in the compound of formula (B-III) is performed in the presence of a second catalyst. In some embodiments, the cyclopropyl group is introduced by reacting the compound of formula (B-III) with a cyclopropanating reagent comprising CH₂I₂ or CH₂Br₂; and one or more metal compounds. In some embodiments, the cyclopropanating reagent comprises CH₂I₂. In other embodiments, the cyclopropylating reagent comprises CH₂Br₂. In some embodiments, the metal compound comprises one or more metals selected from the group consisting of zinc, copper, samarium, aluminum, and silver. In some specific embodiments, the metal compound may be Zn/Cu couple, Zn/Ag couple, Sm/Hg couple, ZnEt₂, or a mixture of zinc and CuCl. In some embodiments, the cyclopropyl group is introduced by reacting the compound of formula (A-III) with CH₂I₂ and Zn/Cu couple. In other embodiments, the cyclopropyl group is introduced by reacting the compound of formula (B-III) with CH₂Br₂, Zn, and CuCl. In yet other embodiments, the cyclopropyl group is introduced by reacting the compound of formula (B-III) with CH₂I₂ and diethylzinc. In some embodiments, the cyclopropyl group is introduced using diazomethane. In some embodiments, the cyclopropyl group is introduced using diazomethane and a palladium catalyst. In some embodiments, the cyclopropyl group is introduced using diazomethane and palladium (II) acetate. In some embodiments, the compound of Formula B-IV is 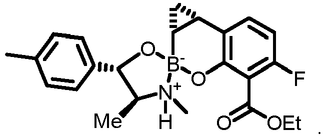 In other embodiments, the compound of Formula B-IV is

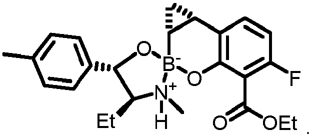

In some embodiments, the compound of formula (A-IV) can be made with an enantiomeric excess of greater than about 50%, about 60%, about 70%, about 80%, about 90%, about 95%, about 98%, about 99%. In some embodiments, the compound of formula (A-IV) can be made with an enantiomeric excess of less than about 50%, about 60%, about 70%, about 80%, about 90%, about 95%, about 98%, about 99%. In some embodiments, the compound of formula (A-IV) can be made with an enantiomeric excess in the range of about 30% - 60%, about 30% - 80%, about 30% - 90%, 40% - 60%, about 40% - 80%, about 40% - 90%, about 50% - 90%, about 30% - 99%, about 40% - 99%, or about 50% - 99%. In some embodiments, the synthesis of the compound of formula (A-IV) can be achieved with an enantiomeric excess of greater than about 50%, about 60%, about 70%, about 80%, about 90%, or about 95%.

In some embodiments, the compound of formula (B-IV) can be made with an enantiomeric excess of greater than about 50%, about 60%, about 70%, about 80%, about 90%, about 95%, about 98%, about 99%. In some embodiments, the compound of formula (B-IV) can be made with an enantiomeric excess of less than about 50%, about 60%, about 70%, about 80%, about 90%, about 95%, about 98%, about 99%. In some embodiments, the compound of formula (B-IV) can be made with an enantiomeric excess in the range of about 30% - 60%, about 30% - 80%, about 30% - 90%, 40% - 60%, about 40% - 80%, about 40% - 90%, about 50% - 90%, about 30% - 99%, about 40% - 99%, or about 50% - 99%. In some embodiments, the synthesis of the compound of formula (B-IV) can be achieved with an enantiomeric excess of greater than about 50%, about 60%, about 70%, about 80%, about 90%, or about 95%.

Scheme A below summarizes the synthetic route for compound I' described herein starting from compound (A-1), which is a compound of Formula (A-I) wherein R¹ is ethyl and the chiral auxiliary is (1S,2S)-(+)-pseudoephedrine. This reaction sequence can lead to high enantioselectivity in the cyclopropanation step, leading to Compound I' with high optical purity. In some embodiments, the reaction can be performed in an organic solvent selected from toluene, THF, MeTHF, dioxane, and any combination thereof.

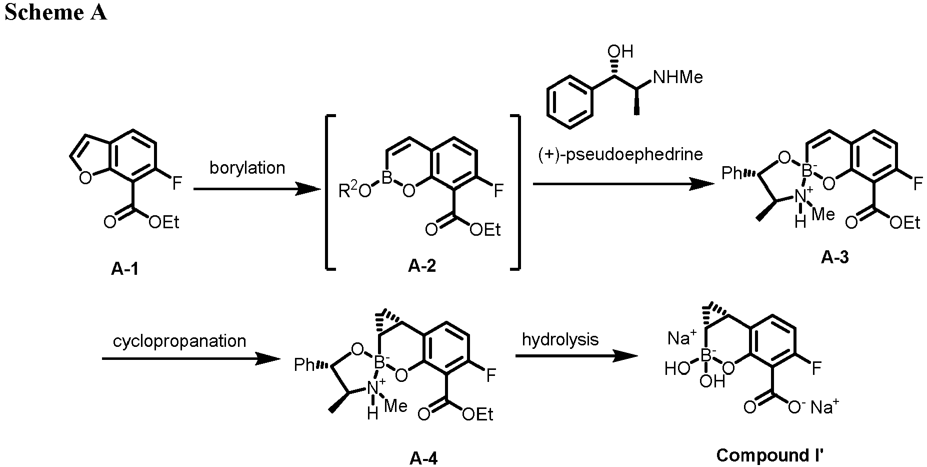

Alternatively, Compound A-3 may first be converted to Compound A-5 and then hydrolyzed to form Compound I'. Conversion of Compound A-3 to A-5 and subsequently hydrolyzing compound A-5 may result in more efficient synthesis and purification leading to improved yields and purity for Compound I'. The synthesis is showin in Scheme A' below.

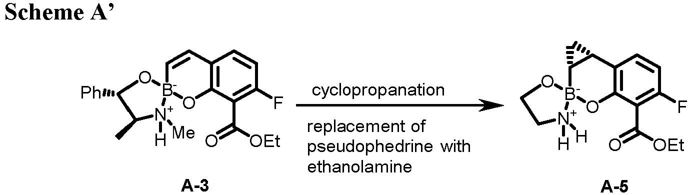

Compound A-3 may be converted to Compound A-5 using the cyclopronating conditions described herein. In some embodiments, Compound A-3 may be treated with zinc/copper couple and CH₂I₂. In other embodiments, Compound A-3 may be treated with zinc/copper couple and CH₂Br₂. In other embodiments, Compound A-3 may be treated with zinc, CuCl, and CH₂Br₂. Once cycloproanation is achieved, an organic the crude product may be washed with aqueous acid to remove pseudoephedrine and subsequently treated with ethanolamine to obtain Compound A-5.

Scheme B below summarizes the synthetic route for compound I' described herein starting from compound (A-1), which is a compound of Formula (A-I) wherein R¹ is ethyl, and the chiral auxiliary is Aux-I-a. Thus, compounds (B-III) and (B-IV) are shown as the ethyl ester. This reaction sequence can lead to high enantioselectivity in the cyclopropanation step, leading to Compound I' with high optical purity. In some embodiments, the reaction can be performed in an organic solvent selected from toluene, THF, MeTHF, dioxane, and any combination thereof. In some embodiments, compound (B-III) may alternatively be cyclopropanated and subsequently converted to compound (B-V), ultimately leading to the formation of Compound I'

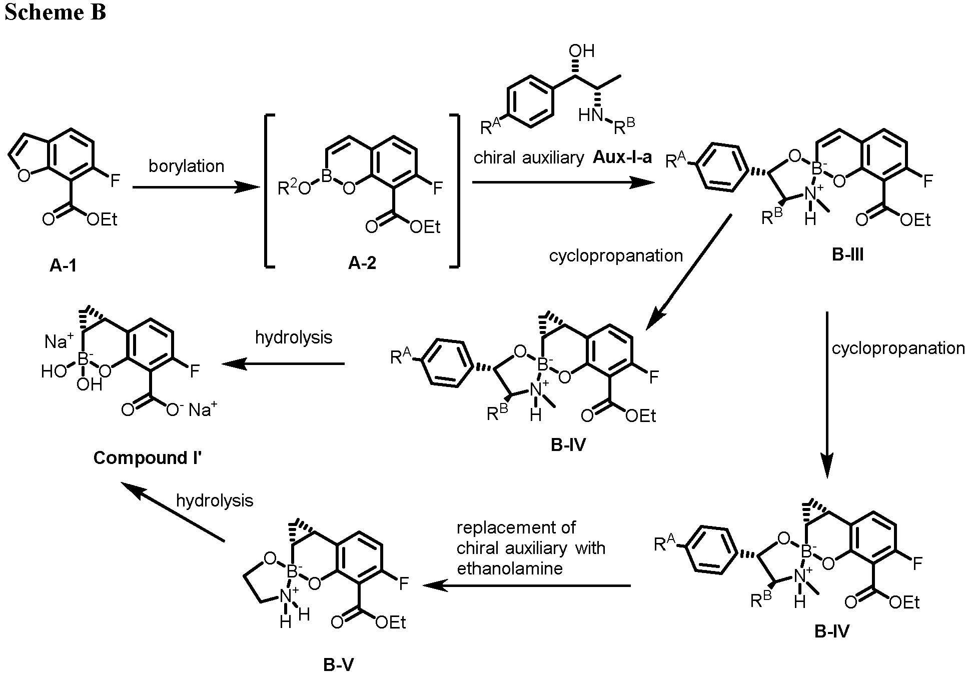

In some embodiments described herein, the compound of Formula (B-IV) or Formula (B-V) may be washed with aqueous acid prior to hydrolysis. Such treatment with aqueous acid may lead to the removal of unwanted impurities and leads to improved purity of Compound I' formed in the hydrolysis step.

Some embodiments relate to a method of making Compound I', comprising the step of: reacting a compound of formula (A-I) with a borylating agent in the presence of a nickel catalyst precursor and a base system to form a compound of Formula (A-II). In some embodiments, the compound of Formula (A-I) is compound A-1. In some embodiments, the compound of Formula (A-II) is compound A-2.

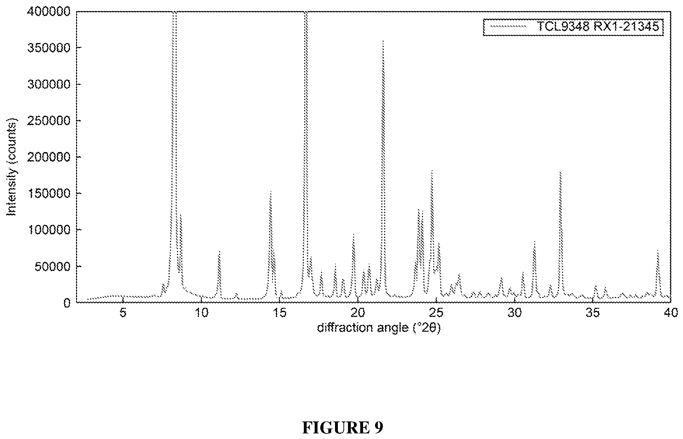

FIGURE 9 shows the crystalline structure of compound A-3 as determined by XRPD. Compound A-3 exhibits prominent peaks at approximately 7.6. 8.0, 10.4, 13.7, 14.0, 16.0, 16.3, 19.0, 20.9, 23.2, 23.4, and 24.4 degrees 2θ. In the claims, a crystalline form of compound A-3 has at least one characteristic peak (*e.g.* , one, two, three, four, five, six, seven, eight, nine, ten, eleven, or twelve characteristic peaks) selected from approximately 7.6. 8.0, 10.4, 13.7, 14.0, 16.0, 16.3, 19.0, 20.9, 23.2, 23.4, and 24.4 degrees 2θ. In some embodiments, a crystalline form of compound A03 has at least three characteristic peaks selected from approximately 7.6. 8.0, 10.4, 13.7, 14.0, 16.0, 16.3, 19.0, 20.9, 23.2, 23.4, and 24.4 degrees 2θ.

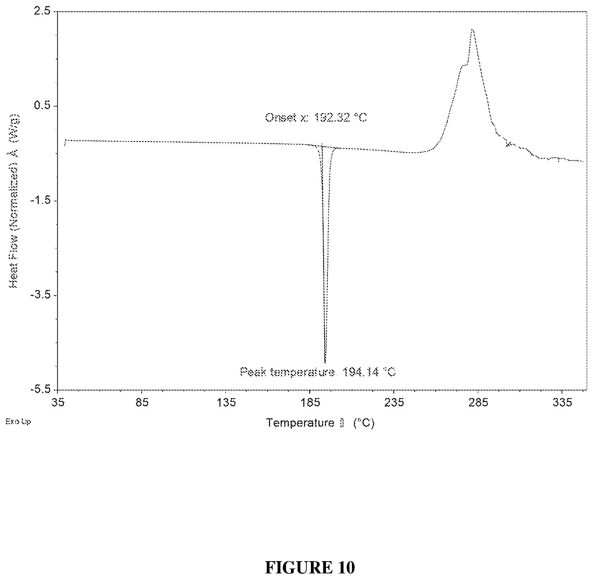

FIGURE 10 shows results obtained by DSC for crystalline compound A-3. The DSC results show a peak at temperature of about 194 °C, which indicates the melting point for the crystal. Accordingly, in some embodiments, crystalline compound A-3 exhibits a melting point from about 191 °C to about 197 °C, from about 192 °C to about 196 °C, or at about 194 °C.

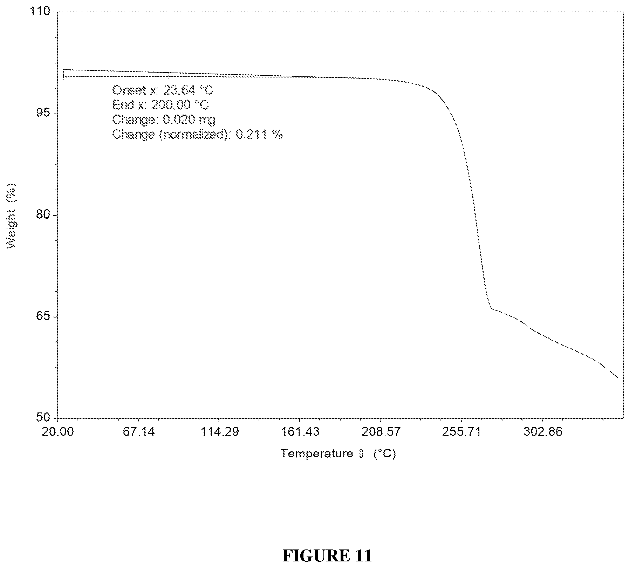

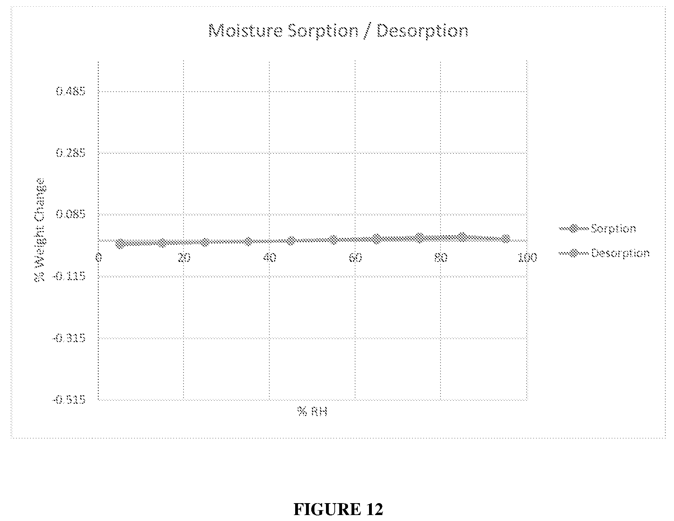

FIGURE 11 shows results obtained by TGA for crystalline compound A-3. The TGA results show that crystalline compound A-3 exhibited a 0.21% weight loss when carried from 25 °C to 350 °C. Meanwhile, FIGURE 12 shows DVS results for crystalline compound A-3, and shows no significant water uptake and indicates that crystalline compound A-3 is not hygroscopic.

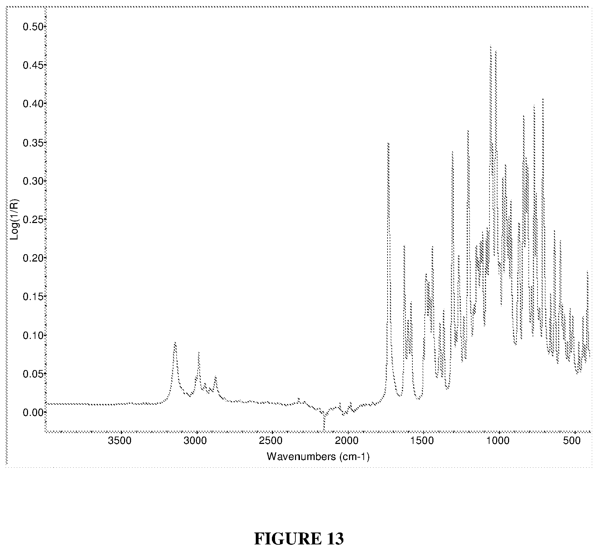

FIGURE 13 shows results obtained by Fourier Transform Infrared (FTIR) spectroscopy for crystalline compound A-3. Crystalline compound A-3 exhibits prominent peaks at approximately 3143, 2984, 2873, 1731, 1622, 1598, 1578, 1306, 1202, 1053, and 1019 cm⁻¹. Thus, in some embodiments, a crystalline form of compound A-3 has at least one characteristic FTIR peak *(e.g.,* one, two, three, four, five, six, seven, eight, nine, ten, or eleven characteristic peaks) selected from approximately 3143, 2984, 2873, 1731, 1622, 1598, 1578, 1306, 1202, 1053, and 1019 cm⁻¹. In some embodiments, a crystalline form of compound A-3 has at least three characteristic peaks selected from 3143, 2984, 2873, 1731, 1622, 1598, 1578, 1306, 1202, 1053, and 1019 cm⁻¹. In some embodiments, peak positions recited herein include variability within ± 2 cm⁻¹.

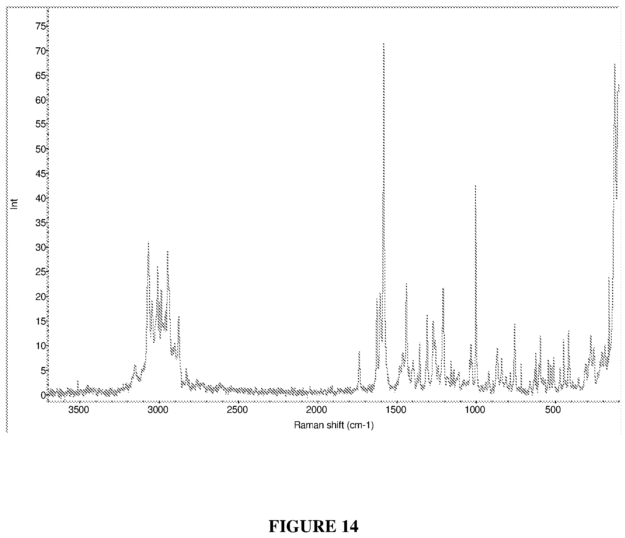

FIGURE 14 shows results obtained by Fourier Transform Raman spectroscopy for crystalline compound A-3. Crystalline compound A-3 exhibits prominent peaks at approximately 3066, 3007, 2943, 2873, 1733, 1623, 1603, 1579, and 1437 cm⁻¹. Thus, in some embodiments, a crystalline form of compound A-3 has at least one characteristic FT Raman peak *(e.g.,* one, two, three, four, five, six, seven, eight, or nine characteristic peaks) selected from approximately 3066, 3007, 2943, 2873, 1733, 1623, 1603, 1579, and 1437 cm⁻¹. In some embodiments, a crystalline form of compound A-3 has at least three characteristic peaks selected from 3066, 3007, 2943, 2873, 1733, 1623, 1603, 1579, and 1437 cm⁻¹. In some embodiments, peak positions recited herein include variability within ± 2 cm⁻¹.

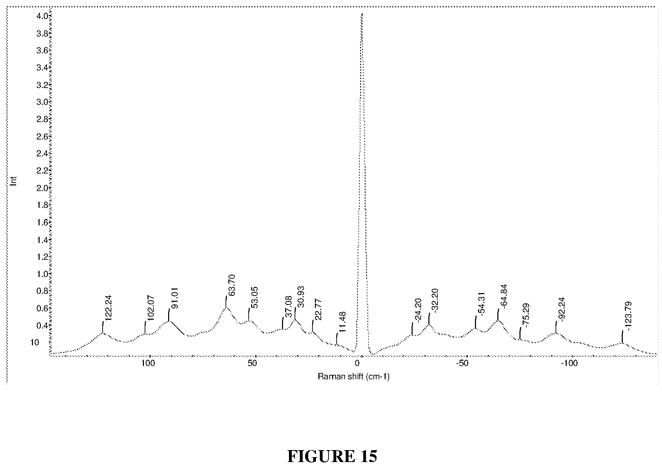

FIGURE 15 shows results obtained by Low Frequency Raman (LF Raman) spectroscopy for crystalline compound A-3. Crystalline compound A-3 exhibits prominent peaks at approximately 122, 102, 91, 64, 53, 37, 31, 23, and 11 cm⁻¹. Thus, in some embodiments, a crystalline form of compound A-3 has at least one characteristic FT Raman peak *(e.g.,* one, two, three, four, five, six, seven, eight, or nine characteristic peaks) selected from approximately 122, 102, 91, 64, 53, 37, 31, 23, and 11 cm⁻¹. In some embodiments, a crystalline form of compound A-3 has at least three characteristic peaks selected from 122, 102, 91, 64, 53, 37, 31, 23, and 11 cm⁻¹. In some embodiments, peak positions recited herein include variability within ± 2 cm⁻¹.

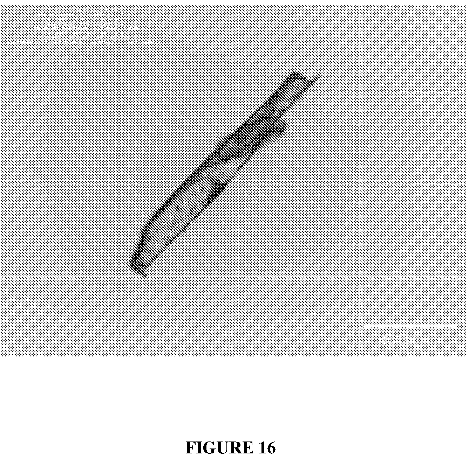

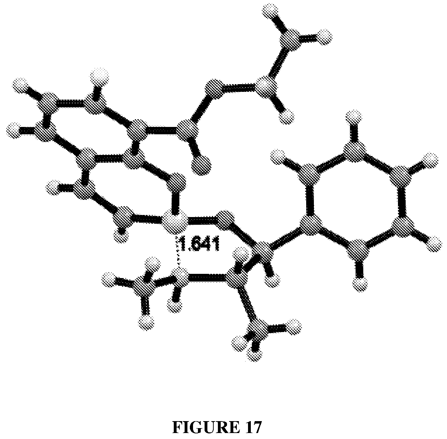

Compound A-3 can therefore be characterized as an unsolvated, non-hygroscopic solid. Compound A-3 also shows good crystallinity with large blade shaped crystals (FIGURE 16) and a relatively high melting point (approximately 194 °C). Single crystal X-ray structure determination reveals that compound A-3 has a monoclinic crystal system with a *P*2₁ space group and has the following dimensions: a (Å) = 11.191 (4), b (Å) = 7.751 (3), c (Å) =11.795 (4), β = 95.867(12) °, V = 1017.8(6) Å³. Z=2. The asymmmetic unit of the crystal structure of compound A-3 has a B-N bond distance of 1.641 Å (FIGURE 17)

Some embodiments relate to a method of making Compound I', comprising the step of: reacting a compound of Formula (A-III) with a cycloproylating agent in the presence of optional catalyst to provide a compound of Formula (A-IV). In some embodiments, the cyclopropanating agent is CH₂N₂. In some embodiments, the optional catalyst is a palladium catalyst. In some embodiments, the palladium catalyst is Pd(OAc)₂. In some embodiments, the compound of Formula (A-IV) is compound A-4.

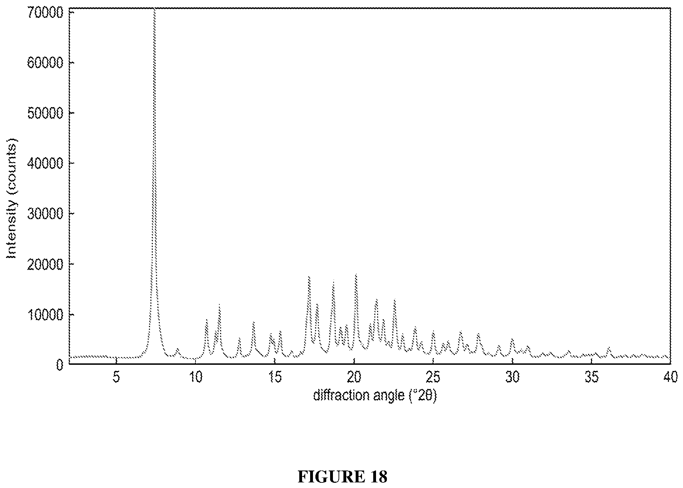

FIGURE 18 shows a crystalline structure of compound A-4 as determined by XRPD. Crystalline compound A-4 exhibits prominent peaks at approximately 7.4, 8.9, 10.7, 11.2, 11.5, 12.8, 13.7, 15.3, 17.0, 17.7, 18.7, 20.1, 21.4, and 22.6 degrees 20. Thus, in some embodiments, a crystalline form of compound A-4 has at least one characteristic peak *(e.g.,* one, two, three, four, five, six, seven, eight, nine, ten, eleven, twelve, thirteen, or fourtenn characteristic peaks) selected from approximately 7.4, 8.9, 10.7, 11.2, 11.5, 12.8, 13.7, 15.3, 17.0, 17.7, 18.7, 20.1, 21.4, and 22.6 degrees 2θ. In some embodiments, a crystalline form of compound A-4 has at least three characteristic peaks selected from approximately 7.4, 8.9, 10.7, 11.2, 11.5, 12.8, 13.7, 15.3, 17.0, 17.7, 18.7, 20.1, 21.4, and 22.6 degrees 2θ.

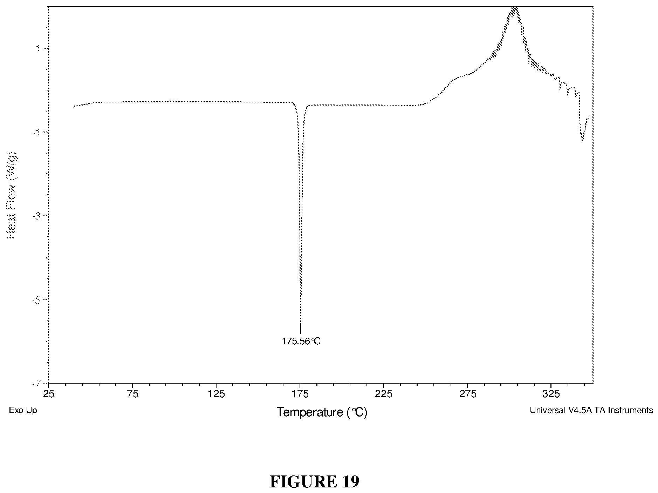

FIGURE 19 shows results obtained by DSC for crystalline compound A-4. The DSC results show a peak at temperature of about 176 °C, which indicates the melting point for the crystal. Accordingly, in some embodiments, crystalline compound A-4 exhibits a melting point from about 173 °C to about 179 °C, from about 174 °C to about 178 °C, or at about 176 °C.

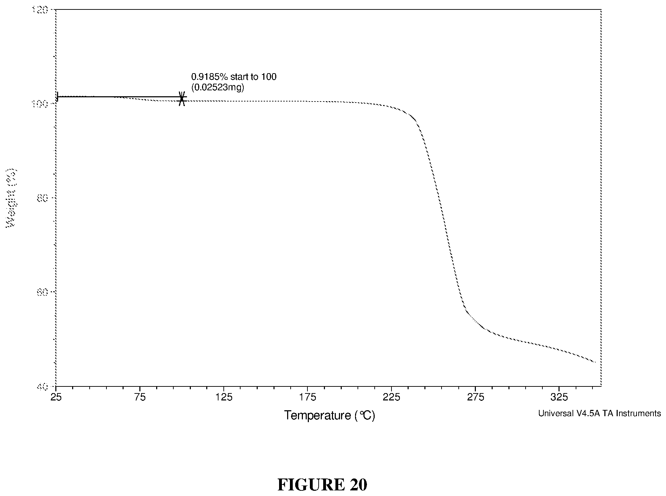

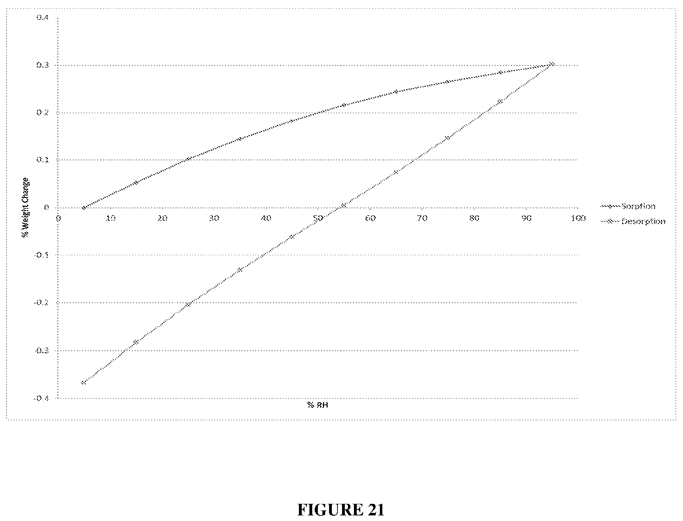

FIGURE 20 shows results obtained by TGA for crystalline compound A-4. The TGA results show that crystalline compound A-4 exhibited a 0.92% weight loss below 100 °C. FIGURE 21 shows DVS results for crystalline compound A-4, shows no significant water uptake and indicates that crystalline compound A-4 is not hygroscopic.

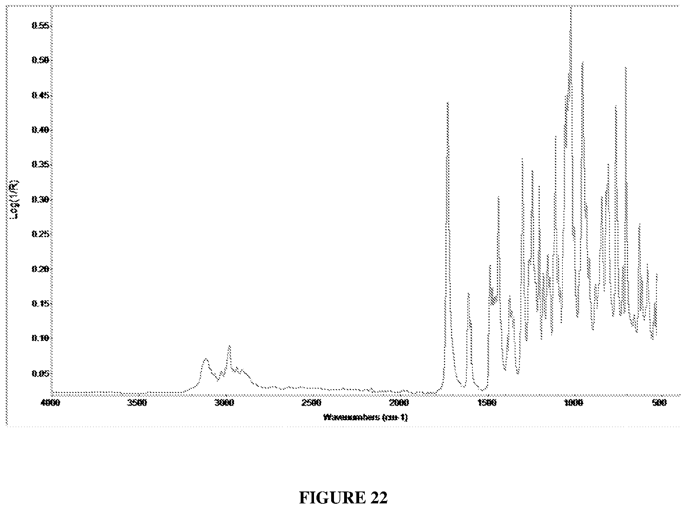

FIGURE 22 shows results obtained by Fourier Transform Infrared (FTIR) spectroscopy for crystalline compound A-4. Crystalline compound A-4 exhibits prominent peaks at approximately 1727, 1608, 1481, 1437, and 1298 cm⁻¹. Thus, in some embodiments, a crystalline form of compound A-4 has at least one characteristic FTIR peak *(e.g.,* one, two, three, four, or five characteristic peaks) selected from approximately 1727, 1608, 1481, 1437, and 1298 cm⁻¹. In some embodiments, a crystalline form of compound A-4 has at least three characteristic peaks selected from 1727, 1608, 1481, 1437, and 1298 cm⁻¹. In some embodiments, peak positions recited herein include variability within ± 1 cm⁻¹.

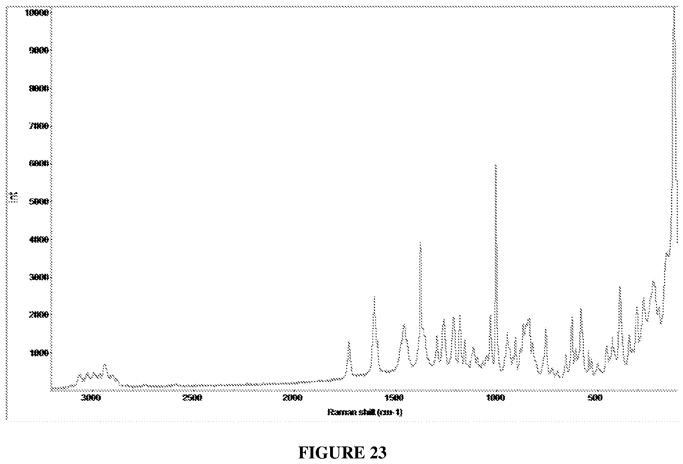

FIGURE 23 shows results obtained by Fourier Transform Raman spectroscopy for crystalline compound A-4. Crystalline compound A-4 exhibits prominent peaks at approximately 1730, 1602, 1455, and 1437 cm⁻¹. Thus, in some embodiments, a crystalline form of compound A-4 has at least one characteristic FT Raman peak *(e.g.,* one, two, three, or four characteristic peaks) selected from approximately 1730, 1602, 1455, and 1437 cm⁻¹. In some embodiments, a crystalline form of compound A-4 has at least three characteristic peaks selected from 1730, 1602, 1455, and 1437 cm⁻¹. In some embodiments, peak positions recited herein include variability within ± 2 cm⁻¹.

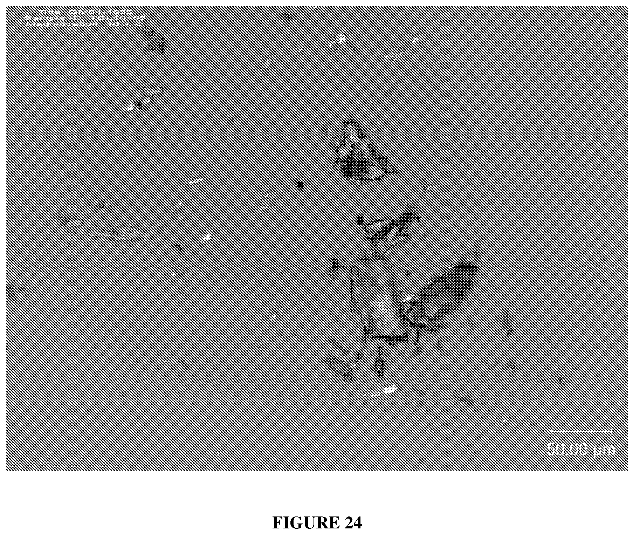

Compound A-4 can therefore be characterized as an unsolvated, non-hygroscopic solid. Compound A-4 also shows good crystallinity with blade shaped crystals of varying size and shape (FIGURE 24) and a relatively high melting point (approximately 176 °C).

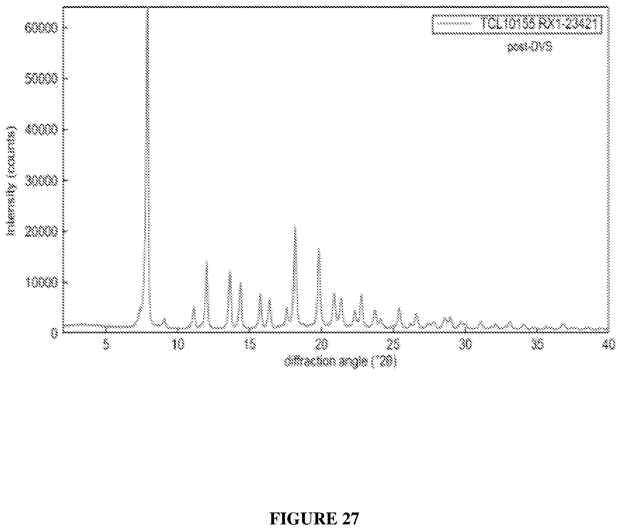

FIGURE 27 shows a crystalline structure of compound A-4 as determined by XRPD. This crystalline form was obtained upon subjecting the crystal form as characterized in FIGURE 18 to dynamic vapor sorption (DVS) analysis where the sample was exposed to 95% relative humidity (RH) and down to 5% RH. The post-DVS crystalline compound A-4 exhibits prominent peaks at approximately 7.4, 7.9, 9.1, 11.1, 12.0, 13.7, 14.4, 15.8, 16.4, 17.6, 19.8, 20.9, and 22.8 degrees 20. Thus, in some embodiments, the post-DVS crystalline form of compound A-4 has at least one characteristic peak (*e.g.* , one, two, three, four, five, six, seven, eight, nine, ten, eleven, twelve, or thirteen characteristic peaks) selected from approximately 7.4, 7.9, 9.1, 11.1, 12.0, 13.7, 14.4, 15.8, 16.4, 17.6, 19.8, 20.9, and 22.8 degrees 2θ. In some embodiments, post-DVS crystalline form of compound A-4 has at least three characteristic peaks selected from 7.4, 7.9, 9.1, 11.1, 12.0, 13.7, 14.4, 15.8, 16.4, 17.6, 19.8, 20.9, and 22.8 degrees 2θ.

Where the compounds disclosed herein have at least one chiral center, they may exist as individual enantiomers and diastereomers or as mixtures of such isomers, including racemates. Separation of the individual isomers or selective synthesis of the individual isomers is accomplished by application of various methods which are well known to practitioners in the art. Unless otherwise indicated, all such isomers and mixtures thereof are included in the scope of the compounds disclosed herein. Furthermore, compounds disclosed herein may exist in one or more crystalline or amorphous forms. Unless otherwise indicated, all such forms are included in the scope of the compounds disclosed herein including any polymorphic forms. In addition, some of the compounds disclosed herein may form solvates with water (i.e., hydrates) or common organic solvents. Unless otherwise indicated, such solvates are included in the scope of the compounds disclosed herein.

The skilled artisan will recognize that some structures described herein may be resonance forms or tautomers of compounds that may be fairly represented by other chemical structures, even when kinetically; the artisan recognizes that such structures may only represent a very small portion of a sample of such compound(s). Such compounds are considered within the scope of the structures depicted, though such resonance forms or tautomers are not represented herein.

### Definitions

Unless defined otherwise, all technical and scientific terms used herein have the same meaning as is commonly understood by one of ordinary skill in the art to which this disclosure belongs. In the event that there is a plurality of definitions for a term herein, those in this section prevail unless stated otherwise.

The term "pharmaceutically acceptable salt" refers to salts that retain the biological effectiveness and properties of a compound and, which are not biologically or otherwise undesirable for use in a pharmaceutical. In many cases, the compounds disclosed herein are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable salts can also be formed using inorganic and organic bases. Inorganic bases from which salts can be derived include, for example, bases that contain sodium, potassium, lithium, ammonium, calcium, magnesium, iron, zinc, copper, manganese, aluminum, and the like; particularly preferred are the ammonium, potassium, sodium, calcium and magnesium salts. In some embodiments, treatment of the compounds disclosed herein with an inorganic base results in loss of a labile hydrogen from the compound to afford the salt form including an inorganic cation such as Li⁺, Na⁺, K⁺, Mg²⁺ and Ca²⁺ and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987.

As used herein, "alkyl" refers to a straight or branched hydrocarbon chain that is fully saturated (i.e., contains no double or triple bonds). The alkyl group may have 1 to 20 carbon atoms (whenever it appears herein, a numerical range such as "1 to 20" refers to each integer in the given range; *e.g.,* "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 9 carbon atoms. The alkyl group could also be a lower alkyl having 1 to 4 carbon atoms. The alkyl group may be designated as "C₁₋₄ alkyl" or similar designations. By way of example only, "C₁₋₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like.

### Protecting Groups

In some circumstances, a chemical reaction may need to be performed selectively at one reactive site in a multifunctional compound. One such method that is useful for accomplishing such selectivity is to temporarily block one or more reactive sites in the multifunctional compound with a protective group. Such a method is often referred to as "protecting" the functional group. Many protecting groups are known in the art. See, e.g., Greene et al., Protective Groups in Organic Synthesis, Third Ed. (John Wiley & Sons, Inc. 1999); Wutz et al., Greene's Protective Groups in Organic Synthesis, Fourth Ed. (John Wiley & Sons, Inc. 2007). When more than one reactive site in a multifunctional compound requires protecting, or when a compound is prepared that will possess more than one protected functional group, it is important to use orthogonal protecting groups. Protecting groups are orthogonal if they are susceptible to selective removal.

In some embodiments, it may be necessary to protect one or more functional groups so as to prevent their interference in the desired reaction. For example, it may be necessary to protect one or more functional groups such as amines, carboxylic acids, and/or hydroxyl groups.

Suitable protecting groups for carboxylic acids include: esters such as enzymatically cleavable esters including heptyl, 2-N-(morpholino)ethyl, choline, (methoxyethoxy)ethyl, methoxyethyl; alkyl esters such as methyl, ethyl, propyl, isopropyl, butyl, tert-butyl, sec-butyl, pentyl, neopentyl, hexyl, heptyl, nonnyl, decanyl, and configurational isomers thereof; substituted methyl esters such as 9-fluroenylmethyl, methoxymethyl, methylthiomethyl, tetrahydropyranyl, teatrahydrofuranyl, methoxyethoxymethyl, 2-(trimethylsilyl)ethoxymethyl, benzyloxymethyl, pivaloyloxymethyl, phenylacetoxymethyl, triisopropylsilylmethyl, cyanomethyl, acetol, phencacyl, p-bromophenacyl, α-methylphenacyl, p-methoxyphenacyl, desyl, carboamidomethyl, p-azobenzenecarboxamidomethyl, N-phthalidimdomethyl; 2-substituted ethyl esters such as 2,2,2-trichloroethyl, 2-haloethyl, ω-chloroalkyl, 2-(trimethylsilyl)ethyl, 2-methylthioethyl, 1,3-dithianyl-2-methyl, 2-(p-nitrophenylsulfenyl)ethyl, 2-(p-toluenesulfonyl)ethyl, 2-(2'-pyridyl)ethyl, 2-(p-methoxyphenyl)ethyl, 2-(diphenylphosphino)ethyl, 1-methyl-1-phenylethyl, 2-(4-acetyl-2-nitrophenyl)ethyl, 2-cyanoethyl, 3-methyl-3-pentyl, dicyclopropylmethyl, 2,4-dimethyl-3-pentyl, cyclopentyl, cyclohexyl, allyl, methallyl, 2-methylbut-e-en-2-yl, 3-methylbut-2-(prenyl), 3-buten-1-yl, 4-(trimethylsilyl)-2-buten-1-yl, cinnamyl, α -methylcinnamyl, prop-2-ynyl, phenyl; 2,6-dialkylphenyl esters such as 2,6-dimethylphenyl, 2,6-diisopropylphenyl, 2,6-di-t-butyl-4-methylphenyl, 2,6-di-t-butyl-4-methoxyphenyl, p-(methylthio)phenyl, pentafluorophenyl, benzyl; substituted benzyl esters such as triphenylmethyl, diphenylmethyl, bis(o-mitrophenyl)methyl, 9-anthrylmethyl, 2-(9,10-dioxo)anthrylmethyl, 5-dibenzosuberyl, 1-pyreneylmethyl, 2-(trifluoromethyl)-6-chromonylmethyl, 2,4,6-trimethylbenzyl, p-bromobenzyl, o-nitrobenzyl, p-nitrobenzyl, p-methoxybenzyl, 2,6-dimethoxybenzyl, 4-(methylsulfinyl)benzyl, 4-sulfobenzyl, 4-azidomethoxybenzyl, 4-{N-[1-(4,4-dimethyl-2,6-dioxocyclohexylidene)-3-methylbutyl]amino} benzyl, piperonyl, 4-picolyl, polymer supported p-benzyl; silyl esters such as trimethylsilyl, triethylsilyl, t-butyldimethylsilyl, i-propyldimethylsilyl, phenyldimethylsilyl, di-t-butylmethylsilyl, triisopropylsilyl; activated esters such as thiol esters; oxazoles; 2-alkyl-1,3-axazoline; 4-alkyl-5-oxo-1,3-oxazolidine; 2,2-bistrifluoromethyl-4-alkyl-5-oxo-1,3-oxazolidine; 5-alkyl-4-oxo-1,3-dioxolane; dioxanones; ortho esters; pentaaminocobalt(III) complexes; and stannyl esters such as triethylstannyl and tri-n-butylstannyl; amides such as N,N-dimethyl, pyrrolidinyl, piperidinyl, 5,6-dihydrophenanthridinyl, o-nitroanilide, N-7-nitroindolyl, N-8-nitro-1,2,3,4-tetrahydroquinolyl, 2-(2-aminophenyl)acetaldehyde dimethyl acetal amide, and polymer supported p-benzenesulfonamide; hydrazides such as N-phenyl, N,N'diisopropyl; and tetraalkylammonium salts such as methyl, ethyl, propyl, isopropyl, butyl, tert-butyl, sec-butyl, pentyl, neopentyl, hexyl, heptyl, nonnyl, decanyl, and configurational isomers thereof.

### Administration and Pharmaceutical Compositions

The compounds disclosed herein (for example, crystalline Form A of Compound I') are administered at a therapeutically effective dosage. While human dosage levels have yet to be optimized for the compounds described herein, generally, a daily dose may be from about 0.25 mg/kg to about 120 mg/kg or more of body weight, from about 0.5 mg/kg or less to about 70 mg/kg, from about 1.0 mg/kg to about 50 mg/kg of body weight, or from about 1.5 mg/kg to about 10 mg/kg of body weight. Thus, for administration to a 70 kg person, the dosage range would be from about 17 mg per day to about 8000 mg per day, from about 35 mg per day or less to about 7000 mg per day or more, from about 70 mg per day to about 6000 mg per day, from about 100 mg per day to about 5000 mg per day, or from about 200 mg to about 3000 mg per day. The amount of active compound administered will, of course, be dependent on the subject and disease state being treated, the severity of the affliction, the manner and schedule of administration and the judgment of the prescribing physician.

Administration of the compounds disclosed herein can be via any of the accepted modes of administration for agents that serve similar utilities including, but not limited to, orally, subcutaneously, intravenously, intranasally, topically, transdermally, intraperitoneally, intramuscularly, intrapulmonarilly, vaginally, rectally, or intraocularly. Oral and parenteral administrations are customary in treating the indications that are the subject of the preferred embodiments.

The compounds useful as described above can be formulated into pharmaceutical compositions for use in treatment of these conditions. Standard pharmaceutical formulation techniques are used, such as those disclosed in Remington's The Science and Practice of Pharmacy, 21st Ed., Lippincott Williams & Wilkins (2005). Accordingly, some embodiments include pharmaceutical compositions comprising: (a) a safe and therapeutically effective amount of a compound described herein (including enantiomers, diastereoisomers, tautomers, polymorphs, and solvates thereof), or pharmaceutically acceptable salts thereof; and (b) a pharmaceutically acceptable carrier, diluent, excipient or combination thereof.

In addition to the selected compound useful as described above, come embodiments include compositions containing a pharmaceutically-acceptable carrier. The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The choice of a pharmaceutically-acceptable carrier to be used in conjunction with the subject compound is basically determined by the way the compound is to be administered.

The compositions described herein are preferably provided in unit dosage form. As used herein, a "unit dosage form" is a composition containing an amount of a compound that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, though a single administration is not specifically excluded. The skilled artisan will recognize that the formulation does not specifically contemplate the entire course of therapy and such decisions are left for those skilled in the art of treatment rather than formulation.

The compositions useful as described above may be in any of a variety of suitable forms for a variety of routes for administration, for example, for oral, nasal, rectal, topical (including transdermal), ocular, intracerebral, intracranial, intrathecal, intra-arterial, intravenous, intramuscular, or other parental routes of administration. The skilled artisan will appreciate that oral and nasal compositions comprise compositions that are administered by inhalation, and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the inhibitory activity of the compound. The amount of carrier employed in conjunction with the compound is sufficient to provide a practical quantity of material for administration per unit dose of the compound. Techniques and compositions for making dosage forms useful in the methods described herein are described in the following references: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004).

Various oral dosage forms can be used, including such solid forms as tablets, capsules, granules and bulk powders. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carrier suitable for the preparation of unit dosage forms for peroral administration is well-known in the art. Tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Such compositions may also be coated by conventional methods, typically with pH or time-dependent coatings, such that the subject compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit coatings, waxes and shellac.

Compositions described herein may optionally include other drug actives.

Other compositions useful for attaining systemic delivery of the subject compounds include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

A liquid composition, which is formulated for topical ophthalmic use, is formulated such that it can be administered topically to the eye. The comfort should be maximized as much as possible, although sometimes formulation considerations (e.g. drug stability) may necessitate less than optimal comfort. In the case that comfort cannot be maximized, the liquid should be formulated such that the liquid is tolerable to the patient for topical ophthalmic use. Additionally, an ophthalmically acceptable liquid should either be packaged for single use, or contain a preservative to prevent contamination over multiple uses.

For ophthalmic application, solutions or medicaments are often prepared using a physiological saline solution as a major vehicle. Ophthalmic solutions should preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric, acetate and phenylmercuric nitrate. A useful surfactant is, for example, Tween 80. Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

In a similar vein, an ophthalmically acceptable antioxidant includes, but is not limited to, sodium metabisulfite, sodium thiosulfate, acetylcysteine, butylated hydroxyanisole and butylated hydroxytoluene.

Other excipient components, which may be included in the ophthalmic preparations, are chelating agents. A useful chelating agent is edetate disodium, although other chelating agents may also be used in place or in conjunction with it.

For topical use, creams, ointments, gels, solutions or suspensions, etc., containing the compound disclosed herein are employed. Topical formulations may generally be comprised of a pharmaceutical carrier, co-solvent, emulsifier, penetration enhancer, preservative system, and emollient.

For intravenous administration, the compounds and compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. In various embodiments, the pH of the final composition ranges from 2 to 8, or preferably from 4 to 7. Antioxidant excipients may include sodium bisulfite, acetone sodium bisulfite, sodium formaldehyde, sulfoxylate, thiourea, and EDTA. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The compositions for intravenous administration may be provided to caregivers in the form of one more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately.

The actual dose of the active compounds described herein depends on the specific compound, and on the condition to be treated; the selection of the appropriate dose is well within the knowledge of the skilled artisan.

### Methods of Treatment

The references to methods of treatment in the subsequent paragraphs of this description are to be interpreted as references to the compounds, pharmaceutical compositions and medicaments of the present invention for use in a method for treatment of the human body by therapy. Some embodiments of the present disclosure include methods of treating bacterial infections with the compounds and compositions comprising the compounds described herein. Some methods include administering a compound, composition, pharmaceutical composition described herein to a subject in need thereof. In some embodiments, a subject can be an animal, e.g., a mammal (including a human). In some embodiments, the bacterial infection comprises a bacteria described herein. As will be appreciated from the foregoing, methods of treating a bacterial infection include methods for preventing bacterial infection in a subject at risk thereof.

In some embodiments, the subject is a human.

Further embodiments include administering a combination of compounds to a subject in need thereof. A combination can include a compound, composition, pharmaceutical composition described herein with an additional medicament.

Some embodiments include co-administering a compound, composition, and/or pharmaceutical composition described herein, with an additional medicament. By "coadministration," it is meant that the two or more agents may be found in the patient's bloodstream at the same time, regardless of when or how they are actually administered. In one embodiment, the agents are administered simultaneously. In one such embodiment, administration in combination is accomplished by combining the agents in a single dosage form. In another embodiment, the agents are administered sequentially. In one embodiment the agents are administered through the same route, such as orally. In another embodiment, the agents are administered through different routes, such as one being administered orally and another being administered intravenous (i.v.).

Examples of additional medicaments include an antibacterial agent, antifungal agent, an antiviral agent, an anti-inflammatory agent and an anti-allergic agent.

Preferred embodiments include combinations of a compound, composition or pharmaceutical composition described herein with an antibacterial agent such as a β-lactam. Examples of such β-lactams include Amoxicillin, Ampicillin (e.g., Pivampicillin, Hetacillin, Bacampicillin, Metampicillin, Talampicillin), Epicillin, Carbenicillin (Carindacillin), Ticarcillin, Temocillin, Azlocillin, Piperacillin, Mezlocillin, Mecillinam (Pivmecillinam), Sulbenicillin, Benzylpenicillin (G), Clometocillin, Benzathine benzylpenicillin, Procaine benzylpenicillin, Azidocillin, Penamecillin, Phenoxymethylpenicillin (V), Propicillin, Benzathine phenoxymethylpenicillin, Pheneticillin, Cloxacillin (e.g., Dicloxacillin, Flucloxacillin), Oxacillin, Methicillin, Nafcillin, Faropenem, Biapenem, Doripenem, Ertapenem, Imipenem, Meropenem, Panipenem, Cefazolin, Cefacetrile, Cefadroxil, Cefalexin, Cefaloglycin, Cefalonium, Cefaloridine, Cefalotin, Cefapirin, Cefatrizine, Cefazedone, Cefazaflur, Cefradine, Cefroxadine, Ceftezole, Cefaclor, Cefamandole, Cefminox, Cefonicid, Ceforanide, Cefotiam, Cefprozil, Cefbuperazone, Cefuroxime, Cefuzonam, Cefoxitin, Cefotetan, Cefmetazole, Loracarbef, Cefixime, Ceftazidime, Ceftriaxone, Cefcapene, Cefdaloxime, Cefdinir, Cefditoren, Cefetamet, Cefmenoxime, Cefodizime, Cefoperazone, Cefotaxime, Cefpimizole, Cefpiramide, Cefpodoxime, Cefsulodin, Cefteram, Ceftibuten, Ceftiolene, Ceftizoxime, Flomoxef, Latamoxef, Cefepime, Cefozopran, Cefpirome, Cefquinome, Ceftobiprole, Ceftaroline, Ceftiofur, Cefquinome, Cefovecin, Aztreonam, Tigemonam and Carumonam.

Preferred embodiments include β-lactams such as Ceftazidime, Biapenem, Doripenem, Ertapenem, Imipenem, Meropenem, Tebipenem, Tebipenem pivoxil, Apapenem, and Panipenem.

Additional preferred embodiments include β-lactams such as Aztreonam, Tigemonam, and Carumonam.

Some embodiments include a combination of the compounds, compositions and/or pharmaceutical compositions described herein with an additional agent, wherein the additional agent comprises a monobactam. Examples of monobactams include aztreonam, tigemonam, nocardicin A, carumonam, and tabtoxin. In some such embodiments, the compound, composition and/or pharmaceutical composition comprises a class A, C, or D beta-lactamase inhibitor. Some embodiments include co-administering the compound, composition or pharmaceutical composition described herein with one or more additional agents.

Some embodiments include a combination of the compounds, compositions and/or pharmaceutical compositions described herein with an additional agent, wherein the additional agent comprises a class B beta lactamase inhibitor. An example of a class B beta lactamase inhibitor includes ME1071 **(**Yoshikazu Ishii et al, "In Vitro Potentiation of Carbapenems with ME1071, a Novel Metallo-β-Lactamase Inhibitor, against Metallo-β-lactamase Producing Pseudomonas aeruginosa Clinical Isolates." Antimicrob. Agents Chemother. doi:10.1128/AAC.01397-09 (July 2010)). Some embodiments include co-administering the compound, composition or pharmaceutical composition described herein with one or more additional agents.

Some embodiments include a combination of the compounds, compositions and/or pharmaceutical compositions described herein with an additional agent, wherein the additional agent comprises one or more agents that include a class A, B, C, or D beta lactamase inhibitor. Some embodiments include co-administering the compound, composition or pharmaceutical composition described herein with the one or more additional agents.

### Indications

The compounds and compositions comprising the compounds described herein can be used to treat bacterial infections. Bacterial infections that can be treated with the compounds, compositions and methods described herein can comprise a wide spectrum of bacteria. Example organisms include gram-positive bacteria, gram-negative bacteria, aerobic and anaerobic bacteria, such as *Staphylococcus, Lactobacillus, Streptococcus, Sarcina, Escherichia, Enterobacter, Klebsiella, Pseudomonas, Acinetobacter, Mycobacterium, Proteus, Campylobacter, Citrobacter, Nisseria, Baccillus, Bacteroides, Peptococcus, Clostridium, Salmonella, Shigella, Serratia, Haemophilus, Brucella* and other organisms.

More examples of bacterial infections include *Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis,* or *Staphylococcus saccharolyticus.*

To further illustrate this invention, the following examples are included. The examples should not, of course, be construed as specifically limiting the invention. Variations of these examples within the scope of the claims are within the purview of one skilled in the art and are considered to fall within the scope of the invention as described, and claimed herein. The reader will recognize that the skilled artisan, armed with the present disclosure, and skill in the art is able to prepare and use the invention without exhaustive examples. The following examples will further describe the present invention as claimed, and are used for the purposes of illustration only, and should not be considered as limiting.

### EXAMPLES

Certain examples described herein are provided for illustrative or comparative purposes only and do not fall within the scope of the claimed invention. These examples are included to facilitate understanding of the invention and to highlight its advantages.

### X-ray Powder Diffraction (XRPD)

The Rigaku Smart-Lab X-ray diffraction system was configured for reflection Bragg-Brentano geometry using a line source X-ray beam. The x-ray source was a Cu Long Fine Focus tube that was operated at 40 kV and 44 ma. That source provides an incident beam profile at the sample that changes from a narrow line at high angles to a broad rectangle at low angles. Beam conditioning slits were used on the line X-ray source to ensure that the maximum beam size was less than 10 mm both along the line and normal to the line. The Bragg-Brentano geometry is a para-focusing geometry controlled by passive divergence and receiving slits with the sample itself acting as the focusing component for the optics. The inherent resolution of Bragg-Brentano geometry is governed in part by the diffractometer radius and the width of the receiving slit used. Typically, the Rigaku Smart-Lab is operated to give peak widths of 0.1 °*2θ* or less. The axial divergence of the X-ray beam was controlled by 5.0-degree Soller slits in both the incident and diffracted beam paths. The instrument was qualified using ASTM silicon standard on the same day of the analysis.

Powder samples were prepared in a low background Si holder using light manual pressure to keep the sample surfaces flat and level with the reference surface of the sample holder. Each sample was analyzed from 2 to 40 °2*θ* using a continuous scan of 6 °2*θ* per minute with an effective step size of 0.02 °2*θ*.

### Differential Scanning Calorimetry (DSC)

DSC analyses were carried out using a TA Instruments Q2500 Discovery Series instrument. The instrument temperature calibration was performed using indium. The DSC cell was kept under a nitrogen purge of ~50 mL per minute during each analysis. The sample was placed in a standard, crimped, aluminum pan and was heated from approximately 25 °C to 350 °C at a rate of 10 °C per minute.

### Melt Point (MP) Analysis

Melt point analysis was carried out using a Stuart SMP3 melt point apparatus. The sample was placed in a glass capillary and heated at 10 °C per minute.

### Thermogravimetric (TG) Analysis

TG analysis was carried out using a TA Instruments Q5500 Discovery Series instrument. The instrument balance was calibrated using class M weights and the temperature calibration was performed using alumel. The nitrogen purge was ~40 mL per minute at the balance and ~60 mL per minute at the furnace. Each sample was placed into a pre-tared platinum pan and heated from approximately 25 °C to 350 °C at a rate of 10 °C per minute.

### Dynamic Vapor Sorption (DVS) Analysis

DVS analysis was carried out using a TA Instruments Q5000 Dynamic Vapor Sorption analyzer. The instrument was calibrated with standard weights and a sodium bromide standard for humidity. Approximately 20 mg of sample was loaded into a metal-coated quartz pan for analysis. The sample was analyzed at 25 °C with a maximum equilibration time of one hour in 10% relative humidity (RH) steps from 5 to 95% RH (adsorption cycle) and from 95 to 5% RH (desorption cycle). The movement from one step to the next occurred either after satisfying the equilibrium criterion of 0.01% weight change or, if the equilibrium criterion was not met, after one hour. The percent weight change values were calculated using Microsoft Excel^{®}. The temperature for the DVS analysis can impact the outcome of the results.

### Karl Fischer (KF) Analyses

Karl Fischer analyses were carried out using a Mettler-Toledo C20 Coulometric KF titrator with oven attachment heated at 175 °C. The instrument was calibrated using a Hydranal water standard containing 1% water. The titrant was a Hydranal methanol solution. The sample was analyzed in triplicate.

### Optical Microscopy

Optical microscopy experiments were carried out on a Leica DM 2500 P compound microscope with a 10× magnification eye piece and a 10× magnification objective, for a total magnification of 100×. Images were captured using a QImaging MicroPublisher 3.3 RTV camera.

### Infrared (IR) Spectroscopy

The IR spectra were obtained using a Thermo Nicolet model 6700 Fourier-transform (FT) IR spectrophotometer equipped with a deuterated triglycine sulfate (DTGS) detector, a potassium bromide (KBr) beamsplitter, and an electronically temperature controlled (ETC) Ever-Glo^{®} IR source. The instrument was configured with a SMART iTR diamond attenuated total reflectance (ATR) sampling accessory. The single beam scan of the background (air) and sample were collected with 128 signal-averaged scans at a resolution of 2 cm⁻¹ over the spectral range 4000-400 cm⁻¹. The final sample spectrum was automatically calculated and presented in Log 1/R units. The wavelength calibration was verified using a certified polystyrene standard. Data collection and processing was performed using Omnic 9.7.46 software.

### Raman Spectroscopy

Fourier transform (FT) Raman spectra were acquired on a Nicolet model 6700 spectrometer interfaced to a Nexus Raman accessory module. This instrument is configured with a Nd:YAG laser operating at 1024 nm, a CaF₂ beamsplitter, and a indium gallium arsenide detector. OMNIC 8.1 software was used for control of data acquisition and processing of the spectra. Samples were packed into a 7.62 cm/ 3-inch glass NMR tube for analysis.

### Low-Frequency Raman Spectroscopy

Raman spectroscopy is a complementary technique to infrared (IR) spectroscopy and both techniques provide a full vibrational analysis of an entity being studied. Commercial Raman instruments typically utilize notch filters that block Rayleigh scattering and only allow for good quality Raman spectra to be obtained down to ~100 cm⁻¹. The spectral region from approximately 500 to 50 cm⁻¹ or lower, depending upon the type of filter, is referred to as the low frequency Raman spectral region. In this region, vibrational modes originate from the crystalline lattice of organic compounds, or from heavy atoms such as those incorporated into organometallic or inorganic molecules. The natural frequency of the crystal lattice is termed a phonon mode. Phonon modes arise from a fundamental structure, namely the specific crystal lattice for the particular compound being studied. Different crystalline forms typically display a unique crystal lattice, and therefore a unique phonon mode is displayed for each distinct crystalline form.

Low frequency (LF) Raman spectra became available owing to new filter designs, and it has been demonstrated that this region permits the identification / differentiation of different crystalline forms (*See* Roy, S., Chamberlin, B., and Matzger, A.J., "Polymorph Discrimination Using Low Wavenumber Raman Spectroscopy," Org. Process Res. Dev. 2013, 17, 976-980) The LF Raman spectroscopy allows the spectral acquisition in the Raman spectrum region, including Stokes region from 2200 cm⁻¹ to 0 cm⁻¹ and Anti-Stokes region from 0 cm⁻¹ to -900 cm⁻¹. The LF Raman spectroscopy allows observation of phonon modes (natural vibration frequency of the crystal lattice) which can be used to differentiate crystalline forms. The same "mirror image" signals corresponding to the phonon modes are visible in both Stokes and anti-Stokes regions, however, Stokes signals are normally used for differentiating crystalline forms owing to their stronger intensity than anti-Stokes signals.

LF Raman spectra were obtained using a Renishaw Raman, equipped with a ONDAX THz Raman system (excitation laser 853 nm, notch filter). The solid sample was analyzed with exposure time of 10 seconds and 32 accumulations. The solid sample was spread on a gold slide and analyzed using ONDAX TR-probe (Marqme TriX) contacting the solid sample. The LF-Raman was calibrated using sulfur reference standard prior to the sample analysis.

### ¹³C Nuclear Magnetic Resonance (NMR) Spectroscopy

The solid-state ¹³C cross polarization magic angle spinning (CPMAS) experiments were carried out on a Bruker Avance II 400 spectrometer. Each sample (approximately 200mg) was packed into a 4-mm zirconia rotor closed with Kel-F end caps for subsequent data acquisition. Adamantane, set to 29.5 ppm, was used as an external standard. Acquisition and processing parameters used are shown in the table below.

| | |
|---|---|
| Nucleus | ¹³C |
| Temperature (K) | 297 |
| Observe Frequency (MHz) | 100.64 |
| Sweep Width (Hz) | 29762 |
| Dwell Time (*µ*sec) | 16.8 |
| Acquisition Time (msec) | 275 |
| Recycle Delay (sec) | 20 |
| Spin Speed (kHz) | 7.0 |
| Number of Scans | 10240 |

| **Processing Parameters** | |
|---|---|
| Reference | external |
| Line Broadening (Hz) | 10 |

### EXAMPLE 1

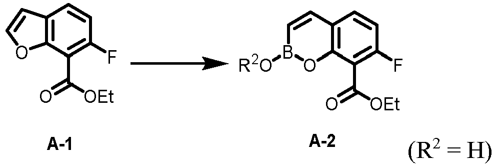

### Solvent screening for borylation of A-1

Catalyst preparation: Inside the N₂ filled glovebox, NiCl₂ anhydrous (0.45 mmol; 58.2 mg) and P(Oct)₃ (0.9 mmol; 333.6 mg) were added to a 5 mL vial equipped with a magnetic stirrer. Dry and degassed THF (3.0 mL) was added to the vial and the catalyst stock solution was stirred for 1.5 h at 60 °C. After that time, a dark red solution of the NiCl₂(P(Oct)₃)₂ complex was obtained (Note: no unreacted NiCl₂ was observed in the vial).

Reaction: Inside the N₂ filled glovebox, the corresponding base(s) (0.45 to 0.9 mmol) and the B₂Pin₂ (0.46 mmol; 117 mg) were added to a 5 mL vial equipped with a magnetic stirrer. Next, dry and degassed solvent (0.5, 0.95 or 1.0 mL) and an aliquot of the catalyst stock solution (100 µL, 5 mol%) was added to the vial followed by the substrate (0.3 mmol, 62.5 µL). Finally, DI water (50 or 500 µL) was added if needed. The vials were capped and placed inside the stem block where they heated overnight (ca. 17 h) at 80 °C or 90 °C (Toluene reactions) with a stirring speed of 750 rpm.

Reaction conditions: A-1 (0.3 mmol), base (1.5 to 3 equiv/A-1); B₂Pin₂ (1.5 equiv/A-1); Solvent (1.1 mL); 17 h.

Analysis: The capped vials were taken outside of the glovebox, allowed to cool down to room temperature and decapped. DI water (1 mL) and toluene or THF (500 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. Acetonitrile (950 µL) was added and the vial was capped and subjected to HPLC analysis. The results are summarized in Table 1. Qualitative results are presented based on % Area Product (P) and %Area Starting Material (SM). The HPLC yield is determined using the calibration curve (dilution: Reaction volume: 1.50 mL, Aliquot HPLC: 50 µL).

**Table 1: Effect of solvent on yield of A-2**

| | | | | | **Area %** | | | **Quant. HPLC yield Prod (%)** |
|---|---|---|---|---|---|---|---|---|
| # | **Base** | **Base equiv** | **Solvent** | **Additive** | **A(%P)** | **A(% SM)** | **A(%unk)** | |
| 1 | Cs₂CO₃ | 3 | Toluene | H₂O 5% | 51 | 44 | | 51 |
| 2 | | 3 | | H₂O 50% | 40 | 56 | | 44 |
| 3 | Cs₂CO₃ | 3 | THF | none | 55 | 44 | | 73 |
| 4 | | 3 | | H₂O 5% | 88 | 9 | | 98 |
| 5 | | 3 | | H₂O 50% | 39 | 61 | | 45 |
| 6 | Cs₂CO₃ | 3 | DMF | | 43 | 57 | | 51 |
| 7 | | 3 | DMA | | 49 | 51 | | 49 |
| 8 | | 3 | NEt3 | | 51 | 44 | | 71 |
| 9 | | 3 | MTBE | | 63 | 36 | | 85 |
| 10 | | 3 | Bu2O | | 55 | 44 | | 67 |
| 11 | ButOLi | 1.5 | THF | | 55 | 18 | 14 | 58 |
| 12 | ButOLi | 2 | THF | | 59 | 2 | 14 | 56 |
| 13 | ButOLi | 3 | THF | | 46 | 1 | 8 | 47 |
| 14 | ButOLi | 3 | Toluene | | 41 | 1 | 11 | 40 |
| 15 | LiOH | 2 | THF | | 38 | 48 | | 42 |
| 16 | LiOH | 2 | Toluene | | 38 | 57 | | 40 |
| 17 | MeOK | 2 | THF | | 73 | 3 | | 66 |
| 18 | MeOK | 2 | Toluene | | 67 | 6 | | 68 |
| 19 | NEt3 | 3 | Toluene | | 36 | 63 | | 40 |
| 20 | KOH | 2 | THF | | 49 | 37 | | 59 |
| 21 | CsF/ButOLi | 1 + 1 | THF | | 60 | 30 | 2 | 78 |
| 22 | CsF/ButOLi | 0.8 + 1 | THF | | 63 | 31 | 2 | 81 |
| 23 | CsF/ButOLi | 0.6 + 1 | THF | | 66 | 28 | 1 | 65 |
| 24 | CsF/ButOLi | 1 + 1 | Toluene | | 59 | 29 | 6 | 58 |
| 25 | CsF/ButOLi | 0.8 + 1 | Toluene | | 54 | 31 | 9 | 54 |
| 26 | CsF/ButOLi | 0.6 + 1 | Toluene | | 52 | 34 | 8 | 53 |
| 27 | KF/ButOLi | 0.8 + 1 | THF | | 60 | 28 | | 78 |
| 28 | KF/ButOLi | 0.8 + 1 | Toluene | | 44 | 30 | 17 | 48 |
| 29 | CsF/LiOH | 0.8 + 1 | THF | | 57 | 43 | | 77 |
| 30 | KF/LiOH | 0.8 + 1 | THF | | 35 | 54 | | 43 |

When Cs₂CO₃ is used as a base in toluene, the presence of water does not have a significant impact on the yield of desired product. At 50% H₂O, the reaction behaved as a biphasic system. In contrast, the presence of a small amount of H₂O (5% vol) exerted a very beneficial effect when THF was used as solvent However, lower amounts of product were detected at 50% H₂O.

### EXAMPLE 2

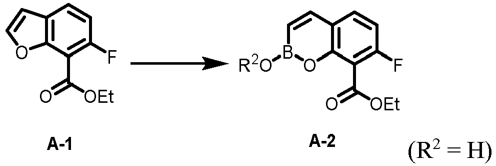

### Role of water in borylation of A-1

To further understand the positive effect of water on the borylation of A-1 to form **A-2,** a new set of experiments was performed using Cs₂CO₃ as base and various amounts of water (1; 2.5; 5; and 10% H₂O) in THF. Four other bases (K₂CO₃; KOH; MeOK and NEt₃) were also investigated in presence of 5% H₂O in THF. The borylation of A-1 was also studied using CsOH·H₂O as base (1 equiv/A-1) in THF. All the reactions were run using NiCl₂/2P(Oct)₃ as catalyst in 5 mol% loading at 80 °C in an overnight reaction. A total of 9 reactions were performed under these conditions.

Catalyst preparation: Inside the N₂ filled glovebox, NiCl₂ anhydrous (0.45 mmol; 58.2 mg) and P(Oct)₃ (0.9 mmol; 333.6 mg) were added to a 5 mL vial equipped with a magnetic stirrer. Dry and degassed THF (3.0 mL) was added to the vial and the catalyst stock solution was stirred for 1.5 h at 60 °C. After that time, a dark red solution of the NiCl₂ 2P(Oct)₃ complex was obtained (note: no unreacted NiCl₂ was observed in the vial).

Reaction: Inside the N₂ filled glovebox, the corresponding base (0.3 to 0.9 mmol) and the B₂Pin₂ (0.46 mmol; 117 mg) were added to a 5 mL vial equipped with a magnetic stirrer. Next, dry and degassed solvent (0.9, 0.95, 0.975, 0.99 or 1.0 mL) and an aliquot of the catalyst stock solution (100 µL, 5 mol%) was added to the vial followed by the substrate (0.3 mmol, 62.5 µL). Finally, DI water (10, 25, 50 or 100 µL) was added if needed. The vials were capped and placed inside the stem block were they heated overnight (ca. 17 h) at 80 °C with a stirring speed of 750 rpm.

Reaction conditions: **A-1** (0.3 mmol), base (1.1 to 3 equiv/ **A-1**); B₂Pin₂ (1.5 equiv/ **A-1**); Solvent (1.1 mL); 17 h.

Analysis: The capped vials were taken outside of the glovebox, allowed to cool down to room temperature and decapped. DI water (1 mL) and diethyl ether (Et₂O) (500 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. Acetonitrile (MeCN) (950 µL) was added and the vial was capped and subjected to HPLC analysis.

**Table 2: Effect of water on yield of A-2**

| | | | **Area %** | | | | **Quantitative HPLC yield Product (%)** |
|---|---|---|---|---|---|---|---|
| # | **Base** | **Additive** | **Base equiv** | **A (% Product)** | **A (% Starting Material)** | **A (% impurities)** | |
| 1 | Cs₂CO₃ | H₂O 5% | 3 | 92 | 22 | - | 105 |
| 2 | Cs₂CO₃ | H₂O 10% | 3 | 63 | 27 | - | - |
| 3 | Cs₂CO₃ | H₂O 1% | 3 | 73 | 14 | 2 | - |
| 4 | Cs₂CO₃ | H₂O 2.5% | 3 | 78 | 10 | - | - |
| 5 | Cs₂CO₃ | H₂O 5% | 3 | 35 | 49 | 1 | - |
| 6 | KOH | H₂O 5% | 2 | 48 | 38 | 1 | - |
| 7 | MeOK | H₂O 5% | 2 | 56 | 16 | 1 | - |
| 8 | NEt₃ | H₂O 5% | 3 | 34 | 53 | 5 | - |
| 9 | CsOH·H₂O | -- | 1.1 | 91 | 0 | - | 84 |

The positive effect of the addition of small amounts of H₂O when Cs₂CO₃ was used as the base was confirmed, with an optimal amount of water of around 5% by volume. The yield of product determined by quantitative HPLC is also very high (higher than 100% due to inaccuracy in volume). It was also observed visually that the addition of H₂O improved dramatically the solubility of the Cs₂CO₃ in THF. The borylation of A-1 reached completion when CsOH·H₂O (1.02 equiv/ **A-**1) was used as base. This result suggests that CsOH may be formed from Cs₂CO₃ and H₂O.

### EXAMPLE 3

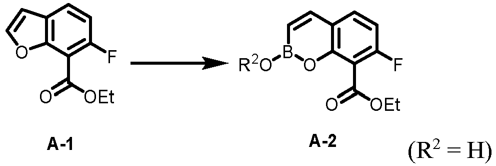

### Catalyst preparation and solvent effect on borylation of A-1

The NiCl₂/2P(Oct)₃ catalyst was prepared in THF, toluene, 1,4-dioxane and MTBE (methyl tert-butyl ether) and the reactions were run in those four solvents too. The catalyst solution in THF was also used in the reactions run in toluene for comparison purposes. The reactions were run in the presence of Cs₂CO₃ or CsOH·H₂O as bases and H₂O (5 and 10% volume) was also added. The reactions in THF were also tested using a lower amount of Cs₂CO₃ (2 equiv/A-1) to identify whether a large excess of base is needed when small amounts of water are present in the reaction. All 24 reactions were run using 5 mol% catalyst loading at 80 °C (in THF) or at 90 °C (in Tol) in overnight.

Catalyst preparation: Inside the N₂ filled glovebox, NiCl₂ anhydrous (0.45 mmol; 58.2 mg) and P(Oct)₃ (0.9 mmol; 333.6 mg) were added to a 5 mL vial equipped with a magnetic stirrer. Dry and degassed THF (3.0 mL) was added to the vial and the catalyst stock solution was stirred for 1.5 h at 60 °C. After that time, a dark red solution of the NiCl₂(P(Oct)₃)₂ complex was obtained (note: no unreacted NiCl₂ was observed in the vial).

Reaction: Inside the N₂ filled glovebox, the corresponding base (0.3 to 0.9 mmol) and the B₂Pin₂ (0.46 mmol; 117 mg) were added to a 5 mL vial equipped with a magnetic stirrer. Next, dry and degassed solvent (0.9, 0.95, 0.975, 0.99 or 1.0 mL) and an aliquot of the catalyst stock solution (100 µL, 5 mol%) was added to the vial followed by the substrate (0.3 mmol, 62.5 µL). Finally, DI water (10, 25, 50 or 100 µL) was added if needed. The vials were capped and placed inside the stem block were they heated overnight (ca. 17 h) at 80 °C or 90 °C (if toluene was used as solvent) with a stirring speed of 750 rpm.

Reaction conditions: **A-1** (0.3 mmol), base (1.1 to 3 equiv/**A-1**); B₂Pin₂ (1.5 equiv/A-1); Solvent (1.1 mL); 17 h.

Analysis: The capped vials were taken outside of the glovebox, allowed to cool down to room temperature and decapped. DI water (1 mL) and Et₂O (350 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. MeCN (950 µL) was added and the vial was capped and subjected to HPLC analysis.

The reactions run in THF and MTBE were analyzed by a slightly different protocol: The capped vials were taken outside of the glovebox, allowed to cool down to room temperature and decapped. DI water (1 mL) and Et₂O (500 µL) was added to each vial. A 6N HCl solution (100-200 µL) was added dropwise to each vial to neutralize the excess of base and reach a pH of 1-2. The vials were stirred for 1 h. The two phases were separated and Et₂O (1.0 mL) was added. The organic phase was extracted and placed in a flask. The solvent was removed under vacuo and then the reaction crude was dissolved in MeCN (1.35 mL). An aliquot of the MeCN solution (50 µL) was added to a GC vial followed by MeCN (950 µL). The GC vial was capped and subjected to HPLC analysis.

The results of the run are summarized in Table 3 below. Qualitative results are presented based on % Area Product (P) and %Area Starting Material (SM).

**Table 3: Effect of catalyst preparation and solvent on borylation of A-1**

| | | | | | | **Area %** | | | | **Quant. HPLC yield Prod (%)** |
|---|---|---|---|---|---|---|---|---|---|---|
| # | **Base** | **Solvent** | **Additive** | **Base equiv** | **Cat solvent** | **A (%P)** | **A (%imp)** | **A (%H2imp)** | **A (%H2imp)** | |
| 1 | Cs₂CO₃ | THF | 5% H₂O | 3 | THF | 80 | 14 | <1 | | 105 |
| 2 | | | 10% H₂O | 3 | THF | 57 | 35 | 1 | | 74 |
| 3 | | | none | 3 | THF | 56 | 44 | <1 | | 80 |
| 4 | Cs₂CO₃ | THF | 5% H₂O | 2 | THF | 62 | 31 | <1 | | 80 |
| 5 | | | 10% H₂O | 2 | THF | 46 | 46 | | | 63 |
| 6 | | | none | 2 | THF | 57 | 42 | | | 83 |
| 7 | CsOH H₂O | THF | | 1 | THF | 70 | | | | 73 |
| 8 | CsOH H₂O | THF | | 1 | THF | 79 | | | | 77 |
| 9 | Cs₂CO₃ | Toluene | 5% H₂O | 3 | THF | 55 | 33 | | | 61 |
| 10 | | | 10% H₂O | 3 | THF | 60 | 29 | 1 | 1 | 61 |
| 11 | Cs₂CO₃ | Toluene | | 3 | THF | 55 | 45 | | | 62 |
| 12 | CsOH H₂O | Toluene | | 1 | THF | 84 | 3 | | | 65 |
| 13 | Cs₂CO₃ | Toluene | | 3 | Toluene | 52 | 48 | | | 59 |
| 14 | Cs₂CO₃ | Toluene | 5% H₂O | 3 | Toluene | 51 | 34 | | | 56 |
| 15 | | Toluene | 10% H₂O | 3 | Toluene | 58 | 34 | 1 | 1 | 62 |
| 16 | CsOH H₂O | Toluene | | 1 | Toluene | 85 | 3 | | | 60 |
| 17 | Cs₂CO₃ | Dioxane | | 3 | Dioxane | 57 | 42 | | | 73 |
| 18 | Cs₂CO₃ | Dioxane | 5% H₂O | 3 | Dioxane | 52 | 33 | | | 64 |
| 19 | | Dioxane | 10% H₂O | 3 | Dioxane | 35 | 52 | 1 | | 46 |
| 20 | CsOH H₂O | Dioxane | | 1 | Dioxane | 91 | | | | 70 |
| 21 | Cs₂CO₃ | MTBE | | 3 | MTBE | 64 | 36 | | | 87 |
| 22 | Cs₂CO₃ | MTBE | 5% H₂O | 3 | MTBE | 66 | 26 | | | 84 |
| 23 | | MTBE | 10% H₂O | 3 | MTBE | 54 | 30 | 1 | 1 | 72 |
| 24 | CsOH H₂O | MTBE | | 1 | MTBE | 95 | | | | 95 |

The positive effect of water was observed, with an optimum at 5% volume. The conversion drops significantly when the excess of Cs₂CO₃ was reduced from 3 to 2 equiv/**A-1** in the reactions run in THF/H₂O. When CsOH·H₂O was used as base in THF solution, the reactions showed full consumption of the starting material. Reactions with Cs₂CO₃ in toluene showed lower conversions than those run in THF. Similar conversions were obtained independently of the solvent used for the catalyst preparation. The reactions with Cs₂CO₃ in dioxane and MTBE also showed slightly poorer conversions than in THF and no positive effect of added water. The reactions run with CsOH·H₂O as base showed full conversion of the starting material in THF, dioxane and MTBE, while small amounts of the starting material (*ca.* 5% area) were observed in toluene. In MTBE, a good quantitative yield of **A-2** is also obtained.

### EXAMPLE 4

### Effect of phosphine, mixture of bases, water and diboron on borylation of A-1

The effect of 4 different phosphines (P(Oct)₃; P(n-Bu)₃; P(n-Prop)₃ and TTMPP) and in combination with PPh₃ (1/1 ratio) on the borylation of A-1 was studied. The NiCl₂ phosphine catalysts were tested in the presence of Cs₂CO₃ as base and in THF as solvent. The commercially available complex NiCl₂(P(n-Bu)₃)₂ was also studied under the same reaction conditions. The combination of NiCl₂/ 2 P(Oct)₃ and dcype (1 equiv/Ni) was also studied. Additionally, mixtures of Cs₂CO₃/K₂CO₃ 1/9 and CsOH/K₂CO₃ 1/9 were investigated in THF/ H₂O solution (0; 2.5; 5 and 10% v/v) at 80 °C using the NiCl₂/ 2 P(Oct)₃ catalyst at 5 mol% loading. Also studied were: (1) CsOH·H₂O (2 equiv/**A-1**) in THF and Cs₂CO₃ in THF/H₂O (95/5 %v/v) at 80 °C using the NiCl₂/ 2 P(Oct)₃ catalyst at 5 mol% loading; (2) reactions with CsOH·H₂O (3; 2 and 1 equiv/**A-1**) in THF and in THF/H₂O (95/5 %v/v) and a reaction with Cs₂CO₃ in THF/H₂O (95/5 %v/v) at 65 °C using the NiCl₂/ 2 P(Oct)₃ catalyst at 5 mol% loading; and (3) two reactions using bis(catecholato)diboron and bis(neopentyl glycolato)diboron as boron sources in the presence of CsOH·H₂O (3 equiv/**A-1**) in THF at 65 °C using the NiCl₂/ 2 P(Oct)₃ catalyst at 5 mol% loading.

Catalyst preparation: Inside the N₂ filled glovebox, NiCl₂ anhydrous (0.45 mmol; 58.2 mg) and the corresponding phosphine (0.9 mmol) were added to a 5 mL vial equipped with a magnetic stirrer. Dry and degassed THF (3.0 mL) was added to the vial and the catalyst stock solution was stirred for 1.5 h at 60 °C. After that time, the dark solutions were stirred at room temperature over the weekend. The catalyse solutions were as follows:

| **Solution** | **A** | **B** | **C** | **D** | **E** | **F** | **G** | **H** |
|---|---|---|---|---|---|---|---|---|
| **Ligand** | P(Oct)3 | P(nBu)3 | P(nProp)3 | TTMPP | P(Oct)3/PPh3 | P(nBu)3/PPh3 | P(nProp)3/PPh3 | TTMPP/PPh3 |
| **Ligand equiv** | 2 | 2 | 2 | 2 | (1/1) | (1/1) | (1/1) | (1/1) |

Reaction: Inside the N₂ filled glovebox, the corresponding base (0.3 to 0.9 mmol) and the B₂Pin₂ (0.46 mmol; 117 mg) were added to a 5 mL vial equipped with a magnetic stirrer. Next, dry and degassed solvent (0.9, 0.95, 0.975 or 1.0 mL) and an aliquot of the catalyst stock solution (100 µL, 5 mol%) was added to the vial followed by the substrate (0.3 mmol, 62.5 mg). Finally, DI water (25, 50 or 100 µL) was added if needed. The vials were capped and placed inside the stem block were they heated overnight (ca. 17 h) at 80 °C or 65 °C with a stirring speed of 750 rpm.

Reaction conditions: A-1 (0.3 mmol), base (1.0 to 3 equiv/A-1); B₂Pin₂ (1.5 equiv/A-1); Solvent (1.1 mL); 17 h.

The capped vials were taken outside of the glovebox, allowed to cool down to room temperature and decapped. DI water (1 mL) and Et₂O (350 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. MeCN (950 µL) was added and the vial was capped and subjected to HPLC analysis.

The results are summarized in Table 4 below. Qualitative results are presented based on % Area P (Product) and %Area SM (Starting Material).

**Table 4: Effect of phosphine, bases, water and diboron on borylation of A-1**

| | | | | | | **Area (%)** | | | | **Quant HPLC yield (%)** |
|---|---|---|---|---|---|---|---|---|---|---|
| # | **Cat sol** | **Base** | **Additive** | **T (°C)** | **Time/h** | **A (%P)** | **A (% SM)** | **A (%imp)** | **A (%H2imp)** | |
| 1 | A | Cs₂CO₃ | none | 80 | 17 | 56 | 38 | | 6 | 54 |
| 2 | B | | | | | 53 | 39 | | 8 | 44 |
| 3 | C | | | | | 49 | 45 | | 6 | 55 |
| 4 | D | | | | | 16 | 71 | 1 | 4 | 19 |
| 5 | E | | | | | 44 | 48 | | 5 | 47 |
| 6 | F | | | | | 41 | 50 | | 6 | 44 |
| 7 | G | | | | | 39 | 51 | | 6 | 40 |
| 8 | H | | | | | 19 | 65 | 1 | 5 | 22 |
| 9 | NiCl₂ 2P(Bu)3 | | | | | 50 | 44 | | 6 | 51 |
| 10 | A and dcype | | | | | 42 | 52 | | 5 | 45 |
| 11 | A | K₂CO₃/CS₂CO₃ 9/1 | | 80 | 17 | 48 | 44 | | 7 | 46 |
| 12 | | | 2.5% H₂O | | | 29 | 53 | | 6 | 29 |
| 13 | | | 5% H₂O | | | 36 | 38 | 2 | 7 | 31 |
| 14 | | | 10% H₂O | | | 36 | 46 | 1 | 6 | 35 |
| 15 | | K₂CO₃/CsOH 9/1 | | | | 47 | 43 | | 6 | 45 |
| 16 | | | 2.5% H₂O | | | 42 | 40 | 1 | 7 | 34 |
| 17 | | | 5% H₂O | | | 40 | 42 | 1 | 6 | 39 |
| 18 | | | 10% H₂O | | | 32 | 49 | 1 | 6 | 31 |
| 19 | A duplo | CsOH H₂O 2 equiv | | 80 | 1 | 76 | 6 | 2 | 9 | |
| 19 | A duplo | CsOH H₂O 2 equiv | | 80 | 17 | 78 | 4 | 1 | 6 | 71 |
| 20 | A duplo | Cs₂CO₃ | 5% H₂O | | 1 | 85 | 4 | 1 | 7 | |
| 20 | A duplo | Cs₂CO₃ | 5% H₂O | | 17 | 87 | | 1 | 7 | 76 |
| 21 | A | CsOH H₂O 3 equiv | | 65 | 1 | 71 | 1 | 1 | 14 | |
| 21 | A | CsOH H₂O 3 equiv | | 65 | 17 | 61 | | | 6 | 54 |
| 22 | | CsOH H₂O 2 equiv | | | 1 | 85 | | 2 | 8 | |
| 22 | | CsOH H₂O 2 equiv | | | 17 | 83 | | 1 | 6 | 76 |
| 23 | | CsOH H₂O 1 equiv | | | 1 | 72 | 19 | | 8 | |
| 23 | | CsOH H₂O 1 equiv | | | 17 | 74 | 19 | 1 | 6 | 76 |
| 24 | | CsOH H₂O 3 equiv | 2.5% H₂O | | 1 | 64 | 10 | 2 | 9 | |
| 24 | | CsOH H₂O 3 equiv | 2.5% H₂O | | 17 | 48 | 12 | | 6 | 50 |
| 25 | | CsOH H₂O 2 equiv | 2.5% H₂O | | 1 | 67 | 16 | 2 | 9 | |
| 25 | | CsOH H₂O 2 equiv | 2.5% H₂O | | 17 | 65 | 17 | 1 | 5 | 72 |
| 26 | | CsOH H₂O 1 equiv | 2.5% H₂O | | 1 | 57 | 31 | 1 | 10 | |
| 26 | | CsOH H₂O 1 equiv | 2.5% H₂O | | 17 | 41 | 37 | 4 | 6 | 44 |
| 27 | | Cs₂CO₃ 3 equiv | 5% H₂O | | 1 | 71 | 21 | | 7 | |
| 27 | | Cs₂CO₃ 3 equiv | 5% H₂O | | 17 | 84 | 2 | 1 | 8 | 58 |
| 28 | A | CsOH H₂O 3 equiv | | 65 | 1 | 37 | 50 | | 12 | |
| 28 | A | CsOH H₂O 3 equiv | | 65 | 17 | 17 | 58 | | 5 | 20 |
| 29 | A | CsOH H₂O 3 equiv | | | 1 | 32 | 47 | | 7 | |
| 29 | A | CsOH H₂O 3 equiv | | | 17 | 21 | 16 | | 4 | 26 |

The three aliphatic phosphines (P(Oct)₃, P(nBu)₃ and P(nProp)₃) showed very similar results during the borylation of **A-1** (entries #1-3). The reaction run with the commercial NiCl₂[P(n-Bu)₃]₂ complex also showed analogous performance to the *in house* prepared complex. In contrast, the reaction run with the aromatic TTMPP phosphine showed much poorer results. The combination of an aliphatic phosphine/PPh₃ (1/1) showed slightly lower product formation than in the presence of aliphatic phosphines alone. The addition of dcype (1 equiv/Ni) to the NiCl₂/ 2P(Oct)₃ catalyst showed a slightly lower conversion than in the presence of the NiCl₂/ 2P(Oct)₃ catalyst alone. The combination of K₂CO₃ with CsOH·H₂O or Cs₂CO₃ resulted in significantly lower yields of product than in those reactions run with CsOH·H₂O or Cs₂CO₃ alone. Reactions run with CsOH·H₂O (2 equiv/A-1) in THF or Cs₂CO₃ (3 equiv/**A-1**) in THF/H₂O (95/5 %v/v) furnished the same results than the reactions run at 80 °C. The reactions at 65 °C appear to be also very fast as very high conversions were already observed after only 1.

Optimal results were found using CsOH·H₂O (2 equiv/**A-1**). The addition of 3 equivalents was detrimental for the reaction as additional byproducts were observed in the chromatogram. The addition of 1 equivalent gave good conversions and a clean reaction, albeit some starting material remained unreacted. Addition of small amounts of water (2.5% v/v) to the reactions run with CsOH·H₂O resulted either in no further improvement of the reaction or caused a slightly negative effect. Reactions run with other boron sources showed some product formation, however, much better results were obtained with B₂Pin₂.

### EXAMPLE 5

### Effect of catalyst loading on borylation of A-1

The effect of catalyst loading in the borylation of **A-1.** The reactions were run using the NiCl₂/ 2 P(Oct)₃ catalyst at 2.5 and 1.25 mol% using the two best reaction conditions: CsOH·H₂O (2 equiv/**A-1**) in THF and Cs₂CO₃ (3 equiv/**A-1**) in THF/H₂O (95/5 %v/v). The reactions were run at 80 and at 65 °C for comparison. A total of 8 reactions were run under these conditions.

Catalyst preparation: Inside the N₂ filled glovebox, NiCl₂ anhydrous (0.45 mmol; 58.2 mg) and the corresponding phosphine (0.9 mmol) were added to a 5 mL vial equipped with a magnetic stirrer. Dry and degassed THF (3.0 mL) was added to the vial and the catalyst stock solution was stirred for 1.5 h at 60 °C. After that time, the dark solutions were stirred at room temperature over the weekend.

Reaction: Inside the N₂ filled glovebox, the corresponding base (0.6 to 0.9 mmol) and the B₂Pin₂ (0.46 mmol; 117 mg) were added to a 5 mL vial equipped with a magnetic stirrer. Next, dry and degassed solvent (0.95 or 1.0 mL) and an aliquot of the catalyst stock solution (100 µL, 5 mol%) was added to the vial followed by the substrate (0.3 mmol, 62.5 mg). Finally, DI water (50 µL) was added if needed. The vials were capped and placed inside the stem block where they heated overnight (ca. 17 h) at 80 °C or 65 °C with a stirring speed of 750 rpm.

Reaction conditions: **A-1** (0.3 mmol), base (2.0 to 3 equiv/**A-1**); B₂Pin₂ (1.5 equiv/A-1); Solvent (1.1 mL); 17 h.

Analysis: The capped vials were taken outside of the glovebox, allowed to cool down to room temperature and decapped. DI water (1 mL) and Et₂O (350 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. MeCN (950 µL) was added and the vial was capped and subjected to HPLC analysis. The results are summrarized in Table 5 below. Qualitative results are presented based on % Area P and %Area SM. Quantitative assay yield for the product **A-2** [Dilution: Reaction volume: 1.35 mL; Aliquot HPLC: 50 µL].

**Table 5: Effect of catalyst loading on borylation of A-1**

| | | | | | | | | | | **Quant HPLC yield (%)** |
|---|---|---|---|---|---|---|---|---|---|---|
| # | **Cat Sol** | **Base** | **Additive** | **T** | **Time/h** | **A (%P)** | **A (%SM)** | **A (%imp)** | **A (%H2imp)** | |
| 30 | A (2.5%) | CsOH H₂O 2 equiv | | 80 | 4 | 29 | 45 | 2 | 8 | |
| 30 | A (2.5%) | CsOH H₂O 2 equiv | | 80 | 17 | 24 | 48 | | 6 | 8 |
| 31 | A (1.25%) | CsOH H₂O 2 equiv | | 80 | 4 | 23 | 52 | 1 | 7 | |
| 31 | A (1.25%) | CsOH H₂O 2 equiv | | 80 | 17 | 22 | 53 | | 6 | 19 |
| 32 | A (2.5%) | Cs₂CO₃ 3 equiv | 5% H₂O | 80 | 4 | 27 | 43 | 4 | 6 | |
| 32 | A (2.5%) | Cs₂CO₃ 3 equiv | 5% H₂O | 80 | 17 | 27 | 42 | | 6 | 27 |
| 33 | A (1.25%) | Cs₂CO₃ 3 equiv | 5% H₂O | 80 | 4 | 22 | 59 | 1 | 7 | |
| 33 | A (1.25%) | Cs₂CO₃ 3 equiv | 5% H₂O | 80 | 17 | 17 | 61 | | 6 | 18 |
| 34 | A (2.5%) | CsOH H₂O 2 equiv | | 65 | 4 | 32 | 47 | 1 | 10 | |
| 34 | A (2.5%) | CsOH H₂O 2 equiv | | 65 | 17 | 20 | 53 | 1 | 5 | 21 |
| 35 | A (1.25%) | CsOH H₂O 2 equiv | | 65 | 4 | 28 | 46 | 1 | 8 | |
| 35 | A (1.25%) | CsOH H₂O 2 equiv | | 65 | 17 | 17 | 49 | | 5 | 18 |
| 36 | A (2.5%) | Cs₂CO₃ 3 equiv | 5% H₂O | 65 | 4 | 38 | 38 | 3 | 7 | |
| 36 | A (2.5%) | Cs₂CO₃ 3 equiv | 5% H₂O | 65 | 17 | 35 | 36 | 1 | 6 | 34 |
| 37 | A (1.25%) | Cs₂CO₃ 3 equiv | 5% H₂O | 65 | 4 | 28 | 56 | 1 | 8 | |
| 37 | A (1.25%) | Cs₂CO₃ 3 equiv | 5% H₂O | 65 | 17 | 19 | 58 | 1 | 6 | 18 |

At catalysts loadings lower than 5 mol% none of the reactions showed full consumption of the starting material.

### EXAMPLE 6

### Base and B₂Pin₂ optimization for borylation of A-1

Three different amounts of base were tested (2.0; 1.5 and 1.2 equiv/A-1) in combination with three different amounts of B₂Pin₂ (1.1; 1.2 and 1.5 equiv/A-1). The reactions were all run in THF at 65 °C using a catalyst loading of 5mol%. A blank reaction with no catalyst was also run to confirm the hydrolysis of the substrate.

Catalyst preparation: Inside the N₂ filled glovebox, NiCl₂ anhydrous (0.45 mmol; 58.2 mg) and P(n-Bu)₃ (0.9 mmol; 182.1 mg) were added to a 5 mL vial equipped with a magnetic stirrer. Dry and degassed THF (3.0 mL) was added to the vial and the catalyst stock solution was stirred for 1.5 h at 60 °C. After that time, a dark red solution of the NiCl₂(P(nBu)₃)₂ complex was obtained (Note: no unreacted NiCl₂ was observed in the vial).

Reaction: Inside the N₂ filled glovebox, the CsOH·H₂O (2.0 to 1.2 equiv/1) and the B₂Pin₂ (1.1 to 1.5 equiv/**1**) were added to a 5 mL vial equipped with a magnetic stirrer. Next, dry and degassed THF (1.0 mL) and an aliquot of the catalyst stock solution (100 µL, 5 mol%) was added to the vial followed by the substrate (0.3 mmol, 62.5 mg). The vials were capped and placed inside the stem block where they heated overnight (ca. 17 h) at 65 °C with a stirring speed of 750 rpm. Aliquots were taken after 1h reaction.

Reaction conditions: **A-1** (0.3 mmol), base (1.2 to 2.0 equiv/**A-1**); B₂Pin₂ (1.1 to 1.5 equiv/**A-1**); Solvent (1.1 mL); 17 h.

Analysis: The capped vials were taken outside of the glovebox, allowed to cool down to room temperature and decapped. DI water (1 mL) and Et₂O (1000 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. MeCN (950 µL) was added and the vial was capped and subjected to HPLC analysis. The results are shown below in Table 6. Qualitative results are presented based on % Area P and %Area SM Quantitative analysis was performed for samples 1 to 9.

**Table 6: Base and B₂Pin₂ optimization for borylation of A-1**

| # | **Base** | **B₂Pin₂** | **A(%P)** | **A(%SM)** | **A(%imp)** | **A(%H2imp)** | **Quant HPLC yield (%)** |
|---|---|---|---|---|---|---|---|
| 1 | CsOH 2 equ | 1.1 equiv | 71 | 9 | | 2 | 84 |
| 2 | | 1.2 equiv | 71 | 6 | 1 | 3 | 83 |
| 3 | | 1.5 equiv | 73 | 8 | 1 | 3 | 78 |
| 4 | CsOH 1.5 equ | 1.1 equiv | 74 | 18 | 1 | 3 | 101 |
| 5 | | 1.2 equiv | 90 | 4 | 1 | 3 | 108 |
| 6 | | 1.5 equiv | 75 | 15 | 1 | 3 | 95 |
| 7 | CsOH 1.2 equiv | 1.1 equiv | 73 | 20 | 1 | 2 | 105 |
| 8 | | 1.2 equiv | 76 | 16 | 1 | 3 | 92 |
| 9 | | 1.5 equiv | 81 | 15 | 1 | 3 | 95 |
| 10 | CsOH 2 equiv | 1.5 equiv | | | | | |

In the presence of 2 equivalents of base, the reaction product was detected in good yields but a significant amount of impurities that were not hydrolyzed **A-1** was also observed. Reducing the amount of base to 1.5 equiv/**A-1** gave a cleaner chromatogram. With 1.2 equiv. of B₂Pin₂, a very good conversion was observed. With less B₂Pin₂, the conversion was lower. With more B₂Pin₂, the reaction was also not complete. Reducing the amount of base to only 1.2 equivalents resulted in lower conversions.

### EXAMPLE 7

### Effect of ethanol as solvent on borylation of A-1

Catalyst preparation: Inside the N₂ filled glovebox, NiCl₂ anhydrous (0.45 mmol; 58.2 mg) and P(nBu)₃ (0.9 mmol; 182.1 mg) were added to a 5 mL vial equipped with a magnetic stirrer. Dry and degassed THF (3.0 mL) was added to the vial and the catalyst stock solution was stirred for 1.5 h at 60 °C. After that time, a dark red solution of the NiCl₂ 2P(nBu)₃ complex was obtained (note: no unreacted NiCl₂ was observed in the vial).

Reaction: Inside the N₂ filled glovebox, the corresponding base (0.45 to 0.9 mmol) and the B₂Pin₂ (0.46 mmol; 117 mg) were added to a 5 mL vial equipped with a magnetic stirrer. Next, dry and degassed solvent (0.9, 0.95, 0.975 or 1.0 mL) and an aliquot of the catalyst stock solution (100 µL, 5 mol%) was added to the vial followed by the substrate (0.3 mmol, 62.5 mg). Finally, DI water or absolute ethanol (EtOH) (50 µL) was added if needed. The vials were capped and placed inside the stem block where they heated overnight (ca. 17 h) at 65 °C with a stirring speed of 750 rpm.

Reaction conditions: **A-1** (0.3 mmol), base (1.0 to 3 equiv/**A-1**); B₂Pin₂ (1.5 equiv/**A-1**); Solvent (1.1 mL); 17 h.

Analysis: The capped vials were taken outside of the glovebox, allowed to cool down to room temperature and decapped. DI water (1 mL) and Et₂O (350 µL) was added to each vial. A 6N HCl solution (100-200 µL) was added dropwise to each vial to neutralize the excess of base and reach a pH of 1-2. The vials were stirred for 1 h. The two phases were separated and an aliquot of the organic phase (50 µL) was added to a GC vial. MeCN (950 µL) was added and the vial was capped and subjected to HPLC analysis.

When THF/EtOH (95/5 %v/v) is used as solvent, the borylation proceeded very well showing better conversions than the one obtained for THF/H₂O. The borylation reaction can be run in EtOH as solvent if CsOH is used as a base. A lower excess of base should be used in order to prevent the undesired hydrolysis of the starting material and product. When Cs₂CO₃ was used as base, the use of EtOH as solvent led mostly to the hydrolyzed starting material.

### EXAMPLE 8

### Scale-up reactions for borylation of A-1 using CsOH·H₂O as base

Reaction conditions are shown in Table 7 below. All the reactions were performed using CsOH·H₂O as base (1.5 equiv/**A-1**) and B₂Pin₂ (1.25 equiv/**A-1**) at 65 °C. THF was used as solvent (8 mL) except in Experiment #4, where EtOH was used as the solvent. All the reactions were using the commercial NiCl₂[P(nBu)₃]₂ as catalyst in 5 mol% loading, added as a solid or in THF solution (0.6 mL). Experiments 1-4 were performed in the glovebox with 333 mg of **A-1.** Experiments 5-6 were performed in an Easymax reactor under inert atm with 832mg of **A-1.** A-1 is denoted as starting material

**Table 7: Reaction conditions for scale-up of borylation of A-1**

| # | **mmol sm** | **Protocol** |
|---|---|---|
| 1 | 1.6 | base (solid), B₂Pin₂ (solid), solvent, sm (liq), catalyst (sol). Then start stirring and heat |
| 2 | 1.6 | base (solid), B₂Pin₂ (solid), solvent. Stir 1 h at 30 C. sm (liq), catalyst (sol). Then heat |
| 3 | 1.6 | base (solid; 25% total), B₂Pin₂ (solid), solvent, sm (liq), cat (sol). Start stirring and heating. |
| 4 | 1.6 | Then add the rest of the base (solid) in 4 portions in 1 h interval |
| | | base (solid; 20% total), B₂Pin₂ (solid), EtOH, sm (liq), cat (sol). Start stirring and heating. |
| | | Then add the rest of the base (liq) in 4 portions in 30 min interval |
| 5 | 4 | base (solid), cat (solid), Bpin and sm in solution. Then start stirring and heating |
| 6 | 4 | Base (solid), Bpin and sm in solution. Start stirring. Cat (in sol) then heat. |

The results of the run are summarized below. Qualitative results are presented based on % Area P; %Area SM, % Area PCOOH (hydrolyzed product), % Area smCOOH (hydrolyzed starting material).

| # | **t/h** | **A (%P)** | **A (%SM)** | **A (%imp)** | **A (%H2imp)** | **A (%smCOOH)** | **Quant HPLC yield (%)** |
|---|---|---|---|---|---|---|---|
| 1 | 1 | 87 | 10 | | 3 | | |
| | 2.5 | 89 | 8 | | 3 | | 94 |
| 2 | 1 | 53 | 34 | 3 | 3 | | |
| | 17 | 55 | 34 | 3 | 3 | | 56 |
| 3 | 0.25 | 35 | 63 | | 2 | | |
| | 0.5 | 50 | 48 | | 2 | | |
| | 0.75 | 60 | 38 | | 2 | | |
| | 1 | 69 | 28 | | 2 | | |
| | 17 | 82 | 14 | | 2 | | 86 |
| 4 | 1 | 88 | 5 | 2 | 3 | 1 | |
| | | 89 | 3 | 3 | 3 | 1 | 79 |
| 5 | 1 | 10 | 68 | 3 | 1 | 6 | |
| | 2.5 | 7 | 67 | 3 | 1 | 7 | |
| | 3 | 26 | 50 | 3 | 2 | 6 | |
| | 17 | 25 | 50 | 2 | 2 | 6 | 32 |
| 6 | 1 | 25 | 63 | 2 | 2 | 1 | |
| | 4 | 37 | 43 | 2 | 1 | 2 | 41 |

Observations: Exp #1. Excellent reproducibility was observed when following the same protocol as for thelmL scale reaction: addition of base and B₂Pin₂ as solids followed by THF, then the liquid substrate and finally the catalyst in THF solution. The stirring and heating was started once all the components were added and the vial was capped. Note that the amount of base is here reduced to 1.5 equivalents and the reaction proceeded very cleanly with no hydrolyzed products formation albeit not to full conversion (see chromatograms below). This sample was subjected to isolation and GC purity measurements.

Exp #2. Stirring CsOH·H₂O and B₂Pin₂ at room temperature (ca. 30-32 °C) for 1 h before adding the rest of the components (substrate and catalyst) was found detrimental for the reaction: Only 55% area product was observed. This may be due to the partial decomposition of the reactive sp²-sp³ B₂Pin₂₋CsOH adduct prior to the borylation reaction. Formation of an unknown impurity (7.496min, unknown) was also noted.

Exp #3. Dosing CsOH·H₂O as a solid over a 1 hour period also showed a positive effect, with the conversion increasing gradually with the corresponding addition of the base. Even though the reaction was clean, full conversion was not achieved. This could be due to a mismatch between the fast borylation reaction and the slow addition of base.

Exp #4. The reaction with CsOH·.H₂O was also performed in EtOH as a solvent with the base being dosed from an EtOH stock solution over a 30 min period (300mg in 2mL). Under these conditions, slightly better conversion (89 area %) was obtained compared to the previous experiment. The reaction was a homogenous solution instead of a slurry.

Exp #5. Premixing the solid base and the solid catalyst prior to the addition of solvent and substrates was found very detrimental for the reaction. It appeared that the contact of the catalyst with the base caused catalyst decomposition: Almost no reaction took place after 2.5 h. At this time, the addition of a new batch of catalyst (5 mol%) resulted in the formation of moderate amounts of product but full conversion was not reached.

Exp #6. The addition of B₂Pin₂ and substrate in THF solution to the solid CsOH followed stirring prior to the addition of the catalyst solution also showed poor conversion (37% area after 4h). This may also be related to the decomposition of B₂Pin₂-base adduct (See Exp # 2).

### EXAMPLE 9

### Scale-up reactions for borylation of A-1 using Cs₂CO₃ as base

All the reactions were run using Cs₂CO₃ as base (3.0 equiv/A-1) and B₂Pin₂ (1.5 equiv/A-1) at 65 °C. THF was used as solvent (7.6 mL) with EtOH (0.4 mL) added as co-solvent. All the reactions were run using the commercial NiCl₂[P(nBu)₃]₂ as catalyst in 5 mol% loading added in THF solution (0.6 mL).

| **#** | **Protocol** |
|---|---|
| 1 | base (solid), B2Pin2 (solid), solvent, sm (liq), catalyst (sol). Then start stirring and heat |
| 2 | Base (solid), B2Pin2 (solid), THF. **Stir 2.5 h at 30 C.** sm (liq), catalyst (sol), **EtOH. Then heat.** |
| 3 | Base (solid), B2Pin2 (solid), THF and EtOH . **Stir 2.5 h at 30 C.** sm (liq), catalyst (sol).**Then heat.** |

Reaction conditions: substrate (1.6 mmol; 333 mg), B₂Pin₂ (2.4 mmol; 609 mg), Cs₂CO₃ (4.8 mmol; 1.563 g), THF (7.6 mL), EtOH (0.4 mL), NiCl₂[P(nBu)₃]₂ (0.08 mmol; 42.7 mg in 0.5 mL THF).

The results of the reactions are summarized below. Qualitative results are presented based on % Area P and %Area SM.

| # | **A (%P)** | **A (%SM)** | **A (%imp)** | **A (%H2imp)** | **Quant HPLC yield (%)** |
|---|---|---|---|---|---|
| 1 | 97 | | | 1 | 98 |
| 2 | 98 | | | 2 | 93 |
| 3 | 30 | 64 | 1 | 2 | 22 |

The results obtained with Cs₂CO₃ at small scale were reproduced showing full conversion and only a minor impurity. Pre-stirring the base and B₂Pin₂ in THF for 2.5 h at 30 °C before adding the rest of the components did not affect the results, which is contrary to what is observed with CsOH·H₂O as the base. This could be the best procedure at scale. Pre-stirring the base and B₂Pin₂ in THF/EtOH (95/5 %v/v) for 2.5 h at 30 °C before adding the rest of the components showed a negative effect on the reaction with only a small fraction of the starting material being converted to the product.

### EXAMPLE 10

### Preparation of (ethyl 7-fluoro-2-hydroxy-2H-benzo[e][1,2]oxaborinine-8-carboxylate (A-2) with CsOH·H₂O in THF

All steps described were performed insude an N₂ filled glovebox. Commercial NiCl₂[P(*n*-Bu)₃]₂ (42.7 mg, 0.08 mmol, 0.05 eq.) Aldrich # 332062; CAS # 15274-43-8) was weighed into a 5 mL vial. Anhydrous THF (250 µL) was added to the vial. The catalyst dissolved immediately upon stirring.

In a 25 mL reaction vial equipped with a magnetic stirrer, solid CsOH·H₂O (403 mg, 2.4 mmol, 1.5 eq.; Aldrich, 99.95% trace metal basis) was added. Solid B₂Pin₂ (508 mg, 2 mmol, 1.25 eq.; Aldrich 99%) was next added to the vial, followed by anhydrous THF (8 mL). Benzofuran **A-1** (333 mg, 1.6 mmol, 1 eq.) was added to the vial with a micropipette. The Ni catalyst in THF solution was then added to the reaction vial with a micropipette. The 5 mL vial of the catalyst solution was washed with THF (250 µL), and the wash added to the reaction vial with a micropipette. The reaction vial was capped and placed in a heating block and heated at 65 °C while stirring at 750 rpm. 92% area of benzofuran **A-1** was converted after 1h of reaction (89% area product).

### EXAMPLE 11

### Preparation of (ethyl 7-fluoro-2-hydroxy-2H-benzo[e][1,2]oxaborinine-8-carboxylate (A-2) with CsOH·H₂O in Ethanol Solution

All steps described were performed insude an N₂ filled glovebox. Commercial NiCl₂[P(nBu)₃]₂ (42.7 mg, 0.08 mmol, 0.05 eq.) Aldrich # 332062; CAS # 15274-43-8) was weighed into a 5 mL vial. Anhydrous THF (250 µL) was added to the vial. The catalyst dissolved immediately upon stirring.

A base solution was prepared by adding solid CsOH·H₂O (324 mg, 80% total amount of base) was added to a 5 mL vial. EtOH absolute (2 mL) was then added to the vial. After 1-2 min stirring at 30-32 °C all the CsOH was dissolved.

In a 25 mL vial equipped with a magnetic stirrer, solid CsOH·H₂O (80 mg, 20% total amount of base) was added. Solid B₂Pin₂ was next added to the vial and EtOH (6 mL) was added by syringe. Benzofuran **A-1** (333 mg, 1.6 mmol, 1 eq.) was added to the vial followed by the Ni catalyst in THF solution. The vial of the catalyst solution was washed with THF (250 µL) and the wash was added to the reaction vial. The reaction vial was capped and placed in a heating block and heated at 65 °C while stirring at 750 rpm. The CsOH·H₂O solution in EtOH was added in 4 portions (500 µL each) to the reaction mixture by means of a syringe. The addition was performed over a total period of 30 minutes. 97% area of benzofuran **A-1** was converted after 2h of reaction (89% area product).

### EXAMPLE 12

### Preparation of (ethyl 7-fluoro-2-hydroxy-2H-benzo[e][1,2]oxaborinine-8-carboxylate (A-2) with Cs₂CO₃ in THF/EtOH (95/5 %v/v)

All steps described were performed insude an N₂ filled glovebox. Commercial NiCl₂[P(nBu)₃]₂ (42.7 mg, 0.08 mmol, 0.05 eq.) Aldrich # 332062; CAS # 15274-43-8) was weighed into a 5 mL vial. Anhydrous THF (250 µL) was added to the vial. The catalyst dissolved immediately upon stirring.

In a 25 mL vial equipped with a magnetic stirrer, solid Cs₂CO₃ (1564 mg, 4.8 mmol, 3.0 eq.; Alrich 99% ReagentPlus) was added. Solid B₂Pin₂ (610 mg, 2.4 mmol, 1.5 eq.; Aldrich 99%) was next added to the vial, followed by anhydrous THF (7.6 mL) and absolute ethanol (0.4 mL). Benzofuran **A-1** (333 mg, 1.6 mmol, 1 eq.) was added to the reaction vial followed by the Ni catalyst in THF solution. The vial of the catalyst solution was washed with THF (250 µL) and the wash was added to the reaction vial. Absoulte ethanol (0.4 mL) was added to the reaction vial. The reaction vial was capped and placed in a heating block and heated at 65 °C while stirring at 750 rpm. Full conversion was observed after 1h of reaction.

### EXAMPLE 13

### Preparation of (ethyl 7-fluoro-2-hydroxy-2H-benzo[e][1,2]oxaborinine-8-carboxylate (A-2) with Cs₂CO₃ in THF/EtOH (95/5 %v/v) with premixing of B₂Pin₂ and a base

All steps described were performed insude an N₂ filled glovebox. Commercial NiCl₂[P(nBu)₃]₂ (42.7 mg, 0.08 mmol, 0.05 eq.) Aldrich # 332062; CAS # 15274-43-8) was weighed into a 5 mL vial. Anhydrous THF (250 µL) was added to the vial. The catalyst dissolved immediately upon stirring.

In a 25 mL vial equipped with a magnetic stirrer, solid Cs₂CO₃ (1564 mg, 4.8 mmol, 3.0 eq.; Alrich 99% ReagentPlus) was added. Solid B₂Pin₂ (610 mg, 2.4 mmol, 1.5 eq.; Aldrich 99%) was next added to the vial, followed by anhydrous THF (7.6 mL). The vial was capped and the mixture was stirred for 2.5 h at 30-32 °C. The vial was decapped and benzofuran **A-1** (333 mg, 1.6 mmol, 1 eq.) was added to the reaction vial followed by the Ni catalyst in THF solution. The vial of the catalyst solution was washed with THF (250 µL) and the wash was added to the reaction vial. Absoulte ethanol (0.4 mL) was added to the reaction vial. The reaction vial was capped and placed in a heating block and heated at 65 °C while stirring at 750 rpm. Full conversion was observed after 1h of reaction.

Once the reaction complete, the reaction vial was cooled down to room temperature. DI water (3 mL) was added to the mixture and the vial was stirred at room temperature, followed by addition of Et₂O (2 mL) was added to the mixture. HCl (6 N) was added with a pipette until a pH of 1 was reached (1.6 mL approx.) The biphasic system was stirred at 0 °C for 1 h and the organic layer was extracted. The solvent was removed *in vacuo* and the crude mixture was redissolved in a small volume of EtOH absolute (ca. 3-4 mL). The solution was stirred overnight in the presence of 37% HCl (3 drops). The solvent was evaporated in vacuo and the residue purified by column chromatography (eluent: DCM/EtOH: 100/0 to 93/7 %v/v).

### EXAMPLE 14

### Preparation of Compound A-3

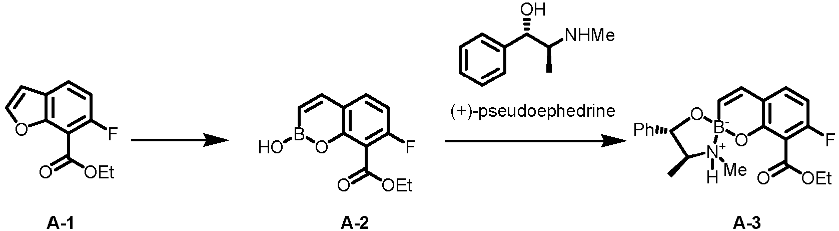

Preparation of (+)-pseudophedrine for reaction: 10 L of 2-methyl tetrahydrofuran (MeTHF) was loaded into 100 L reactor under nitrogen. Pseudoephedrine hydrochloride salt (3070 mg) was loaded into the reactor and suspended in MeTHF with stirring. Another 10 L of 2-MeTHF was loaded to wash down all the solid of pseudoephedrine hydrochloride salt that attached on the glass wall of the reactor. The jacket temperature was set at 20 °C. Under nitrogen, 6.2 L of 4N NaOH (3.4 kg of 30% NaOH) was added into the suspension of pseudoephedrine hydrochloride salt in 2-MeTHF. The addition rate was maintained to keep the internal temperature below 50 °C. The reaction mixture was stirred under nitrogen for about 1 hour, and then stopped stirring to let the solution stand still for more than 30 minutes. The phases were separated at room temperature, and the organic layer was kept inside 100L reactor and washed with Brine (5 L) twice, the pH of the last water layer was pH 9-10. The organic layer was transferred out of the reactor and kept in inside a carboy.

Cesium carbonate (14.1 kg) was loaded into 100 L reactor under nitrogen. Solid diboron pinacolate (5.13 kg) was added to the reactor under nitrogen. 2-MeTHF (25.6 kg, 30 L) was added to wash down all solids attached to inner wall. The mixture was stirred for about 30 minutes to dissolve diboron pinacolate at room temperature. Benzofuran ester **A-1** (3.0 kg) was added into the reactor. The reaction mixture was degassed three times by vacuum/filling with nitrogen. The reaction mixture was heated up to 50 °C under nitrogen with stirring. The P(*n*-Bu)₃ / NiCl₂ catalyst solution in EtOH/2-MeTHF (540 g 1.5 L/6 L) was added portion wise: pumped about 30 seconds in every 3 minutes period, total time for catalyst addition was about 90 minutes. The reaction mixture was sampled by removing 0.2 mL of the mixture and diluting with MTBE (5 mL) and DI water (5 mL), and then acidifying with 6N HCl to pH 0-1. The organic layer was diluted with 10 volumes of acetonitrile for HPLC. Once the conversion of **A-1** was higher than 95%, the reaction mixture was cooled to 0°C, then quenched with cold DI water (20 kg). The temperature of the mixture was maintained below 5°C during quench. The pH of the reaction mixture was adjusted to pH = 0-1 with 37% of HCl (approximately ~ 9.5 kg was used) under nitrogen at 0°C. MTBE (14.8 kg, 20 L) of was added to promote the precipitation of pinacol, and then the reaction mixture was stirred for overnight at 0 °C. Pinacol was removed by filtration and the solid was washed with cold n-heptane (2 x 5.5 kg) to remove color from pinacol solid. All liquids from filtration and washes were pumped back into 100 L reactor. The layers were allowed to separate at room temperature and the the organic layer was kepy inside the reacter. The organic layer was washed with deionized water (4 X 20 kg) until the water layer had a pH of 6. The (+)-pseudophedrine solution was added to the reaction mixture at room temperature and the reaction mixture was stirred under nitrogen overnight. The jacket temperature was increased to 70 °C and volume of reaction was reduced to about 35 L by vacuum distillation. Toluene (15 kg) was added into the product mixture inside 100 L reactor to a total volume of about 55 L. Vacuum distillation was continued until about 25 L of reaction mixture was left inside the reactor. Toluene (13 kg) was then added to the reaction mixture until the total volume was about 40 L). Vacuum distillation was continued until about 10 L of reaction mixture remained inside the 100 L reactor, and toluene (10 kg) was added back into the reactor to raise the total volume to about 25 L. The reaction mixture was sampled and the content of 2-MeTHF was determined to be less than 5 wt. %. 3.5k g of n-heptane was added, rasing the total volume was about 30 L. The product solution was cooled down to 0 °C and held for overnight prior to filtration to give crude compound **A-3. A-3** was then washed with cold n-heptane and dried under vacuum at 45°C for more than 2 days to give compound **A-3.**

### Single Crystal X-ray Structure Determination For Compound A-3

A yellow rod shaped crystal of compound A-3 with formula C₂₁H₂₃BFNO₄ having approximate dimensions of 0.42 × 0.22 × 0.13 mm was mounted on a Mitegen micromesh mount in a random orientation. Preliminary examination and data collection were performed using Cu Kα radiation (λ = 1.54178 Å) on Bruker AXS D8 Quest CMOS diffractometer equipped with a four axis kappa stage, an I-µ-S microsource X-ray tube laterally graded multilayer optics, a Photon2 CMOS area detector. The initial unit cell was determined and data were collected using Apex3 v2017.3-0 (Saint V8.38A, Bruker AXS Inc.: Madison (WI), USA, 2013/2014) at a temperature of 301 K (room temperature). Frames were integrated using SAINT V8.38A. A total of 27,647 reflections were collected, of which 4,242 were unique. Cell constants for data collection were obtained from least-squares refinement using 9,257 reflections between 5.7514 and 79.5737°. The monoclinic cell parameters and calculated volume are a = 11.191(4) Å, b = 7.751(3) Å, c = 11.795(4) Å, β = 95.867(12)°, V = 1017.8(6) Å³. For Z = 2 and a formula weight of 383.21 the calculated density is 1.250 g/cm³. The linear absorption coefficient is 0.753/mm for Cu Kα radiation. Scaling and a multi-scan absorption correction using SADABS (Apex3 v2017/3) was applied. Transmission coefficients ranged from 0.5825 to 0.7543. Intensities of equivalent reflections were not averaged during data processing.

The space group was determined by the program XPREP as embedded in SHELXTL (Version 6.14, Bruker AXS Inc., Madison, Wisconsin: USA). Systematic absences and intensity statistics indicated the space group *P*2₁ (# 4). The structure was solved by direct methods using SHELXS and refined by full matrix least squares against *F*² with all reflections using SHELXL-2018 (Sheldrick, G.M. (2008). Acta Cryst., A64, 112-122.; Sheldrick, G. M. (2018). SHELXL2018. University of Göttingen, Germany) and the graphical user interface ShelXle (Hübschle, C. B., Sheldrick, G. M. and Dittrich, B. (2011). J. Appl. Cryst., 44, 1281-1284). Additional atoms were located in succeeding difference Fourier syntheses. The structure was refined using full-matrix least-squares where the function minimized was Σw(|Fₒ|²-|F_{c}|²)² and the weight w is defined as w = 1/[σ²(*F*ₒ²) + (0.0638*P*)² + 0.0447P] where P = (*F*ₒ² + 2*F*_{c}²)/3. Scattering factors were taken from the International Tables for Crystallography (Vol C Tables 4.2.6.8 and 6.1.1.4). A total of 4,242 independent reflections were used in the refinements. 4,015 reflections with *F*² > *2*σ(*F*²) were used in the calculation of R1. H atoms attached to carbon and nitrogen atoms were positioned geometrically and constrained to ride on their parent atoms, with carbon hydrogen bond distances of 0.95 Å for aromatic C-H, and 1.00, 0.99 and 0.98 Å for aliphatic C-H, CH₂ and CH₃ moieties, and 0.98 Å for N-H bond distances, respectively. Methyl H atoms were allowed to rotate but not to tip to best fit the experimental electron density. Uᵢₛₒ(H) values were set to a multiple of U_{eq}(C/N) with 1.5 for methyl, alcohol and water H atoms, and 1.2 for N-H, C-H and CH₂ units, respectively.

The absolute structure parameter (Flack x) was determined using 1,748 quotients [(I+)-(I-)]/[(I+)+(I-)] using the method described by Parsons et al., Acta Cryst. 2013, B69, 249-259, and refined to -0.03(6). Completeness of Friedel pairs to 67.679 ° in theta is 97.2 %. For all data it is 93.6 %.

The final cycle of refinement included 256 variable parameters and 1 restraint (for the origon) and converged (the largest parameter shift was 0.001 times its standard uncertainty) with unweighted and weighted agreement factors of: R1 = Σ |Fₒ| - |F_{c}| / Σ |Fₒ| = 0.038;wR² = {Σ [w (Fₒ² - F_{c}²)²] / Σ [w(Fₒ²)²]}^{0.5} = 0.1119. The goodness-of-fit parameter was 1.110. The highest peak in the final difference Fourier had a height of 0.167 e/Å³. The minimum negative peak had a height of - 0.117 e/Å³.

### EXAMPLE 15

### Preparation of Compound A-4

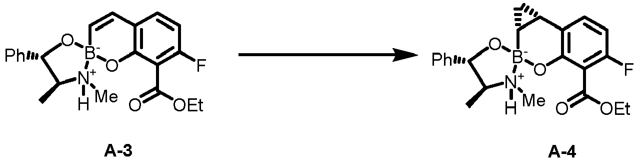

### Preparation of Diazald solution

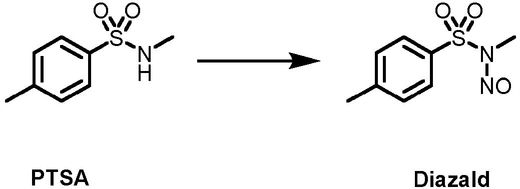

Diethyl ether (30 L) was charged into 100 L reactor under nitrogen with stirring. p-toulenesulfonic acid (PTSA) (15 kg) was charged into the reactor and suspended in ethyl ether. 40 L more of ethyl ether was added to wash down all PTSA solid that attached to glass wall. The reaction mixture was cooled down to 0 °C, and 37% HCl (16 kg) was charged into the reactor, while maintained the temperature below 0 °C. The addition line was then washed with 1 kg of DI water. A solution of NaNO₂ (5.85 kg NaNO₂ in 12 kg H₂O) was added slowly while maintaining the temperature at about 0 °C. The reaction mixture was warmed up to 20° C and stirring was continued for one hour to produce an orange-red solution. Stirring was stopped and the reaction mixture was kept still for more than 30 minutes. The organic phase was washed with sodium bicarbonate solution three times (3 x 1.2 kg sodium bicarbonate in 3.5 kg DI H₂O) to pH > 6, then kept at about 0-5°C with slow stirring with most of the diazald crystallizing out of solution.

The Diazald solution was warmed up slowly to 20 °C to dissolve all solid. Concentration and purity were checked with ¹H-NMR (>200ml of solution was concentrated to dryness for concentration).

Inside of a Generator, dimethoxyethane (6.9 kg) and 45% KOH in water (3.8 kg) was charged under nitrogen, then heated to above 53 °C, and maintained between 53-58°C during reaction.

Inside Reactor: compound **A-3** (1.2 kg) was charged into the reactor, followed by THF (21.7 kg). The reactor contents were stirred under nitrogen to dissolve all **A-3,** and then cooled to a temperature between -25 °C and -15 °C. Palladium acetate (12 g) was dissolved in THF (3.1 kg) with stirring at room temperature. An atmospheric distillation apparatus was inerted with a positive nitrogen sweep and checked for leaks (outlet was immersed in 60% acetic acid water solution swap. Continuous nitrogen bubbles were observed).

Once the temperature of the Generator reached 53 °C or higher. About 500 mL of diethyl ether was fed into the Generator. When the ether was received continuously by the reactor (ether drops continued fall into reactor from the tail of the condenser), the desired amount of Diazald solution in ether was continuous fed evenly into generator. The time for feeding Diazald was about 100-110 minutes total. The palladium catalyst solution was added with pump into reactor portion wise: the pump was turned on for about 30s for each 3 minutes period. The time for catalyst addition was about 100-110 minutes. Yellow color diazomethane solution flowed continuously into reactor during reaction and gas bubbles were always observed inside acetic acid swap.

The reaction was sampled at 90 minutes and analyzed by HPLC for completion. The reaction temperature was maintained below -15 °C for 30 mins after catalyst addition in the presence of a nitrogen sweep. The generator contents were killed with 60% HOAc (3.7kg) in water (the inner temperature was maintained below 60°C during acetic acid addition). The reaction mixture was quenched with 60% HOAc in THF (3.22 kg). The quenched reaction mixture was transferred into carboys and kept at room temperature. Three batches of reactions with **A-3** were combined for the workup. The reaction mixture was concentrated to almost dryness under reduced pressure at 50 °C. The residue was dissolved in 18 L (for three combined batches) of THF. *n*-Heptane (25 kg) was continuously fed into the rotovap flask during distillation under reduced pressure. The product mixture was concentrated to almost dryness using rotovap distillation, again redissolved in 18 L THF and concentrated to near dryness after continuously feeding *n*-Heptane (25 kg) into the rotovap flask during distillation under reduced pressure. The residue was dissolved in 18L (6L for one batch of step 2 reaction) of THF, then diluted with isopropyl alcohol (35.4 kg). The solution was then filtered through a filtration bag (filled with about 300 g of Celite), 1-5µm polish filter, and 0.25µm polish filter. The product solution from filtration was concentrated to dryness using a rotovap under reduced pressure. Isopropyl alcohol (14.1kg) was used to dissolve all residue, and then 6 L of n-heptane was added. The acetic acid content of the solution was checked, and it was below 1 wt%. The product solution in IPA was cooled down to 0-10 °C with stirring. The crystalline compound **A-4** was isolated using filtration and washed once with cold isopropyl alcohol (3 L). The volume of filtrate was reduced to half first using distillation under reduced pressure, and then cooled down to 0 °C, and additional **A-4** was isolated using filtration and washed once with cold isopropyl alcohol (3 L). Crystalline compound **A-4** was dried at 45 °C under vacuum for two days.

### EXAMPLE 16

### Alternative Preparation of Compound A-4

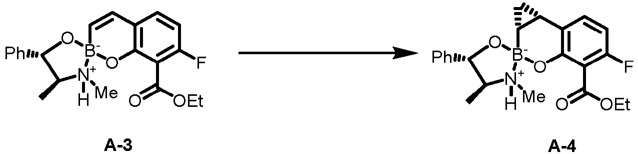

A heterogeneous mixture of Zn/Cu couple (1,71 g, 26.1 mmol, 2 eq,) and compound **A-3** (5 g, 13 mmol) in 2-methyltetrahydofuran (25 mL) was heated in an oil bath to 65 °C (bath 68 °C). Upon reaching 65 °C, diiodomethane (0.5 mL, 6.2 mmol, 0.5 eq) was added. Iodine was then added to initiate the reaction followed by portion-wise addition of the diiodomethane (1.4 mL, 17.4 mmol, 1.3 eq,) to control the exotherm. Upon completion of the addition of diiodomethane, the black heterogeneous mixture was stirred at 65 °C for 30 min then cooled to room temperature, conversion was >99% and chemical purity was 96.9% by HPLC . The cooled reaction mixture was quenched with a 1:1 mixture of saturated NH₄Cl and water (25 mL), and after stirring at room temperature for 5 minutes, the solids were filtered off over celite. The flask and pad were rinsed with MTBE (2 x 10 mL). The combined biphasic filtrates were partitioned and the organic layer was washed with a 1:1 mixture of saturated NH₄Cl and water (3 x 25 mL). The organic layer was concentrated to dryness. The enantiomeric excess was determined to be 98.7% by chiral HPLC. The yellow solid residue was taken up in isopropyl alcohol (25 mL) and the heterogeneous mixture was heated at 60 °C. The clear yellow solution was allowed to cool to room temperature. After stirring at room temperature overnight a pale yellow slurry was obtained. The slurry was cooled to 0 °C. After stirring at 0 °C for 3 h, the solids were collected by filtration and rinsed with cold isopropyl alcohol, air dried then dried under high vacuum to give compound **A-4** (1.78 g, 34% yield, 99.76% chemical purity and 100% e.e).

### EXAMPLE 17

### Alternative Preparation of Compound A-4

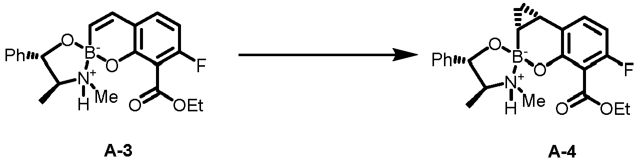

Acetyl chloride (50 µL) was added to an heterogeneous mixture of Zn (3.41 g, 52.2 mmol), CuCl (517 mg, 5.2 mmol) and CH₂Br₂ (0.75 mL, 10.7 mmol) in anhydrous 2-MeTHF (25 mL) at room temperature. The grey mixture was gradually heated in an oil bath to 50 °C monitoring for exotherms and appearance. Upon complete activation of the Zn/Cu couple, solid compound A-3 (5 g, 13 mmol) was added and the reaction mixture was heated at 60 °C. The remaining CH₂Br₂ (2 mL, 28.5 mmol) was added in portions monitoring for exotherms. Upon completion of the addition the conversion was >99%. The black heterogeneous mixture was cooled to room temperature and carefully quenched with a 1:1 mixture of saturated NH₄Cl and water (25 mL). The mixture was filtered over celite and the flask and pad were rinsed with MTBE. The combined biphasic filtrate was partitioned. The organic layer was washed with a 1:1 mixture of saturated NH₄Cl and water (25 mL) and concentrated to dryness to give compound **A-4** as a pale yellow foam with a 98.6% ee

### EXAMPLE 18

### Alternative Preparation of Compound A-4

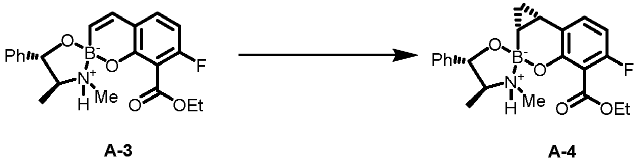

A round bottom flask was charged with a 1 M solution of ZnEt₂ in heptane (6.5 mL, 65.2 mmol) and cooled to -20 °C. A solution of CH₂I₂ (1.05 mL, 13 mmol) in CH₂Cl₂ (2 mL) was added keeping the temperature <-20 °C; a white milky mixture was obtained. The reaction mixture was allowed to warm to -10 °C over 20 min. A solution of compound **A-3** (1 g, 2.6 mmol) in CH₂Cl₂ (5 mL) was added keeping the temperature <-10 °C, a slightly heterogeneous mixture with solids stuck on the sides of the flask was obtained. Upon completion of the addition, conversion was determined to be 71% by HPLC. After 15 min at -10 °C, conversion was 77%. The bath was allowed to warm up slowly. Upon reaching 0 °C, conversion was 79%. Upon reaching 12 °C, conversion was 90%. After stirring 2 more hours (temperature reached 14 °C) conversion was 94%. The reaction mixture was cooled to 0 °C and quenched with sat NH₄Cl (10 mL, exotherm to 10 °C). After stirring vigorously for 1 min, the layers were separated. The organic layer was washed with water then concentrated to dryness. Upon concentration, the product started to crystallize once CH₂Cl₂ was removed. Compound **A-4** was obtained as pale yellow solid was obtained (ee = 97.8%).

### EXAMPLE 19

### Preparation of Compound A-5

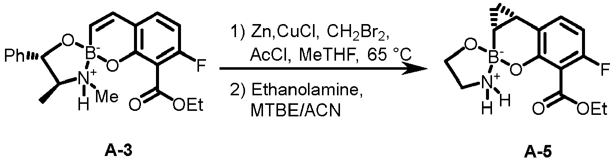

A procedure was developed for the cyclopropanation of the pseudoephedrine complex of ethyl 7-fluoro-2-hydroxy-2H-benzo[e][1,2]oxaborinine-8-carboxylate (**A-3**) that alleviated issues associated with reactivity of the zinc/copper couple by making the reagent *in situ.* In addition, these new conditions allowed for the replacement of CH₂I₂ by the much cheaper CH₂Br₂. However, due to the nature of pseudoephedrine, strongly acidic conditions could not be used during the workup to remove all the zinc salts without substantial loss of the pseudoephedrine moiety. Since pseudoephedrine had fulfilled its role in achieving the high enantioselectivity during the cyclopropanation, it was no longer needed and could be replaced by an alternative amino alcohol complex of the boronate. The procedure is detailed below.

Acetyl chloride (93 µL, 1.3 mmol) was added to a heterogeneous mixture of Zn (6.83 g, 104 mmol), CuCl (1.03 g, 10.4 mmol) and dibromomethane (0.5 mL, 13 mmol) in 2-methyltetrahydrofuran (MeTHF) (50 mL) at room temperature. The grey mixture was heated in an oil bath set to 50 °C. Upon the internal temperature reaching 50 °C, the starting material **A-3** (10 g, 26.1 mmol) was added at once and the flask was rinsed with MeTHF (5 mL). The grey heterogeneous mixture was heated to 60 °C (bath at 60 °C). Upon the internal temperature reaching 59 °C the remaining CH₂Br₂ (4.1 mL, 52.2 mmol) was added in small portions keeping the temperature <65 °C. Upon completion of the addition HPLC showed almost complete conversion. The reaction mixture was heated at 60 °C for an additional 30 min to get 99.4% conversion. The reaction mixture was cooled to 0 °C and poured into a 1:1 mixture of saturated NH₄Cl and water at 0 °C (75 mL). After stirring at room temperature for 10 min, the black mixture was filtered over celite and the flask and pad were rinsed with methyl tert-butyl ether (MTBE). The combined filtrates were partitioned and the organic layer was washed with 1 M HCl (2 x 50 mL) and then water (3 x 50 mL). The organic layer was filtered through celite and the filtrate was concentrated to dryness. The yellow oil was taken up in acetonitrile (ACN) (50 mL) and concentrated to dryness. The residual syrup was taken up in 2/8 ACN/MTBE mixture (70 mL). Ethanolamine was added slowly over 45 min and the salt crystallized rapidly. After stirring at room temperature overnight, the solids were collected by filtration, rinsed with 9/1 MTBE/ACN, air dried and then dried under high vacuum to give **A-5** as a yellow powder 6.63 g, 86.6% yield, 99.57% purity. The complex can then be further reacted in a manner similar to that shown in Example 20 in order to obtain Compound I'.

### EXAMPLE 20

### Preparation of Compound I'

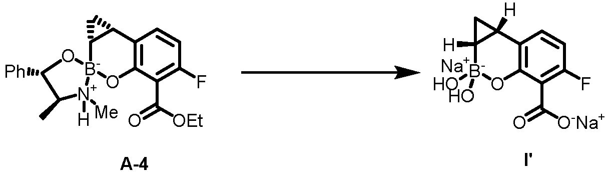

Compound **A-4** (7 kg) was loaded into 100 L reactor under nitrogen. 2-MeTHF (64 kg) was charged into the 100 L reaction vessel to wash down all solid that attached to glass wall. The temperature was increased to 40 °C. Compound **A-4** was dissolved in 2-MeTHF with stirring at about 35 °C. A solution of sodium hydroxide (1.8 kg solid dissolved in 13kg of DI H₂O) was loaded into the reactor and addition line was washed with 1 kg of DI water. The reaction mixture was stirred at 60 °C under nitrogen overnight. The reaction mixture was cooled down to 20°C with stirring. The stirring was then stopped, allowed to sit for 30 minutes, and the aqueous layer was isolated and extracted with MTBE (1 x 14.8 kg). The pH of the aqueous layer was adjusted to 9 with 3.52 kg of acidic resin (Amberlite IR120H resin. Before use, it was washed three times with MTBE, 3 X 10L, then washed four times with DI water, 4 X 1 0L). The pH 9 solution was filtered through a filtration bag (for removing resin), carbon cartridge (2300 cm², 8-in stack, for removing palladium and color), and 1-5 µm polish filter. The water was then removed using azeotropic distillation with methyl ethyl ketone (238 kg). The liquid was sampled for the analysis of water content, which was about 2.5% water by volume. The mixture was concentrated to a slurry at 50 °C under reduced pressure. The slurry was cooled to room temperature, filtered under nitrogen, and washed with methyl ethyl ketone (2 x 6 L) and dried under vacuum at 50°C for 48 hours to give compound **I'.**

### EXAMPLE 21

### Effect of Chiral Auxiliary on the Preparation of Compound (B-IV)

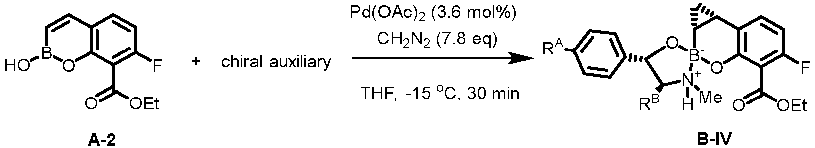

Preparation of chiral auxiliaries for this reaction may be achieved by procedures described in, e.g., Tetrahedron Asymm. 2016, 27, 338 and Tetrahedron Asymm 2010, 21, 837.

A stock solution of substrate **A-2** in dry THF (30 mg/mL) and a stock solution of Pd(OAc)₂ in dry THF (1 mg/mL) were prepared. For each cyclopropanation reaction, approximately 20 mg of the chiral auxiliary was dissolved in 4 mL of dry THF. The substrate stock solution (1 mL) was added and the mixture was stirred for at least 1h at ambient temperature. The reaction vial was cooled to -15°C. Subsequently, 1 mL of catalyst stock solution was added, followed by 3 mL of diazomethane solution (approximately 0.33 M). The reaction mixture was stirred at -15°C for 60 minutes and subsequently the temperature was allowed to increase to room temperature. The enantiomeric excess of compound **B-IV** (wherein R¹ is ethyl) was analyzed by chiral HPLC. The results are presented in Table 8 below.

**Table 8: Enantiomeric Excess Compound B-IV (Ethyl Ester) For Various Chiral Auxiliaries**

| **Chiral Auxiliary** | **Enantiomeric excess B-IV [%]** |
|---|---|
| 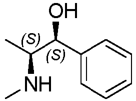 (1*S*,2*S*)-(+)*pseudo*-ephedrine | 73 |
| 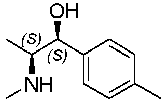 Aux-3 | 70 |
| | 71 |
| 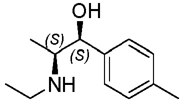 Aux-4 | 55 |
| | 59 |
| 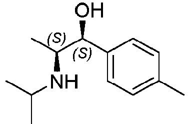 Aux-5 | 3 |
| | 3 |
| 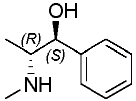 (1*S*,2*S*)-(+)ephedrine | -28 |
| 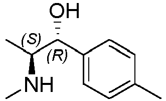 Aux-11 | 33 |
| 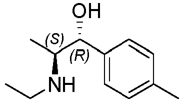 Aux-13 | 16 |
| 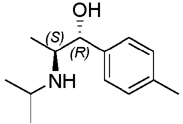 Aux-14 | 4 |

The data indicates that Aux-3 shows the best performance as auxiliary in the cyclopropanation reaction. The enantiomeric excess obtained with this auxiliary is near identical to that obtained with (1*S*,2*S*)-(+) *pseudo*-ephedrine. With increasing size of the *N*-substituent, the stereoselectivity of the cyclopropanation reaction decreases. With the *N*-isopropyl **Aux-5** near racemic product was obtained.

Application of ephedrine-analogue **Aux-11** resulted in a significantly lower asymmetric induction. The absolute value of enantiomeric excess achieved with the close analogue is comparable to that achieved with (1*S*,2*R*)-(+) ephedrine. For the three ephedrine analogues **Aux-11, Aux-13** and **Aux-14** a same trend emerges as for the *pseudo*-ephedrine analogues **Aux-3, Aux-4, and Aux-5**: With increasing size of the *N*-substituent, the stereoselectivity of the cyclopropanation reaction decreases. With **Aux-14,** near racemic product was obtained, as was the case with **Aux-5**.

### EXAMPLE 22

### Effect of palladium catalyst loading on cyclopropanation of A-3 with diazomethane

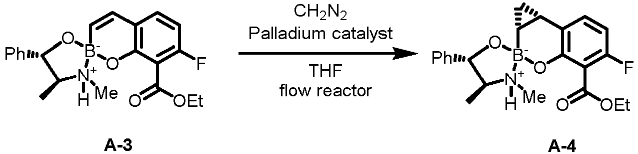

The effect of palladium catalyst for cyclopropanation of A-3 for the following catalysts: Pd(OAc)₂, Pd₂(DVTMS)₃, Pd(acac)₂, Pd(TFA)₂, and Pd(OPiv)₂. The effect was measured in a flow reactor using 2% mol palladium catalyst, three equivalents of CH₂N₂, a 10 minute residence time in the flow reactor, a -20 °C bath temperature and a 210,000 Pa (30 psi) system backpressure.

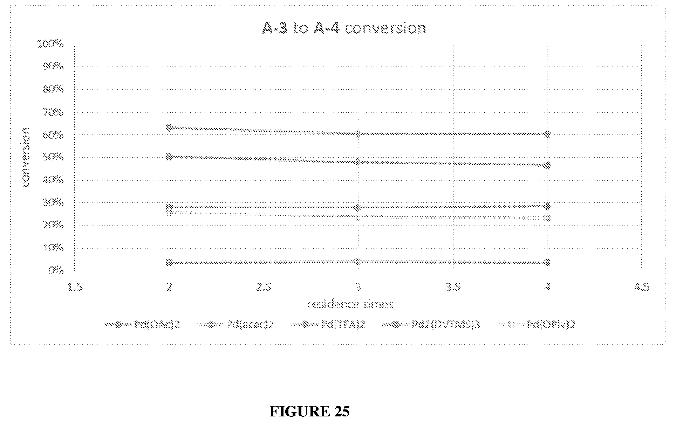

The results are provided in Figure 25. Pd(OPiv)₂ performed very similarly to Pd(OAc)2 with respect to **A-3** conversion. However the reaction with Pd(OPiv)₂ showed rapid polymer buildup, forcing shut-down of the reactor after only 5 residence times. In contrast, the reactions with Pd(OAc)₂ are relatively slow to build up polymer and the reactor was used multiple times before requiring cleanout. Pd(TFA)₂ gave better conversion than eitherPd(OAc)₂ or Pd(OPiv)₂. The Pd(0) catalyst Pd₂(DVTMS)₃ performed well in the cyclopropanation of **A-3,** showing the best conversion among the catalysts tested. The reactions with Pd₂(DVTMS)₃ ran with nearly zero pressure fluctuation in the system. No solids were observed in the effluent. These observations suggest that polymer formation may be reduced relative to the other catalysts.

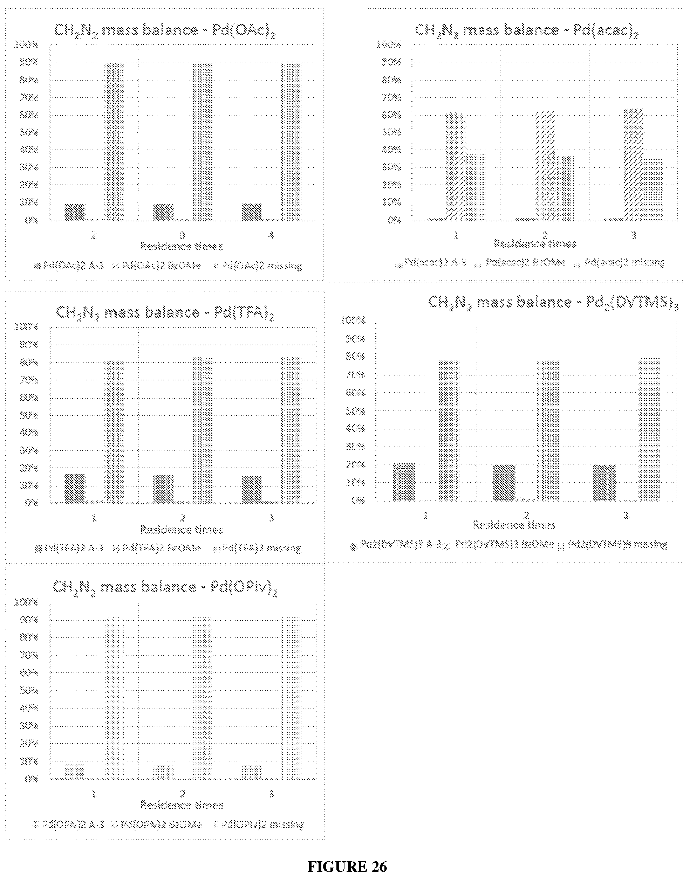

Figure 26 shows mass balance for CH₂N₂ where the product stream was quenched into benzoic acid where any residual CH₂N₂ is converted to methyl benzoate. As visible in Figure 26, in all cases except Pd(acac)₂, very little if any methyl benzoate is observed. Much of the CH₂N₂ mass is unaccounted for and presumably is being converted to polymer. When considering the Pd(II) catalysts, Pd(acac)₂ showed very little activity toward either cyclopropanation or polymerization of diazomethane, and a large portion of the diazomethane was converted to methyl benzoate. Unaccounted CH₂N₂ for the other palladium catalysts may be leading to the formation of a polymer.

### EXAMPLE 23

### Preparation and Isolation of Crystalline Form A of Compound I'

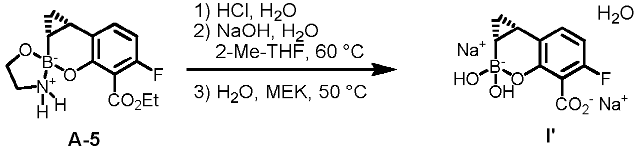

Crystalline Form A of Compound I' is the monohydrate of the disodium salt of Compound I.

2-Methyltetrahydrofuran (2-Me-THF, 8.0 kg / kg Compound A-5) was charged into a reactor, evacuated and backfilled with N₂, at 20 °C (15-25 °C), followed by the addition of Compound A-5 to give a suspension. Then dilute hydrochloric acid (HCl_{aq.}, 4-6%, 4.0 kg / kg Compound A-5) was dosed while stirring, converting Compound A-5 into its uncomplexed form. The bottom aqueous phase containing ethanolamine hydrochloride was separated (the product is in the top organic phase) and this extraction was subsequently repeated with more dilute hydrochloric acid (4.0 kg / kg Compound A-4) and finally with water (4.0 kg / kg Compound A-5).

To the remaining organic phase in the reactor was dosed dilute sodium hydroxide solution (NaOH_{aq.}, 4,2-5,0%, 7.35 kg / kg Compound A-5) while stirring, and the biphasic mixture was heated to 60 °C (55-65 °C), which converts Compound A-5 to Compound I. The reaction was run for a minimum of 6 h and then cooled to 20 °C (15-25 °C).

The aqueous bottom phase was separated and the organic top phase was discarded. The aqueous phase was charged back into the reactor to which was then added sufficient prepared acidic ion exchange resin to achieve a pH of 9.8 (9.0-10.5; in case the pH drops below this range, it may be corrected using dilute sodium hydroxide solution). Then the resin was filtered off in a filter dryer, the reactor and the resin were rinsed with water and the filtrates are passed through a cartridge of activated carbon and a polish filter. The aqueous product solution was then concentrated *in vacuo* to 2.11 kg / kg Compound A-5 at a maximum of 65 °C and subsequently dosed to stirred methylethyl ketone (MEK, 33.4 kg / kg Compound A-5) at 50 °C (45-55 °C) over 90 min and stirred for a minimum of 10 h, which crystallizes Compound I' (in case the water content in the MEK supernatant is too high, the slurry may be concentrated azeotropically). Following this, the product was filtered off in a filter dryer, washed with a mixture of MEK (2.5 kg / kg Compound A-5) and water (0.073 kg / kg Compound A-5) and finally dried *in vacuo* (approx. 100 mbar) at maximum 50 °C jacket temperature to obtain Compound I' as a monohydrate.

### EXAMPLE 24

### Crystallization of Form A of Compound I'

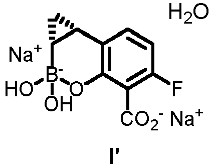

A reactor of suitable size was evacuated and backfilled with N₂. Then methyl ethyl ketone (MEK, 36.2 kg / kg Compound I') was transferred into the reactor and kept blanketed with N₂ at all times. Meanwhile, deionized water (1.30 kg / kg Compound I') was degassed by bubbling N₂ through for approx. 20 min, Compound I' was added, the mixture was degassed again for approx. 5 min and subsequently agitated until completely dissolved.

The jacket of the reactor containing the MEK antisolvent was brought to 95 °C and the solvent was refluxed for degassing for 45 min. After cooling the antisolvent back to 51 °C, the Compound I' solution prepared above was dosed over 85 min and 300 rpm stirrer speed, causing Compound I' to precipitate and crystallize as the disodium monohydrate salt. Residual Compound I' in the equipment used for dosing was rinsed into the reactor with degassed water (0.21 kg / kg Compound I'). Stirring was continued for 10 hours before the slurry was filtered through a nutsch filter at 50 °C. The reactor was rinsed with a mixture of MEK (2.7 kg / kg Compound I') and water (0.081 kg / kg Compound I'), the wet product was washed with the rinse mixture and the product was dried *in vacuo* (approx. 100 mbar) at a maximum of 50 °C to obtain Compound I' monohydrate in a highly crystalline form.

Differential Scanning Calorimetry (5 °C/min), Thermogravimetic analysis (5 °C/min), and Dynamic Vapor Sorption (stepwise RH decrease from 45% to 0% at 50 °C) data for highly crystalline Form A of Compound I' prepared analogously to the crystallization method above is shown in Figures 28-30, respectively. The data shows that the highly crystalline sample of Form A has an endotherm at 152 °C, exhibits a 5.3% weight loss when carried from 34 °C to 140 °C, and a 0.71 % weight loss from 140 °C to 238 °C. The highly crystalline sample does not begin to show appreciable water loss until heated above 100 °C, and resists gain/loss of water over a relative humidity range of 5% to 75%.

## Claims

1. A crystalline form of Compound I': 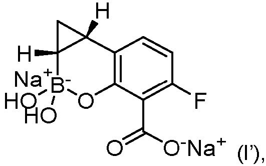 or a solvate thereof,
wherein the crystalline form exhibits an X-ray powder diffraction pattern comprising at least one characteristic peak, wherein said characteristic peak is selected from the group consisting of 6.2, 11.7, 12.4, 12.8, 16.7, 17.6, 19.1, 20.2, 20.6, 21.7, 22.0, 22.7, and 24.7 degrees 2θ ± 0.5 degrees 2θ; preferably
wherein the crystalline form exhibits an X-ray powder diffraction pattern comprising at least three characteristic peaks, wherein said characteristic peaks are selected from the group consisting of 6.2, 11.7, 12.4, 12.8, 16.7, 17.6, 19.1, 20.2, 20.6, 21.7, 22.0, 22.7, and 24.7 degrees 2θ ± 0.5 degrees 2θ; and/or
wherein the crystalline form has an endotherm at 142 °C and at 167 °C; or wherein the crystalline form has an endotherm at 152 °C; and/or
wherein the crystalline form is a hydrate, preferably wherein the crystalline form is a monohydrate.

2. A method of making a compound of Formula (A-II) or pharmaceutically acceptable salt thereof, 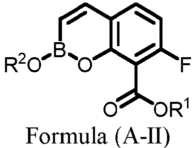 comprising the step of:
reacting a compound of Formula (A-I) 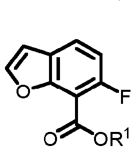 with a borylating agent in a solvent system, in the presence of a nickel catalyst and a base system;
wherein:
R¹ is a carboxylic acid protecting group
R² is hydrogen or C₁-C₆ alkyl; and
the solvent system and base system is selected from the group consisting of: THF and CsOH·H₂O, respectively; THF/H₂O and Cs₂CO₃, respectively; ethanol and CsOH·H₂O, respectively; THF/ethanol and Cs₂CO₃, respectively, MeTHF and CsOH·H₂O, respectively; MeTHF/H₂O and Cs₂CO₃, respectively; MeTHF/ethanol and Cs₂CO₃, respectively; THF and a mixture of Cs₂CO₃ and K₂CO₃, respectively; MeTHF and a mixture of Cs₂CO₃ and K₂CO₃, respectively; THF/H₂O and a mixture of Cs₂CO₃ and K₂CO₃, respectively; MeTHFH₂O and a mixture of Cs₂CO₃ and K₂CO₃, respectively; and MeTHF/ethanol and a mixture of Cs₂CO₃ and K₂CO₃, respectively; and
wherein the molar ratio of base system to the compound of formula (A-I) is from 1:1 to 2:1; preferably
wherein the nickel catalyst is formed by combining a nickel catalyst precursor and a ligand; preferably wherein the Ni catalyst precursor is selected from the group consisting of NiCl₂, Ni(Acac)₂, and Ni(COD)₂; preferably wherein the Ni catalyst precursor is NiCl₂; and/or
preferably wherein the ligand is selected from the group consisting of NHC ligand, phosphine, phosphite, phosphoramidite, amine, alcohol, aminoalcohol, and a combination thereof; preferably wherein the ligand is P(*n*-Bu)₃;
further comprising the step of reacting the organoboron intermediate of Formula (A-II) with (+)-pseudoephedrine as a chiral auxiliary to form an organoboron intermediate of Formula (A-III) 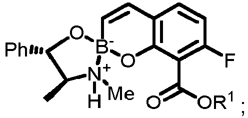 further comprising the step of converting the organoboron intermediate of Formula (A-III) to an organoboron intermediate of Formula (A-IV) 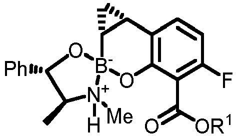
in the presence of a cyclopropanating agent and optional catalyst, wherein the cyclopropanating agent comprises CH₂I₂ and Zn/Cu; or
further comprising the step of reacting the organoboron intermediate of Formula (A-II) with a chiral auxiliary of Formula (Aux-I): 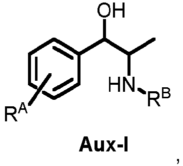 or stereoisomers thereof, to form an organoboron intermediate of Formula (B-III) 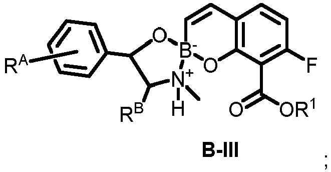 optionally further comprising the step of converting the organoboron intermediate of Formula (B-III) to an organoboron intermediate of Formula (B-IV) 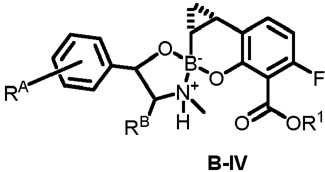 in the presence of a cyclopropanating agent and optional catalyst; wherein the cyclopropanating agent is CH₂N₂ and the catalyst is a palladium catalyst; wherein the cyclopropanating agent comprises CH₂I₂ and Zn/Cu couple;
wherein:
R^{A} is hydrogen, halogen, or C₁-C₆ alkyl; and
R^{B} is C₁-C₆ alkyl; preferably wherein the chiral auxiliary is a compound having the formula 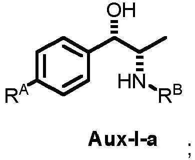
wherein R^{A} is hydrogen, halogen, or methyl; and/or
wherein R^{B} is selected from the group consisting of methyl, ethyl, and isopropyl.

3. The method of Claim 2, wherein the molar ratio of base system to the compound of formula (A-I) is 2:1; or wherein the molar ratio of base system to the compound of formula (A-I) is 1.5:1.

4. The method of any one of Claims 2 to 3, wherein the solvent system is THF/H₂O 5% v/v and the base system is Cs₂CO₃; or
wherein the solvent system is THF/ethanol 5% v/v and the base system is Cs₂CO₃; or
wherein the solvent system is MeTHF/H₂O 5% v/v and the base system is Cs₂CO₃; or
wherein the solvent system is MeTHF/ethanol 5% v/v and the base system is Cs₂CO₃; or
wherein the solvent system is THF/H₂O 5% v/v and the base system is a mixture of Cs₂CO₃ and K₂CO₃; or
wherein the solvent system is THF/ethanol 5% v/v and the base system is a mixture of Cs₂CO₃ and K₂CO₃; or
wherein the solvent system is MeTHF/H₂O 5% v/v and the base system is a mixture of Cs₂CO₃ and K₂CO₃; or
wherein the solvent system is MeTHF/ethanol 5% v/v and the base system is a mixture of Cs₂CO₃ and K₂CO₃.

5. The method of Claim 2, wherein the molar ratio of Cs₂CO₃ and K₂CO_{3 is} from 1:2 to 2:1 or
wherein the molar ratio of Cs₂CO₃ and K₂CO₃ is from 1.5:1 to 1.8.

6. The method of any one of Claims 2 to 5, wherein the ratio of borylating agent to the compound of formula (A-I) is from 1:1 to 2:1; or
wherein the ratio of borylating agent to the compound of formula (A-I) is 1.5:1; or
wherein the ratio of borylating agent to the compound of formula (A-I) is 1.25:1.

7. The method of any one of Claims 2 to 6, wherein the borylating agent is selected from the group consisting of (HO)₂B-B(OH)₂, B₂(Pin)₂, B₂(Cat)₂, and B₂neop₂; preferably wherein the borylating agent is B₂(Pin)₂.

8. The method of any one of Claims 2 to 7, wherein the nickel catalyst is selected from the graoup consisting of: NiCl₂(PPh₃)₂, NiCl₂(PCy₂Ph)₂, NiCl₂(PPh₂CH₂CH₂PPh₂), NiCl₂(1,3-Bis(diphenylphosphino)propane), and NiCl₂(1,3-Bis(diphenylphosphino)ethane) and NiCl₂(P(*n*-Bu)₃)₂ preferably wherein the nickel catalyst is NiCl₂(P(*n*-Bu)₃)₂.

9. The method of any one of Claims 2 to 8, wherein the method further comprises converting the organoboron intermediate of Formula (A-IV) to the organoboron intermediate of Formula (B-V); 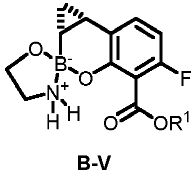
and/or further comprising the step of hydrolyzing the compound of Formula (A-IV) or Formula (B-V) to form Compound I: 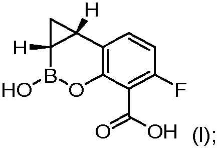 and/or
further comprising converting the organoboron intermediate of Formula (B-IV) to the organoboron intermediate of Formula (B-V); and/or 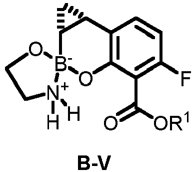
further comprising the step of hydrolyzing the compound of Formula (B-IV) or Formula (B-V) to form Compound I:

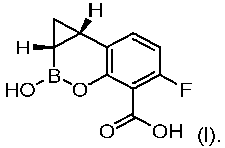

10. The method of any one of Claims 2 to 5, wherein R¹ is C₁₋₆ alkyl; preferably wherein R¹ is ethyl.

11. The method of any one of Claims 9 to 10, further comprising the step of washing the compound of Formula (B-IV), or Formula (B-V) with aqueous acid prior to hydrolysis; preferably
further comprising the step of hydrolyzing the compound of Formula (A-IV), Formula (B-IV), or Formula (B-V) in the presence of a base to form crude Compound I': 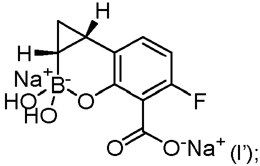
preferably
wherein the base is aqueous sodium hydroxide; and/or
wherein the step of hydrolyzing the compound of Formula (A-IV) is performed at a temperature of from 50 °C to 70 °C; preferably
wherein the step of hydrolyzing the compound of Formula (A-IV) is performed at a temperature of 60 °C; and/or
wherein the step of hydrolyzing the compound of Formula (A-IV) is performed for from 30 minutes to 6 hours; and/or
further comprising treating crude Compound I' with an ion exchange resin to achieve a pH of from 9 to 10.5; preferably
comprising treating crude Compound I' with the ion exchange resin to achieve a pH of 9.8; preferably
further filtering off the ion exchange resin to form an aqueous filtrate, preferably
wherein the aqueous filtrate is concentrated in vacuo to form a concentrated aqueous filtrate, more preferably
wherein the aqueous filtrate is concentrated in vacuo at a temperature of from 20 °C to 60 °C, more preferably
wherein the concentrated aqueous filtrate is added to methyl ethyl ketone to form a crystallization solution; most preferably
wherein the crystallization solution comprises from 1.0% to 5.0% water by weight relative to the quantity of methyl ethyl ketone; or
wherein the crystallization solution comprises from 2.5% to 4.5% water by weight relative to the quantity of methyl ethyl ketone.

12. A crystalline form of compound A-3, 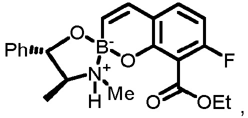 or a solvate thereof, wherein the crystalline form exhibits an X-ray powder diffraction pattern comprising at least one characteristic peak, wherein said characteristic peak is selected from the group consisting of 7.6. 8.0, 10.4, 13.7, 14.0, 16.0, 16.3, 19.0, 20.9, 23.2, 23.4, and 24.4 degrees 2θ ± 0.5 degrees 2θ; preferably
wherein the crystalline form exhibits an X-ray powder diffraction pattern comprising at least three characteristic peaks, wherein said characteristic peaks are selected from the group consisting of 7.6. 8.0, 10.4, 13.7, 14.0, 16.0, 16.3, 19.0, 20.9, 23.2, 23.4, and 24.4 degrees 2θ ± 0.5 degrees 2θ; and/or
wherein the crystalline form has a melting point of 194 °C; and/or
wherein the crystalline form is anhydrous.

13. A crystalline form of a compound of Formula (A-4): 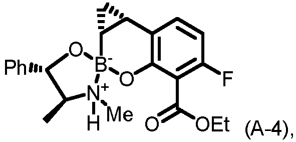 or a solvate thereof, wherein the crystalline form exhibits an X-ray powder diffraction pattern comprising at least one characteristic peak, wherein said characteristic peak is selected from the group consisting of 7.4, 8.9, 10.7, 11.2, 11.5, 12.8, 13.7, 15.3, 17.0, 17.7, 18.7, 20.1, 21.4, and 22.6 degrees 2θ ± 0.5 degrees 2θ; preferably
wherein the crystalline form exhibits an X-ray powder diffraction pattern comprising at least three characteristic peaks, wherein said characteristic peaks are selected from the group consisting of 7.4, 8.9, 10.7, 11.2, 11.5, 12.8, 13.7, 15.3, 17.0, 17.7, 18.7, 20.1, 21.4, and 22.6 degrees 2θ ± 0.5 degrees 2θ; and/or wherein the crystalline form has an endotherm at 176 °C; and/or
wherein the crystalline form is unsolvated.

14. A crystalline form of a compound of Formula (A-4): 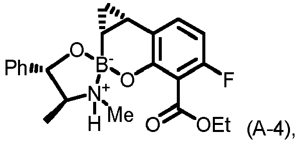 wherein the crystalline form exhibits an X-ray powder diffraction pattern comprising at least one characteristic peak, wherein said characteristic peak is selected from the group consisting of 7.4, 7.9, 9.1, 11.1, 12.0, 13.7, 14.4, 15.8, 16.4, 17.6, 19.8, 20.9, and 22.8 degrees 2θ ± 0.5 degrees 2θ; preferably
wherein the crystalline form exhibits an X-ray powder diffraction pattern comprising at least three characteristic peaks, wherein said characteristic peaks are selected from the group consisting of 7.4, 7.9, 9.1, 11.1, 12.0, 13.7, 14.4, 15.8, 16.4, 17.6, 19.8, 20.9, and 22.8 degrees 2θ ± 0.5 degrees 2θ.

15. A compound having the Formula (B-III): 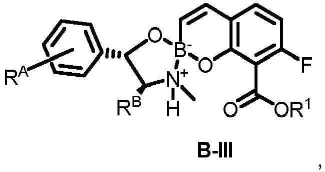 or a pharmaceutically acceptable salt or solvate thereof, **wherein**
R^{A} is C₁₋₆ alkyl or halogen;
R^{B} is hydrogen or C₁₋₆ alkyl; and
R¹ is C₁₋₆ alkyl; preferably wherein the compound is: 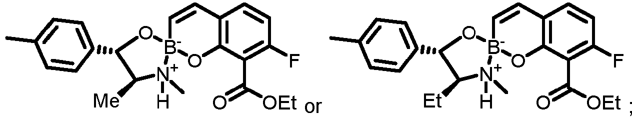 or a compound having the Formula (B-IV): 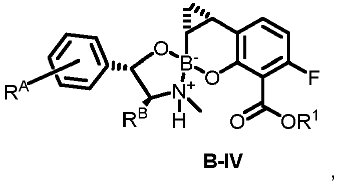 or a pharmaceutically acceptable salt or solvate thereof,
**wherein**
R^{A} is C₁₋₆ alkyl or halogen;
R^{B} is hydrogen or C₁₋₆ alkyl; and
R¹ is C₁₋₆ alkyl; preferably wherein the compound is: 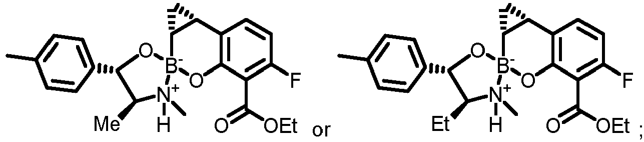 or a compound having the Formula (B-V): 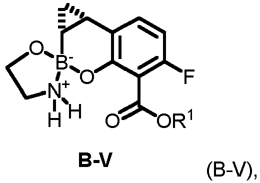 or a pharmaceutically acceptable salt or solvate thereof,
wherein R1 is C₁₋₆ alkyl; preferably wherein the compound is:

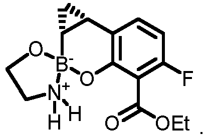

## Patentansprüche

1. Kristalline Form von Verbindung I': 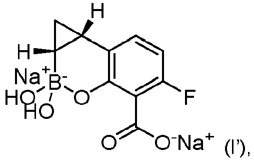 oder ein Solvat davon,
wobei die kristalline Form ein Röntgenpulverbeugungsmuster aufweist, das mindestens einen charakteristischen Peak umfasst, wobei der charakteristische Peak aus der Gruppe bestehend aus 6,2, 11,7, 12,4, 12,8, 16,7, 17,6, 19,1, 20,2, 20,6, 21,7, 22,0, 22,7 und 24,7 Grad 2θ ± 0,5 Grad 2θ ausgewählt ist; vorzugsweise
wobei die kristalline Form ein Röntgenpulverbeugungsmuster aufweist, das mindestens drei charakteristische Peaks umfasst, wobei die charakteristischen Peaks aus der Gruppe bestehend aus 6,2, 11,7, 12,4, 12,8, 16,7, 17,6, 19,1, 20,2, 20,6, 21,7, 22,0, 22,7 und 24,7 Grad 2θ ± 0,5 Grad 2θ ausgewählt sind; und/oder
wobei die kristalline Form eine Endotherme bei 142 °C und bei 167 °C aufweist; oder wobei die kristalline Form eine Endotherme bei 152 °C aufweist; und/oder
wobei es sich bei der kristallinen Form um ein Hydrat handelt, vorzugsweise wobei es sich bei der kristallinen Form um ein Monohydrat handelt.

2. Verfahren zur Herstellung einer Verbindung der Formel (A-II) oder eines pharmazeutisch unbedenklichen Salzes davon, 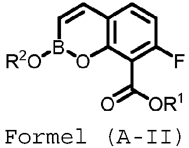
umfassend den Schritt: Umsetzen einer Verbindung der Formel (A-I) 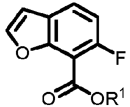
mit einem Borylierungsmittel in einem Lösungsmittelsystem in Gegenwart eines Nickelkatalysators und eines Basensystems;
wobei:
R¹ für eine Carbonsäureschutzgruppe steht,
R² für Wasserstoff oder C₁-C₆-Alkyl steht und
das Lösungsmittelsystem und das Basensystem ausgewählt werden aus der Gruppe bestehend aus: THF bzw. CsOH·H₂O; THF/H₂O bzw. Cs₂CO₃; Ethanol bzw. CsOH·H₂O; THF/Ethanol bzw. Cs₂CO₃, MeTHF bzw. CsOH H₂O; MeTHF/H₂O bzw. Cs₂CO₃; MeTHF/Ethanol bzw. Cs₂CO₃; THF bzw. einer Mischung von Cs₂CO₃ und K₂CO₃; MeTHF bzw. einer Mischung von Cs₂CO₃ und K₂CO₃; THF/H₂O bzw. einer Mischung von Cs₂CO₃ und K₂CO₃; MeTHFH₂O bzw. einer Mischung von Cs₂CO₃ und K₂CO₃ und MeTHF/Ethanol bzw. einer Mischung von Cs₂CO₃ und K₂CO₃; und
wobei das Molverhältnis von Basensystem zu Verbindung der Formel (A-I) 1:1 bis 2:1 beträgt; vorzugsweise wobei der Nickelkatalysator durch Kombinieren eines Nickelkatalysatorvorläufers und eines Liganden gebildet wird; vorzugsweise wobei der Ni-Katalysatorvorläufer aus der Gruppe bestehend aus NiCl₂, Ni(Acac)₂ und Ni(COD)₂ ausgewählt wird; vorzugsweise wobei es sich bei dem Ni-Katalysatorvorläufer um NiCl₂ handelt; und/oder
vorzugsweise wobei der Ligand aus der Gruppe bestehend aus NHC-Ligand, Phosphin, Phosphit, Phosphoramidit, Amin, Alkohol, Aminoalkohol und einer Kombination davon ausgewählt wird; vorzugsweise wobei es sich bei dem Liganden um P(n-Bu)₃ handelt;
ferner umfassend den Schritt des Umsetzens des Organobor-Zwischenprodukts der Formel (A-II) mit (+)-Pseudoephedrin als chiralem Auxiliar zur Bildung eines Organobor-Zwischenprodukts der Formel (A-III) 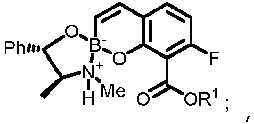 ferner umfassend den Schritt des Umwandelns des Organobor-Zwischenprodukts der Formel (A-III) in ein Organobor-Zwischenprodukt der Formel (A-IV) 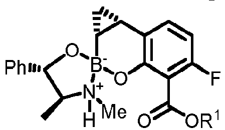 in Gegenwart eines Cyclopropanierungsmittels und eines fakultativen Katalysators, wobei das Cyclopropanierungsmittel CH₂I₂ und Zn/Cu umfasst; oder
ferner umfassend den Schritt des Umsetzens des Organobor-Zwischenprodukts der Formel (A-II) mit einem chiralen Auxiliar der Formel (Aux-I): 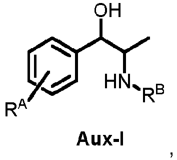 oder Stereoisomeren davon zur Bildung eines Organobor-Zwischenprodukts der Formel (B-III) 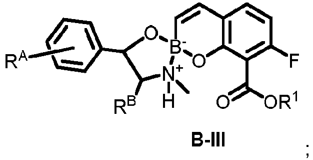
gegebenenfalls ferner umfassend den Schritt des Umwandelns des Organobor-Zwischenprodukts der Formel (B-III) in ein Organobor-Zwischenprodukt der Formel (B-IV) 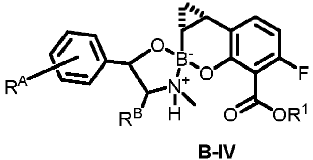 in Gegenwart eines Cyclopropanierungsmittels und eines fakultativen Katalysators; wobei es sich bei dem Cyclopropanierungsmittel um CH₂N₂ handelt und es sich bei dem Katalysator um einen Palladiumkatalysator handelt; wobei das Cyclopropanierungsmittel CH₂I₂ und Zn/Cu-Paar umfasst;
wobei:
R^{A} für Wasserstoff, Halogen oder C₁-C₆-Alkyl steht und
R^{B} für C₁-C₆-Alkyl steht; vorzugsweise wobei es sich bei dem chiralen Auxiliar um eine Verbindung der Formel 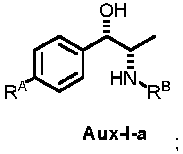 handelt;
wobei R^{A} für Wasserstoff, Halogen oder Methyl steht und/oder
wobei R^{B} aus der Gruppe bestehend aus Methyl, Ethyl und Isopropyl ausgewählt ist.

3. Verfahren nach Anspruch 2, wobei das Molverhältnis von Basensystem zu Verbindung der Formel (A-I) 2:1 beträgt oder wobei das Molverhältnis von Basensystem zu Verbindung der Formel (A-I) 1,5:1 beträgt.

4. Verfahren nach einem der Ansprüche 2 bis 3, wobei es sich bei dem Lösungsmittelsystem um THF/H₂O 5 % v/v handelt und es sich bei dem Basensystem um Cs₂CO₃ handelt; oder
wobei es sich bei dem Lösungsmittelsystem um THF/Ethanol 5 % v/v handelt und es sich bei dem Basensystem um Cs₂CO₃ handelt; oder
wobei es sich bei dem Lösungsmittelsystem um MeTHF/H₂O 5 % v/v handelt und es sich bei dem Basensystem um Cs₂CO₃ handelt; oder
wobei es sich bei dem Lösungsmittelsystem um MeTHF/Ethanol 5 % v/v handelt und es sich bei dem Basensystem um Cs₂CO₃ handelt; oder
wobei es sich bei dem Lösungsmittelsystem um THF/H₂O 5 % v/v handelt und es sich bei dem Basensystem um eine Mischung von Cs₂CO₃ und K₂CO₃ handelt; oder
wobei es sich bei dem Lösungsmittelsystem um THF/Ethanol 5 % v/v handelt und es sich bei dem Basensystem um eine Mischung von Cs₂CO₃ und K₂CO₃ handelt; oder
wobei es sich bei dem Lösungsmittelsystem um MeTHF/H₂O 5 % v/v handelt und es sich bei dem Basensystem um eine Mischung von Cs₂CO₃ und K₂CO₃ handelt; oder
wobei es sich bei dem Lösungsmittelsystem um MeTHF/Ethanol 5 % v/v handelt und es sich bei dem Basensystem um eine Mischung von Cs₂CO₃ und K₂CO₃ handelt.

5. Verfahren nach Anspruch 2, wobei das Molverhältnis von Cs₂CO₃ und K₂CO₃ 1:2 bis 2:1 beträgt oder wobei das Molverhältnis von Cs₂CO₃ und K₂CO₃ 1,5:1 bis 1,8 beträgt.

6. Verfahren nach einem der Ansprüche 2 bis 5, wobei das Verhältnis von Borylierungsmittel zu Verbindung der Formel (A-I) 1:1 bis 2:1 beträgt; oder
wobei das Verhältnis von Borylierungsmittel zu Verbindung der Formel (A-I) 1,5:1 beträgt; oder
wobei das Verhältnis von Borylierungsmittel zu Verbindung der Formel (A-I) 1,25:1 beträgt.

7. Verfahren nach einem der Ansprüche 2 bis 6, wobei das Borylierungsmittel aus der Gruppe bestehend aus (HO)₂B-B(OH)₂, B₂(Pin)₂, B₂(Cat)₂ und B₂neop₂ ausgewählt wird; vorzugsweise wobei es sich bei dem Borylierungsmittel um B₂(Pin)₂ handelt.

8. Verfahren nach einem der Ansprüche 2 bis 7, wobei der Nickelkatalysator ausgewählt wird aus der Gruppe bestehend aus: NiCl₂(PPh₃)₂, NiCl₂(PCy₂Ph)₂, NiCl₂(PPh₂CH₂CH₂PPh₂), NiCl₂(1,3-Bis(diphenylphosphino)propan) und NiCl₂(1,3-Bis(diphenylphosphino)ethan) und NiCl₂(P(n-Bu)₃)₂; vorzugsweise wobei es sich bei dem Nickelkatalysator um NiCl₂(P(n-Bu)₃)₂ handelt.

9. Verfahren nach einem der Ansprüche 2 bis 8, wobei das Verfahren ferner das Umwandeln des Organobor-Zwischenprodukts der Formel (A-IV) in das Organobor-Zwischenprodukt der Formel (B-V) umfasst; 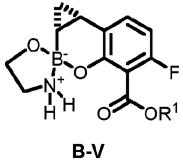 und/oder ferner umfassend den Schritt des Hydrolysierens der Verbindung der Formel (A-IV) oder Formel (B-V) zur Bildung von Verbindung I: 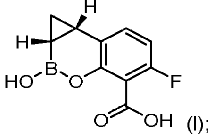 und/oder ferner umfassend das Umwandeln des Organobor-Zwischenprodukts der Formel (B-IV) in das Organobor-Zwischenprodukt der Formel (B-V); und/oder 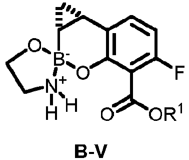 ferner umfassend den Schritt des Hydrolysierens der Verbindung der Formel (B-IV) oder Formel (B-V) zur Bildung von Verbindung I:

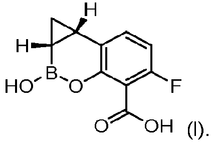

10. Verfahren nach einem der Ansprüche 2 bis 5, wobei R¹ für C₁₋₆-Alkyl steht; vorzugsweise wobei R¹ für Ethyl steht.

11. Verfahren nach einem der Ansprüche 9 bis 10, ferner umfassend den Schritt des Waschens der Verbindung der Formel (B-IV) oder Formel (B-V) mit wässriger Säure vor der Hydrolyse; vorzugsweise
ferner umfassend den Schritt des Hydrolysierens der Verbindung der Formel (A-IV), Formel (B-IV) oder Formel (B-V) in Gegenwart einer Base zur Bildung von roher Verbindung I': 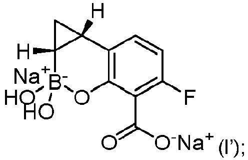 vorzugsweise
wobei es sich bei der Base um wässriges Natriumhydroxid handelt; und/oder
wobei der Schritt des Hydrolysierens der Verbindung der Formel (A-IV) bei einer Temperatur von 50 °C bis 70 °C durchgeführt wird; vorzugsweise
wobei der Schritt des Hydrolysierens der Verbindung der Formel (A-IV) bei einer Temperatur von 60 °C durchgeführt wird; und/oder
wobei der Schritt des Hydrolysierens der Verbindung der Formel (A-IV) über einen Zeitraum von 30 Minuten bis 6 Stunden durchgeführt wird; und/oder
ferner umfassend das Behandeln von roher Verbindung I' mit einem Ionenaustauscherharz zur Erreichung eines pH-Werts von 9 bis 10,5; vorzugsweise
umfassend das Behandeln von roher Verbindung I' mit dem Ionenaustauscherharz zur Erreichung eines pH-Werts von 9,8; vorzugsweise
das Ionenaustauscherharz ferner abfiltriert wird, wobei man ein wässriges Filtrat erhält, vorzugsweise wobei das wässrige Filtrat im Vakuum aufkonzentriert wird, wobei man ein konzentriertes wässriges Filtrat erhält, weiter bevorzugt
wobei das wässrige Filtrat im Vakuum bei einer Temperatur von 20 °C bis 60 C ° aufkonzentriert wird, weiter bevorzugt
wobei das konzentrierte wässrige Filtrat zu Methylethylketon gegeben wird, wobei man eine Kristallisationslösung erhält; ganz besonders bevorzugt wobei die Kristallisationslösung 1,0 bis 5,0 Gew.-% Wasser, bezogen auf die Menge an Methylethylketon, umfasst; oder
wobei die Kristallisationslösung 2,5 bis 4,5 Gew.-% Wasser, bezogen auf die Menge an Methylethylketon, umfasst.

12. Kristalline Form von Verbindung A-3, 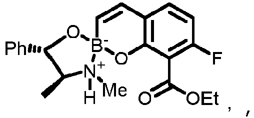 oder ein Solvat davon, wobei die kristalline Form ein Röntgenpulverbeugungsmuster aufweist, das mindestens einen charakteristischen Peak umfasst, wobei der charakteristische Peak aus der Gruppe bestehend aus 7,6, 8,0, 10,4, 13,7, 14,0, 16,0, 16,3, 19,0, 20,9, 23,2, 23,4 und 24,4 Grad 2θ ± 0,5 Grad 2θ ausgewählt ist; vorzugsweise
wobei die kristalline Form ein Röntgenpulverbeugungsmuster aufweist, das mindestens drei charakteristische Peaks umfasst, wobei die charakteristischen Peak aus der Gruppe bestehend aus 7,6, 8,0, 10,4, 13,7, 14,0, 16,0, 16,3, 19,0, 20,9, 23,2, 23,4 und 24,4 Grad 2θ ± 0,5 Grad 2θ ausgewählt sind; und/oder wobei die kristalline Form einen Schmelzpunkt von 194 °C aufweist; und/oder
wobei die kristalline Form wasserfrei ist.

13. Kristalline Form einer Verbindung der Formel (A-4): 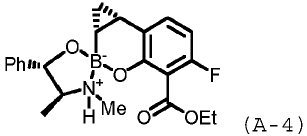 oder ein Solvat davon, wobei die kristalline Form ein Röntgenpulverbeugungsmuster aufweist, das mindestens einen charakteristischen Peak umfasst, wobei der charakteristische Peak aus der Gruppe bestehend aus 7,4, 8,9, 10,7, 11,2, 11,5, 12,8, 13,7, 15,3, 17,0, 17,7, 18,7, 20,1, 21,4 und 22,6 Grad 2θ ± 0,5 Grad 2θ ausgewählt ist; vorzugsweise
wobei die kristalline Form ein Röntgenpulverbeugungsmuster aufweist, das mindestens drei charakteristische Peaks umfasst, wobei die charakteristischen Peaks aus der Gruppe bestehend aus 7,4, 8,9, 10,7, 11,2, 11,5, 12,8, 13,7, 15,3, 17,0, 17,7, 18,7, 20,1, 21,4 und 22,6 Grad 2θ ± 0,5 Grad 2θ ausgewählt sind; und/oder
wobei die kristalline Form eine Endotherme bei 176 °C aufweist; und/oder
wobei die kristalline Form unsolvatisiert ist.

14. Kristalline Form einer Verbindung der Formel (A-4): 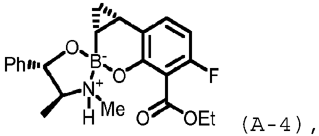 wobei die kristalline Form ein Röntgenpulverbeugungsmuster aufweist, das mindestens einen charakteristischen Peak umfasst, wobei der charakteristische Peak aus der Gruppe bestehend aus 7,4, 7,9, 9,1, 11,1, 12,0, 13,7, 14,4, 15,8, 16,4, 17,6, 19,8, 20,9 und 22,8 Grad 2θ ± 0,5 Grad 2θ ausgewählt ist; vorzugsweise
wobei die kristalline Form ein Röntgenpulverbeugungsmuster aufweist, das mindestens drei charakteristische Peaks umfasst, wobei die charakteristischen Peaks aus der Gruppe bestehend aus 7,4, 7,9, 9,1, 11,1, 12,0, 13,7, 14,4, 15,8, 16,4, 17,6, 19,8, 20,9 und 22,8 Grad 2θ ± 0,5 Grad 2θ ausgewählt sind.

15. Verbindung mit der Formel (B-III): 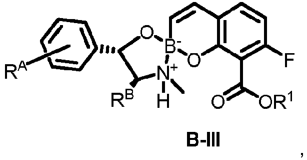 oder ein pharmazeutisch unbedenkliches Salz oder Solvat davon,
wobei
R^{A} für C₁₋₆-Alkyl oder Halogen steht;
R^{B} für Wasserstoff oder C₁₋₆-Alkyl steht und
R¹ für C₁₋₆-Alkyl steht; vorzugsweise wobei es sich bei der Verbindung um Folgendes handelt: 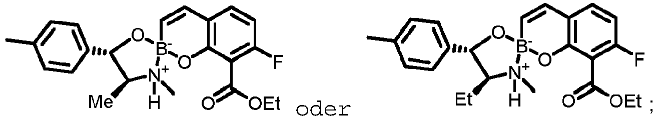 oder
Verbindung mit der Formel (B-IV): 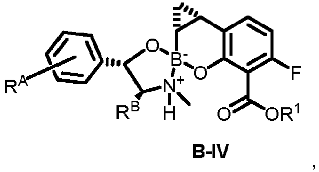 oder ein pharmazeutisch unbedenkliches Salz oder Solvat davon,
wobei
R^{A} für C₁₋₆-Alkyl oder Halogen steht;
R^{B} für Wasserstoff oder C₁₋₆-Alkyl steht und
R¹ für C₁₋₆-Alkyl steht; vorzugsweise wobei es sich bei der Verbindung um Folgendes handelt: 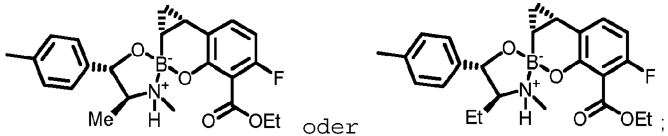 oder Verbindung mit der Formel (B-V): 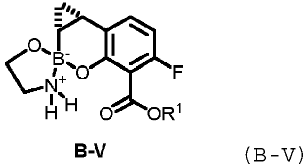 oder ein pharmazeutisch unbedenkliches Salz oder Solvat davon,
wobei R¹ für C₁₋₆-Alkyl steht; vorzugsweise wobei es sich bei der Verbindung um Folgendes handelt:

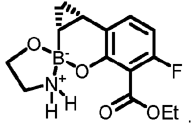

## Revendications

1. Forme cristalline du composé I' : 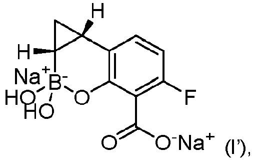 ou d'un solvate de celui-ci, dans laquelle la forme cristalline présente un diagramme de diffraction des rayons X sur poudre comprenant au moins un pic caractéristique, ledit pic caractéristique étant choisi dans le groupe constitué par 6,2, 11,7, 12,4, 12,8, 16,7, 17,6, 19,1, 20,2, 20,6, 21,7, 22,0, 22,7 et 24,7 degrés 2θ ± 0,5 degré 2θ ; de préférence dans laquelle la forme cristalline présente un diagramme de diffraction des rayons X sur poudre comprenant au moins trois pics caractéristiques, lesdits pics caractéristiques étant choisis dans le groupe constitué par 6,2, 11,7, 12,4, 12,8, 16,7, 17,6, 19,1, 20,2, 20,6, 21,7, 22,0, 22,7 et 24,7 degrés 2θ ± 0,5 degré 2θ ; et/ou dans laquelle la forme cristalline a un endotherme à 142 °C et à 167 °C ; ou dans laquelle la forme cristalline a un endotherme à 152 °C ; et/ou
dans laquelle la forme cristalline est un hydrate, de préférence dans laquelle la forme cristalline est un monohydrate.

2. Procédé de préparation d'un composé de formule (A-II) ou d'un sel pharmaceutiquement acceptable de celui-ci, 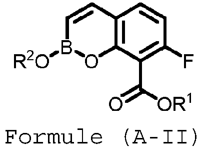 comprenant l'étape de :
mise en réaction d'un composé de formule (A-I) 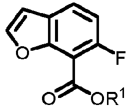 avec un agent de borylation dans un système de solvant, en présence d'un catalyseur au nickel et d'un système de base ;
dans laquelle :
R¹ est un groupe protecteur d'acide carboxylique
R² est hydrogène ou C₁-C₆ alkyle ; et
le système de solvant et le système de base sont choisis dans le groupe constitué par : THF et CsOH·H₂O, respectivement; THF/H₂O et Cs₂CO₃, respectivement; éthanol et CsOH·H₂O, respectivement; THF/éthanol et Cs₂CO₃, respectivement, MeTHF et CsOH H₂O, respectivement; MeTHF/H₂O et Cs₂CO₃, respectivement; MeTHF/éthanol et Cs₂CO₃, respectivement; THF et un mélange de Cs₂CO₃ et K₂CO₃, respectivement; MeTHF et un mélange de Cs₂CO₃ et K₂CO₃, respectivement; THF/H₂O et un mélange de Cs₂CO₃ et K₂CO₃, respectivement; MeTHFH₂O et un mélange de Cs₂CO₃ et K₂CO₃, respectivement; et MeTHF/éthanol et un mélange de Cs₂CO₃ et K₂CO₃, respectivement; et
dans lequel le rapport molaire de système de base sur le composé de formule (A-I) est de 1:1 à 2:1 ; de préférence dans lequel le catalyseur au nickel est formé en combinant un précurseur de catalyseur au nickel et un ligand ; de préférence dans lequel le précurseur de catalyseur au Ni est choisi dans le groupe constitué par NiCl₂, Ni(Acac)₂ et Ni(COD)₂ ; de préférence dans laquelle le précurseur de catalyseur au Ni est NiCl₂ ; et/ou
de préférence, dans lequel le ligand est choisi dans le groupe constitué par un ligand NHC, phosphine, phosphite, phosphoramidite, amine, alcool, aminoalcool, et une combinaison de ceux-ci ; de préférence dans laquelle le ligand est P(n-Bu)₃ ;
comprenant en outre l'étape de mise en réaction de l'intermédiaire organobore de formule (A-II) avec de la (+)-pseudoéphédrine en tant qu'auxiliaire chiral pour former un intermédiaire organobore de formule (A-III) 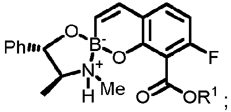 comprenant en outre l'étape de conversion de l'intermédiaire organobore de formule (A-III) en un intermédiaire organobore de formule (A-IV) 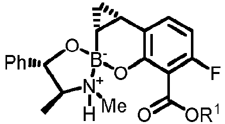 en présence d'un agent de cyclopropanation et d'un catalyseur facultatif, dans laquelle l'agent de cyclopropanation comprend CH₂I₂ et Zn/Cu ; ou
comprenant en outre l'étape de mise en réaction de l'intermédiaire organobore de formule (A-II) avec un auxiliaire chiral de formule (Aux-I) : 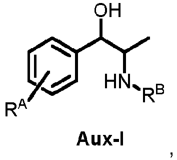 ou des stéréoisomères de celui-ci, pour former un intermédiaire organobore de formule (B-III) 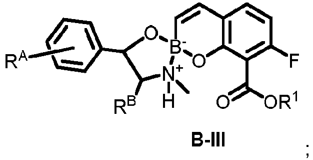
comprenant éventuellement en outre l'étape de conversion de l'intermédiaire organobore de formule (B-III) en intermédiaire organobore de formule (B-IV) 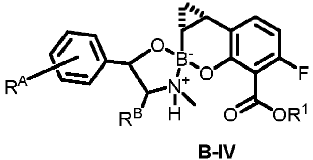 en présence d'un agent de cyclopropanation et d'un catalyseur facultatif ; dans lequel l'agent de cyclopropanation est CH₂N₂ et le catalyseur est un catalyseur au palladium ; dans lequel l'agent de cyclopropanation comprend CH₂I₂ et un couple Zn/Cu ;
dans laquelle :
R^{A} est hydrogène, halogène, ou C₁-C₆alkyle ; et
R^{B} est alkyle en C₁-C₆ ; de préférence dans laquelle l'auxiliaire chiral est un composé ayant la formule 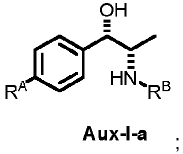
dans laquelle R^{A} est hydrogène, halogène ou méthyle ; et/ou
dans laquelle R^{B} est choisi dans le groupe constitué par méthyle, éthyle et isopropyle.

3. Procédé selon la revendication 2, dans lequel le rapport molaire du système de base sur le composé de formule (A-I) est de 2:1 ; ou dans lequel le rapport molaire du système de base sur le composé de formule (AI) est 1,5:1.

4. Procédé selon l'une quelconque des revendications 2 à 3, dans lequel le système de solvant est THF/H₂O 5 % v/v et le système de base est Cs₂CO₃ ; ou
dans lequel le système de solvant est THF/éthanol à 5 % v/v et le système de base est Cs₂CO₃ ; ou
dans lequel le système de solvant est MeTHF/H₂O 5 % v/v et le système de base est Cs₂CO₃ ; ou
dans lequel le système de solvant est MeTHF/éthanol 5 % v/v et le système de base est Cs₂CO₃ ; ou
dans lequel le système de solvant est THF/H₂O 5 % v/v et le système de base est un mélange de Cs₂CO₃ et K₂CO₃ ; ou
dans lequel le système de solvant est THF/éthanol à 5 % v/v et le système de base est un mélange de Cs₂CO₃ et K₂CO₃ ; ou
dans lequel le système de solvant est MeTHF/H₂O 5 % v/v et le système de base est un mélange de Cs₂CO₃ et K₂CO₃ ; ou
dans lequel le système de solvant est MeTHF/éthanol 5 % v/v et le système de base est un mélange de Cs₂CO₃ et K₂CO₃.

5. Procédé selon la revendication 2, dans lequel le rapport molaire de Cs₂CO₃ et K₂CO₃ est de 1:2 à 2:1 ou dans lequel le rapport molaire de Cs₂CO₃ et K₂CO₃ est de 1,5:1 à 1,8.

6. Procédé selon l'une quelconque des revendications 2 à 5, dans lequel le rapport d'agent de borylation sur le composé de formule (A-I) est de 1:1 à 2:1 ; ou
dans lequel le rapport d'agent de borylation sur le composé de formule (A-I) est 1,5:1 ; ou
dans lequel le rapport d'agent de borylation sur le composé de formule (A-I) est 1,25:1.

7. Procédé selon l'une quelconque des revendications 2 à 6, dans lequel l'agent de borylation est choisi dans le groupe constitué par (HO)₂B-B(OH)₂, B₂(Pin)₂, B₂(Cat)₂ et B₂néop₂ ; de préférence dans lequel l'agent de borylation est B₂(Pin)₂.

8. Procédé selon l'une quelconque des revendications 2 à 7, dans lequel le catalyseur au nickel est choisi dans le groupe constitué par : NiCl₂(PPh₃)₂, NiCl₂(PCy₂Ph)₂, NiCl₂(PPh₂CH₂CH₂PPh₂), NiCl₂(1,3-Bis(diphénylphosphino)propane), et NiCl₂(1,3-Bis(diphénylphosphino)éthane) et NiCl₂(P(n-Bu)₃)₂ ; de préférence dans lequel le catalyseur au nickel est NiCl₂(P(n-Bu)₃)₂.

9. Procédé selon l'une quelconque des revendications 2 à 8, dans lequel le procédé comprend en outre la conversion de l'intermédiaire organobore de formule (A-IV) en l'intermédiaire organobore de formule (B-V) ; 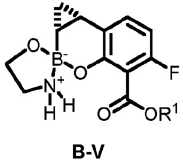 et/ou comprenant en outre l'étape d'hydrolyse du composé de formule (A-IV) ou formule (B-V) pour former le composé I : 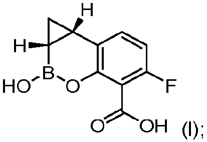 et/ou comprenant en outre la conversion de l'intermédiaire organobore de formule (B-IV) en l'intermédiaire organobore de formule (B-V) ; et/ou 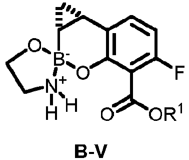 comprenant en outre l'étape d'hydrolyse du composé de formule (B-IV) ou de formule (B-V) pour former le composé I :

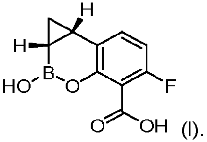

10. Procédé selon l'une quelconque des revendications 2 à 5, dans lequel R¹ est un alkyle en C₁₋₆ ; de préférence dans lequel R¹ est éthyle.

11. Procédé selon l'une quelconque des revendications 9 à 10, comprenant en outre l'étape de lavage du composé de formule (B-IV), ou de formule (B-V) avec un acide aqueux avant hydrolyse ; de préférence comprenant en outre l'étape d'hydrolyse du composé de formule (A-IV), de formule (B-IV) ou de formule (B-V) en présence d'une base pour former du composé I' brut : 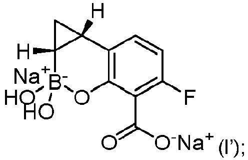 de préférence
dans lequel la base est l'hydroxyde de sodium aqueux ; et/ou
dans lequel l'étape d'hydrolyse du composé de formule (A-IV) est réalisée à une température allant de 50 °C à 70 °C ; de préférence
dans lequel l'étape d'hydrolyse du composé de formule (A-IV) est effectuée à une température de 60 °C ; et/ou
dans lequel l'étape d'hydrolyse du composé de formule (A-IV) est effectuée pendant 30 minutes à 6 heures ; et/ou comprenant en outre le traitement du composé I' brut avec une résine échangeuse d'ions pour atteindre un pH de 9 à 10,5 ; de préférence
comprenant le traitement du composé I' brut avec la résine échangeuse d'ions pour atteindre un pH de 9,8 ; de préférence
on filtre ensuite la résine échangeuse d'ions pour former un filtrat aqueux, de préférence
dans lequel le filtrat aqueux est concentré sous vide pour former un filtrat aqueux concentré, plus préférablement
dans lequel le filtrat aqueux est concentré sous vide à une température de 20 °C à 60 °C, plus préférablement dans lequel le filtrat aqueux concentré est ajouté à la méthyléthylcétone pour former une solution de cristallisation ; de manière préférée entre toutes dans lequel la solution de cristallisation comprend de 1,0 à 5,0% d'eau en poids par rapport à la quantité de méthyléthylcétone ; ou
dans lequel la solution de cristallisation comprend de 2,5 à 4,5 % d'eau en poids par rapport à la quantité de méthyléthylcétone.

12. Forme cristalline du composé A-3, 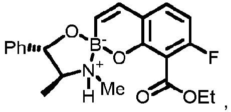 ou d'un solvate de celui-ci, dans laquelle la forme cristalline présente un diagramme de diffraction des rayons X sur poudre comprenant au moins un pic caractéristique, ledit pic caractéristique étant choisi dans le groupe constitué par 7,6, 8,0, 10,4, 13,7, 14,0, 16,0, 16,3, 19,0, 20,9, 23,2, 23,4 et 24,4 degrés 2θ ± 0,5 degré 2θ ; préférentiellement
dans laquelle la forme cristalline présente un diagramme de diffraction des rayons X sur poudre comprenant au moins trois pics caractéristiques, lesdits pics caractéristiques étant choisis dans le groupe constitué par 7,6, 8,0, 10,4, 13,7, 14,0, 16,0, 16,3, 19,0, 20,9, 23,2, 23,4 et 24,4 degrés 2θ ± 0,5 degré 2θ ; et/ou
dans laquelle la forme cristalline possède un point de fusion de 194 °C ; et/ou
dans laquelle la forme cristalline est anhydre.

13. Forme cristalline d'un composé de formule (A-4) : 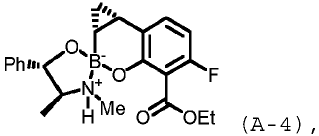 ou d'un solvate de celui-ci, dans laquelle la forme cristalline présente un diagramme de diffraction des rayons X sur poudre comprenant au moins un pic caractéristique, dans laquelle ledit pic caractéristique est choisi dans le groupe constitué par 7,4, 8,9, 10,7, 11,2, 11,5, 12,8, 13,7, 15,3, 17,0, 17,7, 18,7, 20,1, 21,4 et 22,6 degrés 2θ ± 0,5 degré 2θ ; de préférence
dans laquelle la forme cristalline présente un diagramme de diffraction des rayons X sur poudre comprenant au moins trois pics caractéristiques, lesdits pics caractéristiques étant choisis dans le groupe constitué par 7,4, 8,9, 10,7, 11,2, 11,5, 12,8, 13,7, 15,3, 17,0, 17,7, 18,7, 20,1, 21,4 et 22,6 degrés 2θ ± 0,5 degré 2θ ; et/ou
dans laquelle la forme cristalline a un endotherme à 176 °C ; et/ou
dans laquelle la forme cristalline est non solvatée.

14. Forme cristalline d'un composé de formule (A-4) : 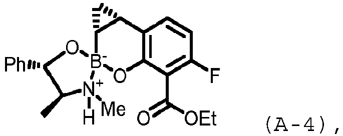 dans laquelle la forme cristalline présente un diagramme de diffraction des rayons X sur poudre comprenant au moins un pic caractéristique, ledit pic caractéristique étant choisi dans le groupe constitué par 7,4, 7,9, 9,1, 11,1, 12,0, 13,7, 14,4, 15,8, 16,4, 17,6, 19,8, 20,9 et 22,8 degrés 2θ ± 0,5 degré 2θ ; préférentiellement
dans laquelle la forme cristalline présente un diagramme de diffraction des rayons X sur poudre comprenant au moins trois pics caractéristiques, lesdits pics caractéristiques étant choisis dans le groupe constitué par 7,4, 7,9, 9,1, 11,1, 12,0, 13,7, 14,4, 15,8, 16,4, 17,6, 19,8, 20,9 et 22,8 degrés 2θ ± 0,5 degré 2θ.

15. Composé ayant la formule (B-III) : 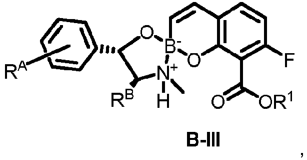 ou sel ou solvate pharmaceutiquement acceptable correspondant,
dans laquelle
R^{A} est C₁₋₆ alkyle ou halogène ;
R^{B} est hydrogène ou C₁₋₆ alkyle ; et
R¹ est C₁₋₆ alkyle ; de préférence dans lequel le composé est : 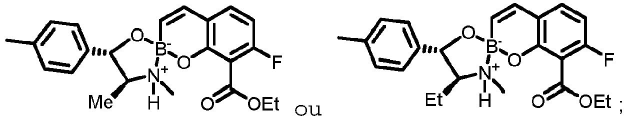 ou composé ayant la formule (B-IV) : 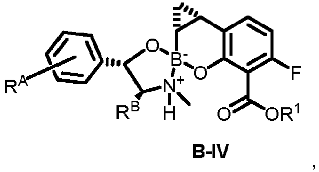 ou sel ou solvate pharmaceutiquement acceptable correspondant,
dans laquelle
R^{A} est C₁₋₆ alkyle ou halogène ;
R^{B} est hydrogène ou C₁₋₆ alkyle ; et
R¹ est C₁₋₆ alkyle ; de préférence dans lequel le composé est : 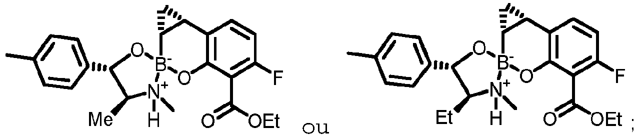 ou composé ayant la formule (B-V) : 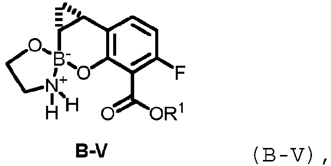 ou sel ou solvate pharmaceutiquement acceptable correspondant,
dans laquelle R¹ est C₁₋₆ alkyle ; préférablement dans lequel le composé est :
